# EUROPEAN PATENT APPLICATION

(11) **EP 4 741 379 A1**
(43) Date of publication of application: **13.05.2026**
(21) Application number: 24835416.9
(22) Date of filing: 04.07.2024
(51) Int. Cl.: C07D 249/04, C07D 403/00, C07D 401/02, C07D 401/14, A61K 31/41, A61P 25/00, A61P 29/00, A61P 31/04

(54) **CLASS OF PROGRAMMED CELL DEATH INHIBITORS, PREPARATION METHOD THEREFOR AND USE THEREOF**

(30) Priority: 05.07.2023 CN 202310819087
(71) Applicant: SHANGHAI INSTITUTE OF ORGANIC CHEMISTRY, CHINESE ACADEMY OF SCIENCES, Shanghai 200032 (CN)
(72) Inventor: TAN, Li, Shanghai 200032 (CN); YUAN, Junying, Shanghai 200032 (CN); LI, Ying, Shanghai 200032 (CN); XIANG, Huaijiang, Shanghai 200032 (CN); QIN, Ying, Shanghai 200032 (CN)
(74) Representative: van Dam, Vincent
(86) International application number: PCT/CN2024/103713
(87) International publication number: WO 2025/007938

(57) **Abstract**

The present invention provides a class of programmed cell death inhibitors, a preparation method therefor and a use thereof. Specifically, the present invention provides a compound, or a pharmaceutically acceptable salt, hydrate or solvate thereof, the compound being as shown in Formula I. The present invention also provides a method for preparing the compound and a pharmaceutical composition comprising the compound or a use thereof in treating or preventing diseases or conditions associated with programmed cell death and/or human receptor-interacting protein 1 kinase (RIPK1).

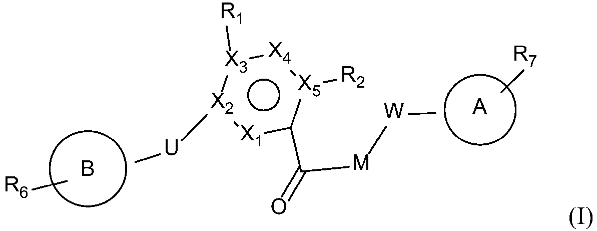

## Description

### Technical Field

The present disclosure belongs to the field of small molecule compounds, and specifically relates to a class of inhibitors of programmed cell necrosis (necroptosis), as well as a method for preparing the same and use thereof.

### Background Art

A human body is always accompanied by dynamic regulation of cell proliferation and death during development and aging. Programmed cell death is indispensable in physiological activities such as normal development, resisting pathogen invasion, and maintaining homeostasis, and its dysregulation often leads to developmental abnormalities, immune system diseases, neurodegenerative diseases, cancers, etc., and even the death of an individual. Therefore, intervening in programmed cell death is of great significance for disease treatment research. Apoptosis is the first elucidated mechanism of programmed cell death. In recent years, programmed cell necrosis has become a new focus in the field of cell death. Numerous studies have reported that in various degenerative diseases (such as Alzheimer's disease (AD), multiple sclerosis (MS), amyotrophic lateral sclerosis (ALS), retinal degenerative diseases, etc.), ischemia-reperfusion injuries (such as cerebral infarction, myocardial infarction, etc.), as well as pathogen infections, programmed cell necrosis occurs as an important pathological feature.

On the other hand, the activation of programmed cell necrosis pathways is often accompanied by inflammation. Persistent inflammation can cause irreversible damage to human tissues or organs and also promote the progression of various diseases such as cardiovascular diseases, diabetes, cancers, non-alcoholic fatty liver diseases, degenerative diseases, metabolic syndromes, etc., and is described as the root of all diseases. Various chronic inflammatory diseases, such as rheumatoid arthritis (RA), inflammatory bowel disease (IBD), psoriasis, or hepatitis, not only severely affect patients' health and quality of life but may also trigger fatal heart attacks or cancers; and acute inflammatory storms caused by infections, such as sepsis or severe pneumonia caused by COVID-19 infection, can directly threaten life. Tumor immunotherapies using CAR-T cells or PD-1/PD-L1 antibodies often also have acute inflammatory side effects of varying degrees, seriously affecting patients' quality of life or safety. In addition, programmed cell necrosis and inflammation are also involved in regulating tumor microenvironments: lung cancer cells can induce programmed cell necrosis in specific cells of vascular wall to facilitate metastasis through the circulatory system; high expression of main components of necrosome in pancreatic cancer can induce the expression of chemokine CXCL1, thereby suppressing body immune response. Therefore, inhibiting the occurrence of programmed cell necrosis is widely recognized as beneficial for the treatment and alleviation of various diseases.

Researches have shown that tumor necrosis factor α (TNF-α) is one of the main pathways to stimulate programmed cell necrosis and inflammation in the body, and its downstream signaling pathway is also the necrosis signaling pathway with the most clear mechanism at present. In this pathway, RIPK1 plays a crucial regulatory role: by forming signaling Complex I with TNFα receptor protein TNFR1 and other regulatory factors (TRADD, TRAF2, cIAP1/2, etc.), it activates IKK complex and NF-κB inflammatory pathway; after the kinase activity is activated, it enters the cytoplasm and forms Complex IIa with apoptotic factors (FADD, Caspase-3, etc.) to mediate apoptosis; or it activates RIPK3 and polymerizes with it to form necrosome Complex IIb, ultimately inducing necroptosis. During necroptosis, the cell membrane structure is destroyed and a large number of inflammatory factors are released, which in turn triggers a chain reaction of inflammation and cell death. Studies have shown that genetic mutations in RIPK1 gene (D324V, D324H) lead to abnormal accumulation and activation of RIPK1 protein, and can cause the occurrence of autoimmune diseases; during aging, mutations and inactivation of upstream inhibitory regulatory factors (such as OPTN, TBK1, TAK1, A20, CYLD, etc.) also lead to RIPK1 activation, further aggravating degenerative diseases and inflammatory conditions; in various fungal or viral infections and organ ischemia-reperfusion injuries, RIPK1 is also abnormally activated, inducing inflammatory cell death. In the treatment of central nervous system diseases, antibody drugs have the disadvantages of poor blood-brain barrier penetration and immunogenicity, while many existing targeted small molecule drugs may affect normal functions of neurons; meanwhile, inhibition of certain key immune pathways inevitably affects normal immune functions of the human body, posing a risk of severe infections. Therefore, RIPK1 kinase is recognized as a highly promising therapeutic target for various degenerative or inflammatory diseases.

The pioneering RIPK1 inhibitor Necrostatin-1 (Nec-1) and its analogues have demonstrated clear efficacy in preclinical studies for a variety of degenerative diseases, inflammation, and cancers. For example, they have shown alleviating effects on Alzheimer's disease (AD), amyotrophic lateral sclerosis (ALS), multiple sclerosis (MS), Parkinson's disease (PD), Huntington's disease (PD), inflammatory bowel diseases, age-related macular degeneration. etc.; protective effects on psoriasis, retinitis pigmentosa, inflammatory bowel diseases, autoimmune diseases, caerulein-induced acute pancreatitis, and sepsis/systemic inflammatory response syndrome (SIRS); effective mitigation of ischemic brain injury, ischemic myocardial injury, retinal ischemia/reperfusion injury, retinal detachment induced photoreceptor necrosis, glaucoma, renal ischemia-reperfusion injury, cisplatin-induced kidney injury, and traumatic brain injury; and at least partial alleviation of other diseases associated with RIPK1-dependent apoptosis, necrosis, or cytokine production, including malignant tumors of blood and solid organs, bacterial and viral infections (including tuberculosis and influenza), and lysosomal storage diseases (particularly Gaucher disease). Currently, several RIPK1 inhibitors are also undergoing clinical trials for treating various degenerative or immune-related diseases. For instance, Sanofi is conducting Phase II clinical trials for SAR443820 in ALS and MS, and SAR443122 in lupus and ulcerative colitis.

However, existing RIPK1 inhibitors all have shortcomings of varying degrees. On one hand, chronic diseases associated with necroptosis, especially degenerative diseases of central nervous system, require a high level of drug safety. For example, SAR443060 from Sanofi, which initially entered Phase I clinical trials, was found to have chronic toxic side effects in primates even after reaching the Phase I endpoints, forcing it to be replaced by SAR443820 for further clinical trials. On the other hand, most RIPK1 inhibitors in clinical trials are analogs of GSK2982772, and their common structural backbone limits the scope for optimizing druggability. These inhibitors often cannot efficiently inhibit RIPK1 in non-primate animals, which also restricts their verification in preclinical disease models and hinders the selection of clinical indications. Finally, RIPK1 inhibitors that both have high activity and brain penetration are very limited, and their long-term safety still requires further verification. Therefore, small-molecule RIPK1 kinase activity inhibitors with a chemical backbone different from GSK2982772, possessing high activity, high specificity, and high blood-brain barrier permeability, remain a major challenge and focus in the development of new drugs for clinical treatment of necroptosis-related diseases. In summary, there is an urgent need in the field for new RIPK1 inhibitors and/or necroptosis inhibitors with novel chemical structures and superior pharmacokinetic and pharmacodynamic profiles, to serve as potential drugs for the prevention and treatment of diseases involving cell death and/or inflammation.

### Summary

An objective of the present disclosure is to provide a class of inhibitors of programmed cell necrosis, to be used as a candidate drug for preventing and treating diseases or conditions associated with programmed cell necrosis and/or human receptor-interacting protein kinase 1 (RIPK1).

A first aspect of the present disclosure provides a compound of Formula I, or a pharmaceutically acceptable salt thereof, a hydrate or a solvate thereof, 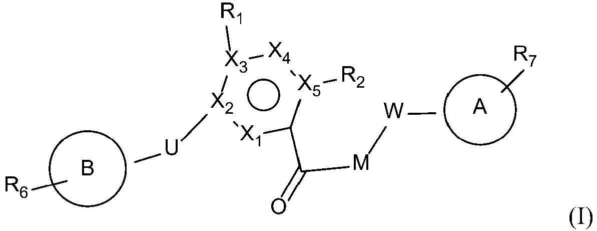 wherein:
X₁ is selected from the group consisting of: CH, N and a chemical bond;
X₂, X₃, X₄ and X₅ are each independently selected from the group consisting of: CH, and N;
with a proviso that a ring formed by X₁, X₂, X₃, X₄ and X₅ is an aromatic ring;
M is selected from the group consisting of: O, S, NR₃, CHR₃ and C(R₃)₂;
W and U are each independently selected from the group consisting of: O, S, NR₄, CHR₄ and C(R₄)₂;
ring A and ring B are each independently selected from the group consisting of: substituted or unsubstituted C6-C10 aryl, and substituted or unsubstituted 5-12 membered heteroaryl;
R₁ and R₂ are each independently selected from the group consisting of: none, H, substituted or unsubstituted C1-C6 alkyl, and halogen;
and when M is NR₃, CHR₃ or C(R₃)₂, R₂, R₃ can form a substituted or unsubstituted 5-7 membered ring together with the C or N atoms they are connected to, as well as -C-C(O)-;
R₃ and R₄ are selected from the group consisting of: H, substituted or unsubstituted C1-C6 alkyl, and halogen;
R₆ is selected from the group consisting of: H, halogen, CN, substituted or unsubstituted C1-C6 alkyl, substituted or unsubstituted C2-C6 alkenyl, substituted or unsubstituted C2-C6 alkynyl, and -CH≡CR₅; wherein, R₅ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
R₇ is selected from the group consisting of: H, halogen, CN, substituted or unsubstituted C1-C6 alkyl, substituted or unsubstituted C2-C6 alkenyl, substituted or unsubstituted C2-C6 alkynyl, and -CH≡CR₈; wherein, R₈ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
wherein, said "substituted" refers to the hydrogen atoms on the group are replaced by one or more (such as 2, 3, 4 and the like) substituents selected from the group consisting of: halogen, C1-C6 alkyl, halogenated C1-C6 alkyl, C3-C8 cycloalkyl, halogenated C3-C8 cycloalkyl, C1-C6 alkoxy, halogenated C1-C6 alkoxy, C1-C6 hydroxyalkyl, methylsulfonyl, -S(=O)₂NH₂, oxo (=O), -CN, hydroxy, -NH₂, carboxyl, C2-C6 acylamino (-C(=O)-N(Rc)₂ or -NH-C(=O) (Rc), Rc is H or C1-C5 alkyl), C1-C6 alkyl-(C2-C6 acylamino), C1-C6 amino, deuterated C1-C6 amino, -NHRd (Rd is C3-C8 cycloalkyl, a 4-7 membered heterocyclic group or a heterocyclic group substituted with C1-C6 alkyl), C6-C10 aryl, a 5-7 membered heteroaryl group having 1-3 heteroatoms selected from N, S and O, a 4-8 membered heterocyclic group having 1-3 heteroatoms selected from N, S and O, and a 4-7 membered heterocyclic group substituted with 1 or 2 Re (Re is halogen, C1-C6 alkyl, C1-C6 amino, -CN, C1-C6 alkoxy or a 4-7 membered heterocyclic group).

In another preferred example, said Formula I has a structure represented by the following Formula II: 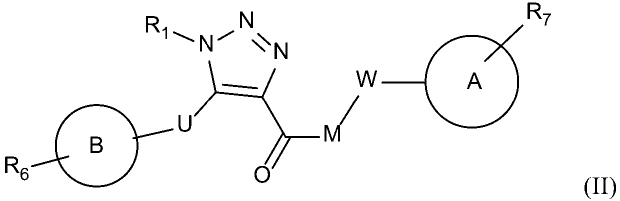 M, W, U, A, B, R₁, R₆, R₇ are as defined above.

In another preferred example, said ring A and ring B are each independently selected from the group consisting of: substituted or unsubstituted phenyl, and substituted or unsubstituted 5-7membered heteroaryl;
said "substituted" is as defined above.

In another preferred example, said ring A and ring B are each independently selected from the group consisting of:

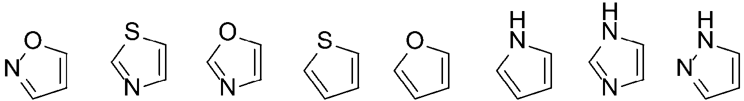

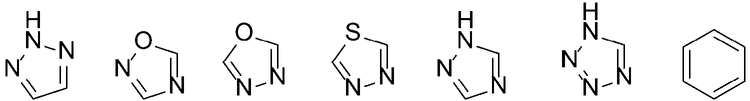

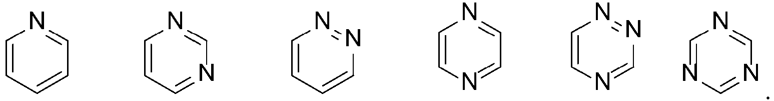

In another preferred example, said ring A is unsubstituted phenyl.

In another preferred example, said ring B is unsubstituted phenyl.

In another preferred example, said M is NR₃, and/or
W is CHR₄;
R₆ is selected from the group consisting of: H, halogen, CN, and substituted or unsubstituted C1-C6 alkyl;
R₇ is selected from the group consisting of: H, halogen, CN, substituted or unsubstituted C1-C6 alkyl, substituted or unsubstituted C2-C6 alkynyl, and -CH≡CR₈; where R₈ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
R₃, R₄, said "substituted" are as defined above.

In another preferred example, said R₇ is selected from the group consisting of: H, halogen, CN, and -CH≡CR₈; where R₈ is selected from the group consisting of: substituted or unsubstituted 5-12 membered nitrogen-containing heteroaryl, and a substituted or unsubstituted 4-8 membered nitrogen-containing heterocyclic group.

In another preferred example, said Formula I has a structure represented by the following Formula III: 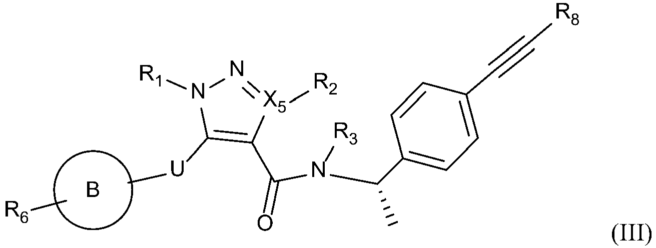 M, W, U, A, B, X₅, R₁, R₂, R₃, R₆, R₈ are as defined above.

In another preferred example, said Formula I has a structure represented by the following Formula II: 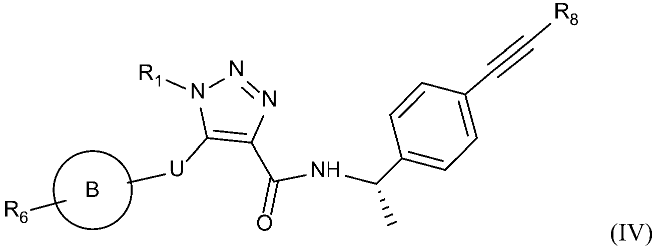 wherein M, W, U, A, B, R₁, R₆, R₈ are as defined above.

In another preferred example, the compound represented by Formula I is selected from those in the following Table:

| Compound No. | Chemical structure | Compound No. | Chemical structure |
|---|---|---|---|
| QY-17-24 | 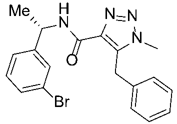 | QY-17-25 | 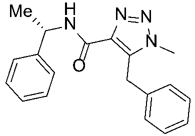 |
| QY-18-15 | 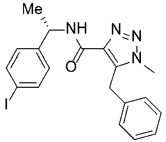 | QY-18-25 | 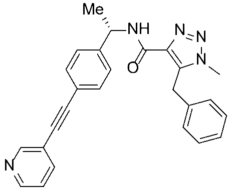 |
| QY-18-26 | 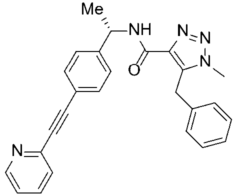 | QY-18-27 | 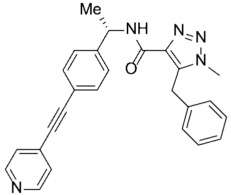 |
| QY-18-28 | 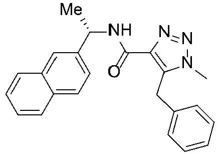 | QY-18-41 | 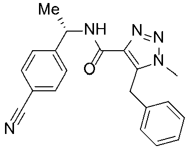 |
| QY-18-42 | 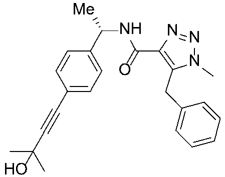 | QY-18-43 | 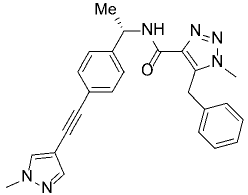 |
| QY-18-44 | 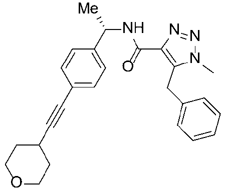 | QY-18-47 | 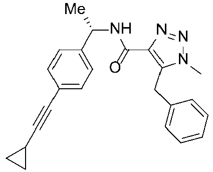 |
| QY-18-49 | 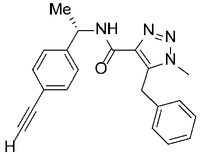 | QY-18-63 | 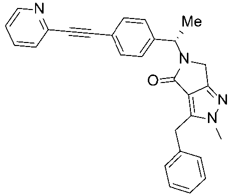 |
| QY-18-77 | 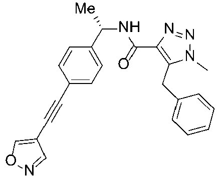 | QY-18-102 | 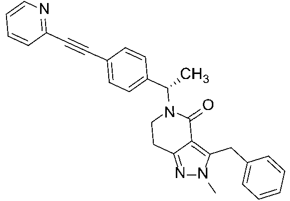 |
| QY-19-53 | 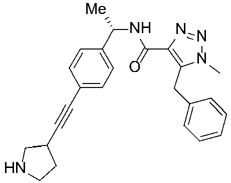 | QY-19-54 | 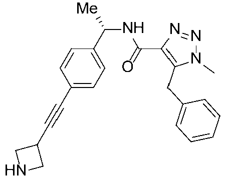 |
| QY-19-55 | 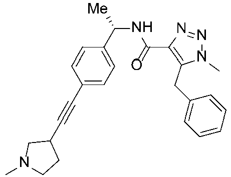 | QY-19-56 | 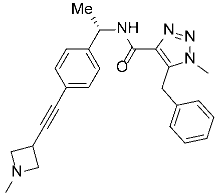 |
| QY-19-60 | 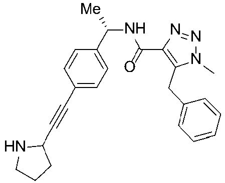 | QY-19-62 | 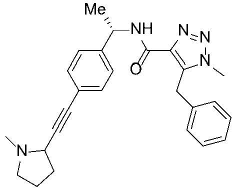 |
| SYL-30-52 | 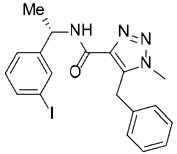 | SYL-30-56 | 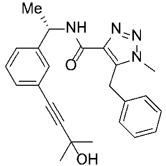 |
| SYL-30-58 | 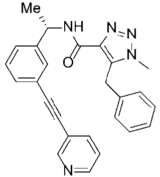 | SYL-30-62 | 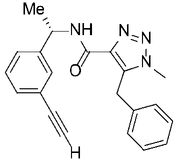 |
| SYL-30-65 | 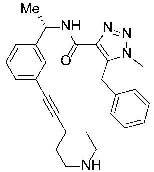 | QY-20-1 | 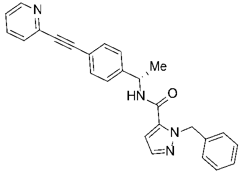 |
| QY-20-2 | 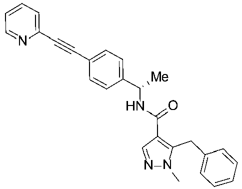 | QY-20-5 | 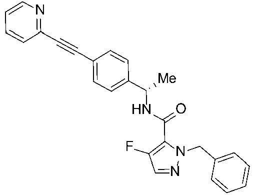 |
| QY-20-18 | 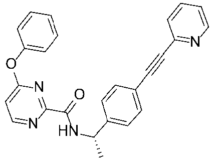 | QY-20-53 | 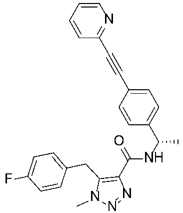 |
| QY-20-54 | 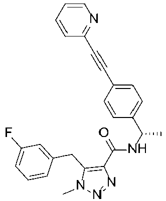 | QY-20-55 | 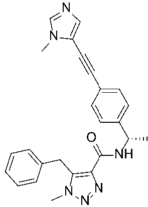 |
| QY-20-67 | 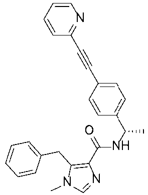 | QY-20-72 | 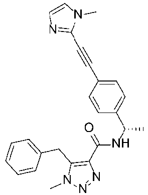 |
| QY-20-73 | 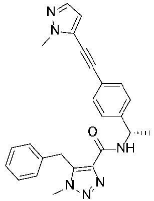 | QY-20-74 | 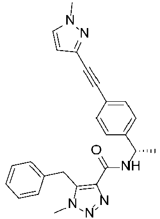 |
| QY-20-76 | 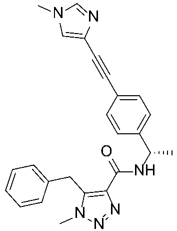 | QY-20-80 | 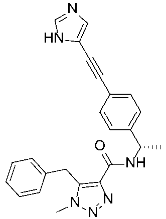 |
| QY-20-81 | 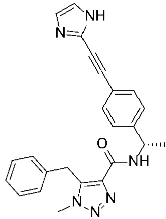 | QY-20-82 | 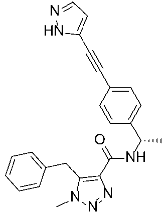 |
| IRCBC-002 | 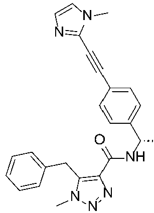 | IRCBC-003 | 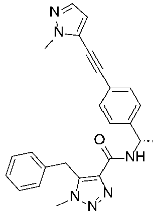 |
| IRCBC-004 | 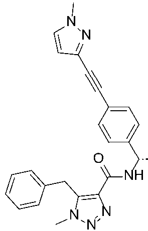 | IRCBC-005 | 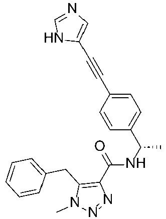 |
| IRCBC-006 | 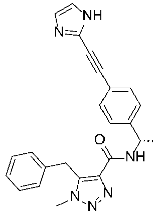 | IRCBC-010 | 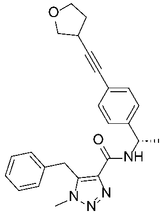 |
| IRCBC-007 | 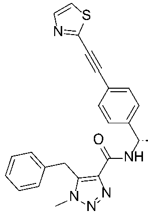 | IRCBC-016 | 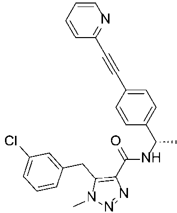 |
| IRCBC-033 | 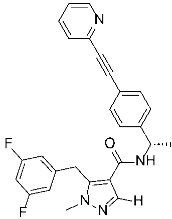 | IRCBC-050 | 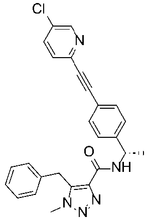 |
| IRCBC-043 | 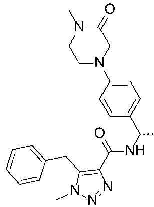 | IRCBC-054 | 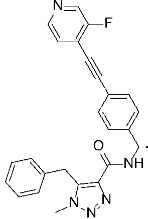 |
| IRCBC-041 | 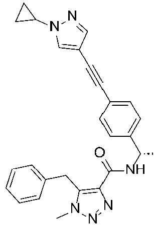 | IRCBC-046 | 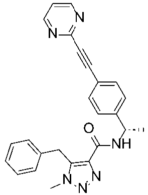 |
| IRCBC-049 | 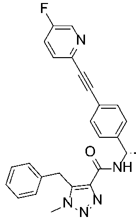 | IRCBC-048 | 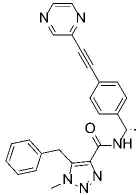 |
| IRCBC-052 | 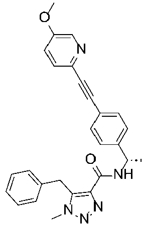 | IRCBC-067 | 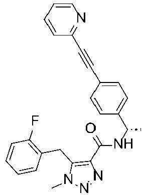 |
| IRCBC-032 | 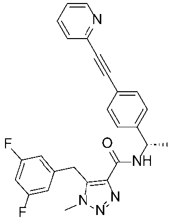 | IRCBC-053 | 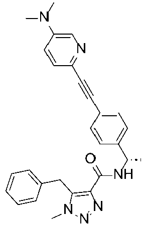 |
| IRCBC-051 | 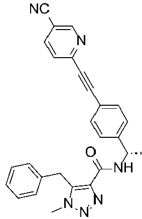 | IRCBC-058 | 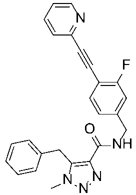 |
| IRCBC-059 | 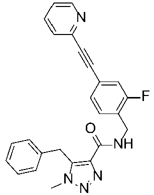 | IRCBC-071 | 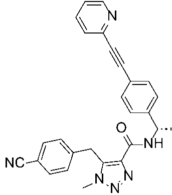 |
| IRCBC-072 | 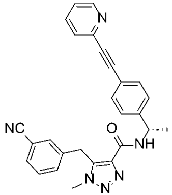 | IRCBC-095 | 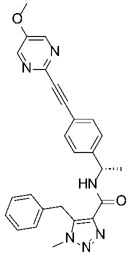 |
| IRCBC-096 | 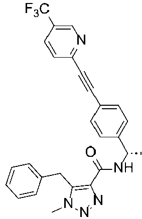 | IRCBC-094 | 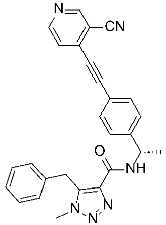 |
| IRCBC-093 | 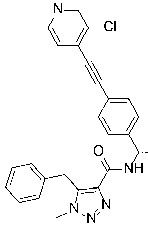 | IRCBC-101 | 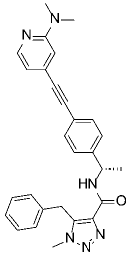 |
| IRCBC-057 | 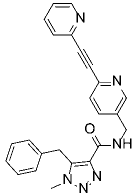 | IRCBC-102 | 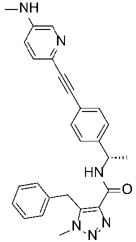 |
| IRCBC-091 | 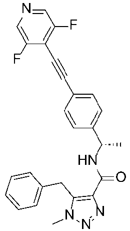 | IRCBC-105 | 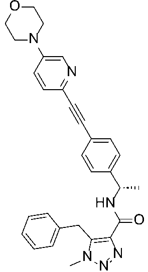 |
| IRCBC-099 | 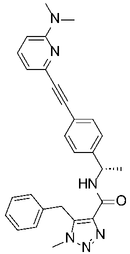 | IRCBC-106 | 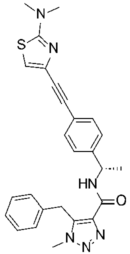 |
| IRCBC-100 | 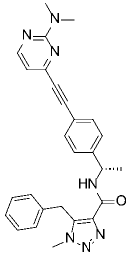 | IRCBC-119 | 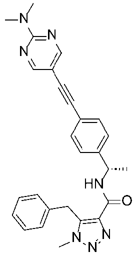 |
| IRCBC-098 | 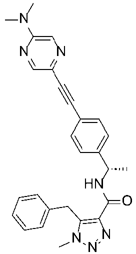 | IRCBC-122 | 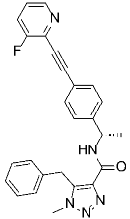 |
| IRCBC-120 | 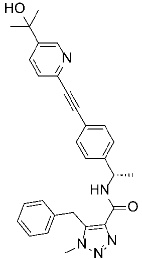 | IRCBC-131 | 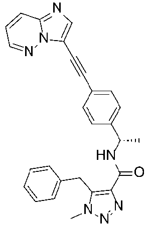 |
| IRCBC-121 | 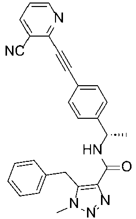 | IRCBC-103 | 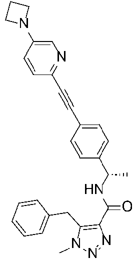 |
| IRCBC-125 | 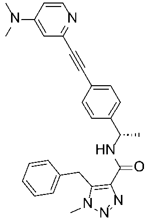 | IRCBC-104 | 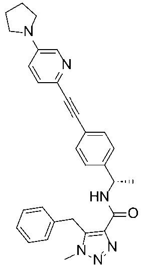 |
| IRCBC-145 | 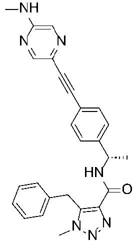 | IRCBC-129 | 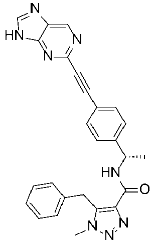 |
| IRCBC-097 | 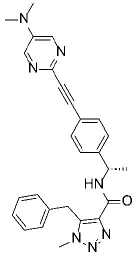 | IRCBC-146 | 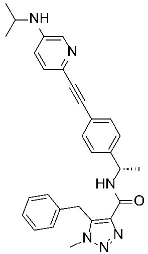 |
| IRCBC-134 | 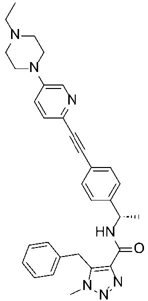 | IRCBC-124 | 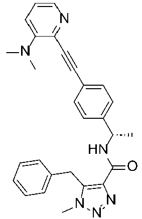 |
| IRCBC-123 | 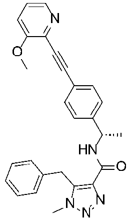 | IRCBC-149 | 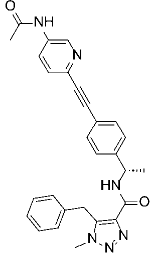 |
| IRCBC-150 | 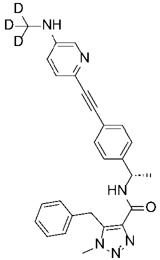 | IRCBC-036 | 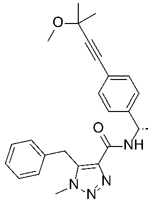 |
| IRCBC-152 | 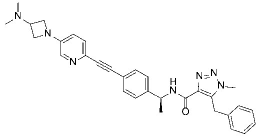 | IRCBC-154 | 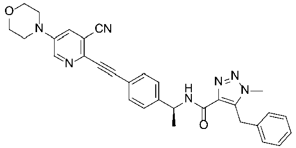 |
| IRCBC-153 | 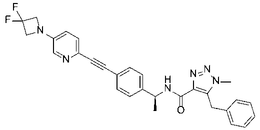 | IRCBC-159 | 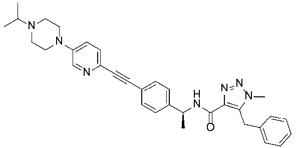 |
| IRCBC-155 | 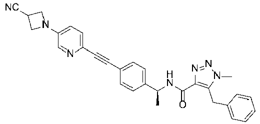 | | |
| IRCBC-156 | 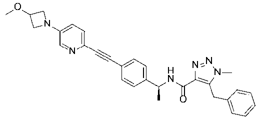 | IRCBC-157 | 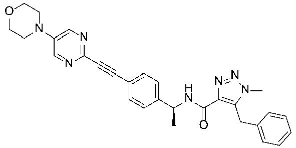 |
| IRCBC-161 | 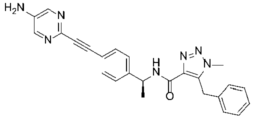 | IRCBC-158 | 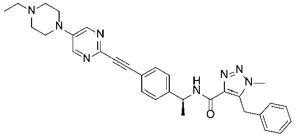 |
| IRCBC-008 | 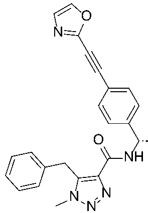 | IRCBC-259 | 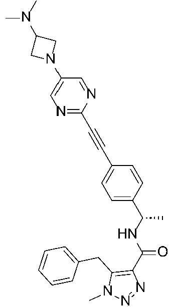 |
| IRCBC-260 | 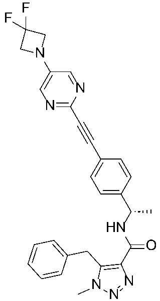 | IRCBC-258 | 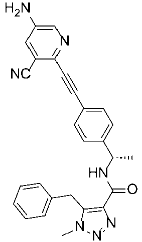 |
| IRCBC-160 | 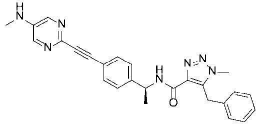 | IRCBC-261 | 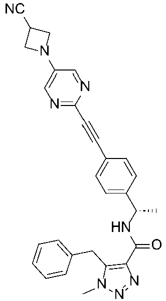 |
| IRCBC-257 | 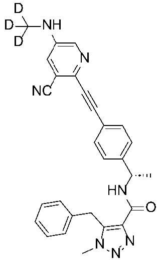 | IRCBC-262 | 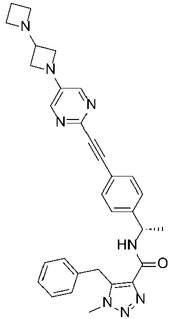 |
| IRCBC-256 | 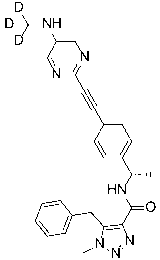 | IRCBC-276 | 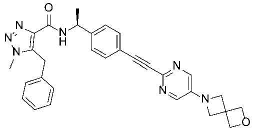 |
| IRCBC-269 | 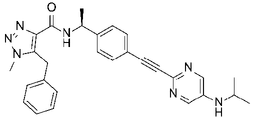 | IRCBC-271 | 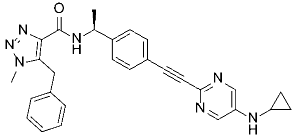 |
| IRCBC-265 | 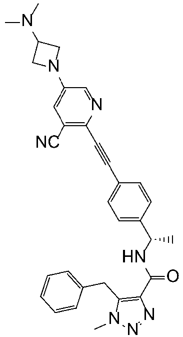 | IRCBC-266 | 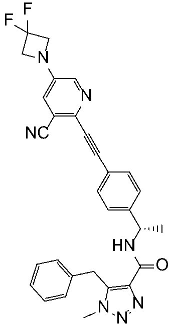 |
| IRCBC-267 | 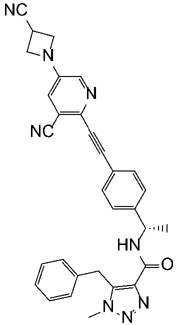 | IRCBC-268 | 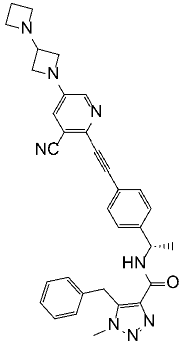 |
| IRCBC-270 | 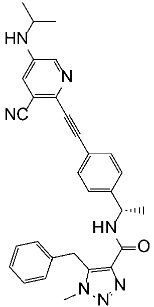 | IRCBC-272 | 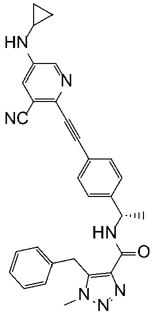 |
| IRCBC-273 | 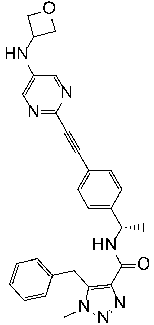 | IRCBC-274 | 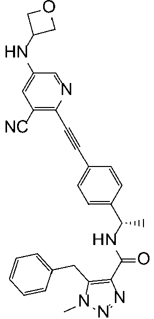 |
| IRCBC-275 | 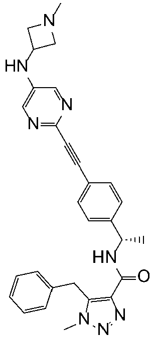 | IRCBC-277 | 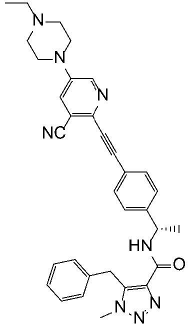 |
| IRCBC-279 | 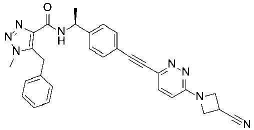 | IRCBC-280 | 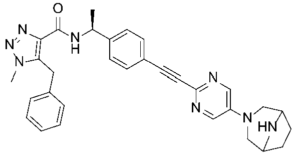 |
| IRCBC-281 | 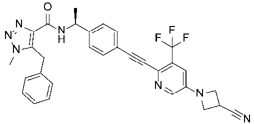 | IRCBC-282 | 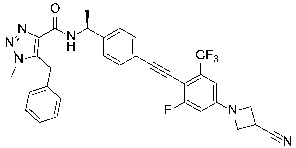 |
| IRCBC-283 | 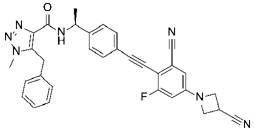 | IRCBC-284 | 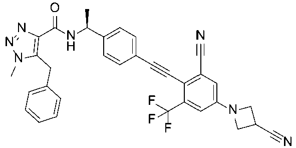 |
| IRCBC-285 | 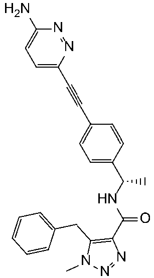 | IRCBC-286 | 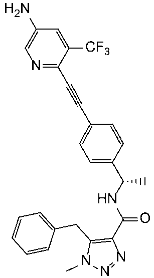 |
| IRCBC-287 | 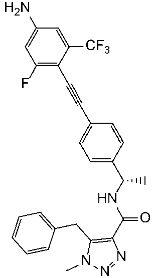 | IRCBC-288 | 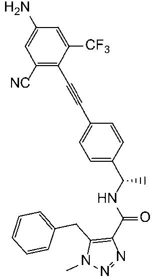 |
| IRCBC-289 | 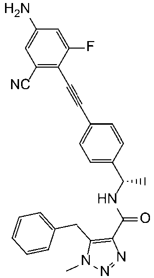 | IRCBC-290 | 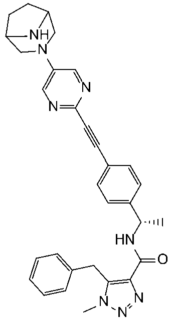 |
| IRCBC-291 | 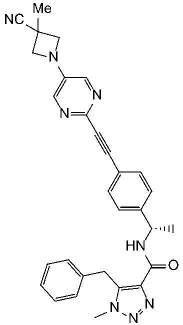 | IRCBC-292 | 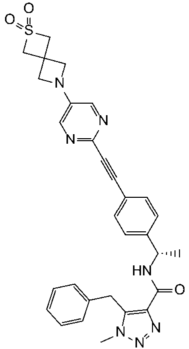 |
| IRCBC-293 | 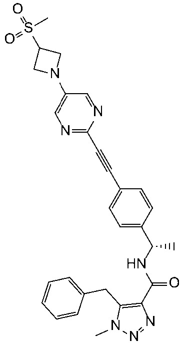 | IRCBC-294 | 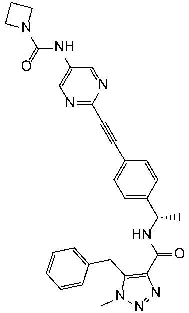 |
| IRCBC-295 | 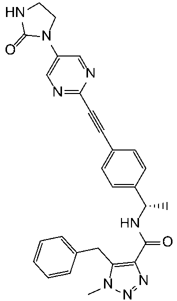 | IRCBC-296 | 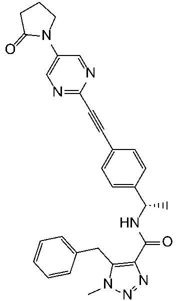 |

A second aspect of the present disclosure provides a pharmaceutical composition comprising (a) a therapeutically effective amount of the compound according to the first aspect of the present disclosure, or a pharmaceutically acceptable salt, a hydrate, or a solvate thereof, and (b) a pharmaceutically acceptable carrier.

A fourth aspect of the present disclosure provides use of the compound according to the first aspect, or the pharmaceutical composition according to the third aspect in the manufacture of drugs for treating or preventing a disease or condition associated with programmed cell necrosis and/or human receptor-interacting protein kinase 1 (RIPK1).

In another preferred example, said compound or pharmaceutical composition treats or prevents the disease or condition by inhibiting human receptor-interacting protein kinase 1 (RIPK1).

In another preferred example, said human receptor-interacting protein kinase 1 (RIPK1) includes deactive (or non-activated) RIPK1 and activated RIPK1.

In another preferred example, said compound or pharmaceutical composition may also treat or prevent (especially treat) the disease or condition by inhibiting human receptor-interacting protein kinase 1 (RIPK1) in its activated state.

In another preferred example, said inhibiting human receptor-interacting protein kinase 1 (RIPK1) includes one or more of the following: inhibiting the activity of RIPK1, or inhibiting the phosphorylation of RIPK1.

In another preferred example, said compound or pharmaceutical composition treats or prevents the disease or condition by inhibiting programmed cell necrosis signaling pathway.

In another preferred example, said inhibiting programmed cell necrosis signaling pathway includes one or more of the following: inhibiting the activity of RIPK1, inhibiting the phosphorylation of RIPK1 or inhibiting the phosphorylation of MLKL.

In another preferred example, the disease or condition is one or more selected from the group consisting of: degenerative diseases, inflammation, ischemia-reperfusion injury, pathogenic infections, Parkinson's disease (PD), age-related macular degeneration, autoimmune diseases, retinal detachment induced photoreceptor necrosis, glaucoma, cisplatin-induced kidney injury and traumatic brain injury, atherosclerosis caused by hyperlipidemia, other diseases associated with RIPK1-dependent apoptosis, necrosis, or cytokine production, bacterial infections, viral infections, and lysosomal storage disorders.

In another preferred example, said degenerative diseases includes, for example, Alzheimer's disease (AD), multiple sclerosis (MS), amyotrophic lateral sclerosis (ALS), retinal degenerative diseases.

In another preferred example, said inflammation includes one or more of the following: enteritis, rheumatoid arthritis, psoriasis, retinitis pigmentosa, inflammatory bowel diseases, Huntington's disease (PD), inflammatory bowel diseases, caerulein-induced acute pancreatitis, sepsis/systemic inflammatory response syndrome (SIRS).

In another preferred example, said ischemia-reperfusion injury includes one or more of the following: cerebral infarction, myocardial infarction, ischemic brain injury, ischemic myocardial injury, retinal ischemia/reperfusion injury, renal ischemia-reperfusion injury.

In another preferred example, said other diseases associated with RIPK1-dependent apoptosis, necrosis, or cytokine production include one or more of the following: malignant tumors of blood and solid organs.

In another preferred example, said viral infections include one or more of the following diseases or conditions: tuberculosis, influenza, coronavirus infections, and pneumonia caused thereby.

In another preferred example, said lysosomal storage disorders include Gauchers diseases.

A fourth aspect of the present disclosure provides a method for treating or preventing diseases or conditions associated with programmed cell necrosis and/or human receptor-interacting protein kinase 1 (RIPK1), which comprises: administering to a subject in need thereof a therapeutically effective amount of the compound according to the first aspect or the pharmaceutical composition according to the second aspect.

A fifth aspect of the present disclosure provides a method for inhibiting programmed cell necrosis, comprising a step of: culturing cells in the presence of the compound according to the first aspect, thereby inhibiting programmed cell necrosis.

In another preferred example, said method is in vitro for non-treatment purpose.

A sixth aspect of the present disclosure provides a method for inhibiting RIPK1 protein kinase activity, comprising the step of: contacting RIPK1 protein kinase with the compound according to the first aspect, thereby inhibiting RIPK1 protein kinase activity.

In another preferred example, said method is in vitro for non-treatment purpose.

It should be understood that, within the scope of the present disclosure, the above technical features of the present disclosure and the technical features particularly described below (such as in the examples) can be combined with each other, thereby forming new or preferred technical solutions. Due to space limitations, they will not be enumerated one by one herein.

### Description of the Drawings

Fig.1 shows the effect of a representative compound QY-18-26 on RIPK1 kinase activity.
Fig.2 shows the pharmacokinetic properties of a representative compound QY-18-26.
Fig.3 shows the brain penetration properties of a representative compound QY-18-26.
Fig.4 shows the effect of a representative compound QY-18-26 on TNFα-induced systemic inflammatory response syndrome.

### Detailed Description

After long-term and in-depth research, the inventors unexpectedly discovered a class of programmed cell necrosis inhibitors with a novel structure. The programmed cell necrosis inhibitors exhibit excellent RIPK1 inhibitory activity and thus can be used to prepare pharmaceutical compositions for preventing and/or treating diseases associated with cell death, RIPK1 and/or inflammation. In particular, the preferred compounds provided by the present disclosure also exhibit excellent inhibitory activity against activated RIPK1. Thus, compared to the existing RIPK1 inhibitors that only inhibit deactive RIPK1, the compounds provided by the present disclosure can more rapidly improve or treat diseases or conditions (such as inflammation) associated with cell death and/or RIPK1-mediated inflammation. Based on the above findings, the inventors completed the present disclosure.

### Terms

Unless explicitly indicated otherwise, the terms according to the present disclosure and used herein have the following meanings:

The term "alkyl" refers to a linear or branched alkyl group with a specified number of carbon atoms. For example, C1-C6 alkyl refers to a linear or branched alkyl group with 1 to 6 carbon atoms. Examples of alkyl groups include, but are not limited to: methyl, ethyl, propyl, isopropyl, butyl, isobutyl, sec-butyl, tert-butyl, and the like.

The term "cycloalkyl" refers to a cyclic alkyl group with a specified number of carbon atoms. For example, "C3-C8 cycloalkyl" refers to a cyclic alkyl group having 1 to 8 carbon atoms. Examples of cycloalkyl groups include, but are not limited to: cyclopropyl, cyclobutyl, cyclopentyl, cyclohexyl, cycloheptyl, and cyclooctyl.

The term "alkoxy" refers to the alkyl as defined above connected to the rest of a molecule through an oxygen atom. For example, C1-C6 alkoxy refers to C1-C6 alkyl-O-.

The term "halogen" refers to F, Cl, Br or I.

The term "halogenated alkyl" refers to alkyl substituted with halogen (alkyl is as defined above).

The term "aryl" refers to a polyunsaturated (usually aromatic) hydrocarbon group with a specified number of ring atoms, which can be a single ring or fused or covalently connected multiple rings (such as bicyclic group). Examples of aryl groups include, but are not limited to, phenyl and naphthyl.

The term "heteroaryl" refers to an aryl group (or ring) that contains a specified number of ring atoms and 1 to 5 (for example, 1, 2, 3, 4, or 5) heteroatoms selected from N, O, and S, where the nitrogen and sulfur atoms may be optionally oxidized, and the nitrogen atoms may be optionally quaternized. For example, 5-10 membered heteroaryl (or ring) refers to a heteroaryl group (or ring) containing 5, 6, 7, 8, 9, or 10 ring atoms. As used herein, nitrogen-containing heteroaryl refers to heteroaryl containing at least one nitrogen heteroatom. Heteroaryl can be connected to the rest of a molecule through heteroatoms. Examples of heteroaryl include, but are not limited to: pyridyl, pyridazinyl, pyrazinyl, pyrimidinyl, triazinyl, quinolyl, quinoxalinyl, quinazolinyl, cinnolinyl, phthalazinyl, benzotriazinyl, purinyl, benzimidazolyl, benzopyrazolyl, benzotriazolyl, benzisoxazolyl, isobenzofuryl, isoindolyl, indolizinyl, benzotriazinyl, thienopyridinyl, thienopyrimidinyl, pyrazolopyrimidinyl, imidazopyridinyl, benzothiazolyl, benzofuranyl, benzothienyl, indolyl, quinolyl, isoquinolyl, isothiazolyl, pyrazolyl, indazolyl, pteridyl, imidazolyl, triazolyl, tetrazolyl, oxazolyl, isoxazolyl, thiadiazolyl, pyrrolyl, thiazolyl, furanyl, thienyl.

An "pharmaceutically acceptable" ingredient refers to a substance suitable for use in humans and/or animals that does not cause excessive adverse effects (such as toxicity, irritation, and hypersensitivity), i.e., a substance having a reasonable benefit/risk ratio.

"Effective amount" refers to the amount of a therapeutic agent that treats, alleviates, or prevents a target disease or condition, or that exhibits a detectable therapeutic or preventive effect. The precise effective amount for a particular subject depends on the size and health condition of the subject, the nature and severity of the disease, and the choice of the therapeutic agent and/or combinations of the therapeutic agent administered. Therefore, it is not useful to specify an exact effective amount in advance. However, for a given condition, the effective amount can be determined through routine experiments, which can be assessed by a clinician.

Unless otherwise specified, in the present disclosure, all compounds mentioned are intended to include all possible optical isomers, such as a compound with single chirality or mixtures of various chiral compounds (i.e., racemates). In all compounds of the present disclosure, each chiral carbon atom may optionally be of R configuration or S configuration, or a mixture of R and S configurations.

The term "the compound of the present disclosure" refers to the compound represented by Formula I. This term also includes various crystalline forms of the compound of Formula I, pharmaceutically acceptable salts, hydrates, or solvates thereof.

The term "pharmaceutically acceptable salt" refers to a salt formed by the compound of the present disclosure with an acid or a base that is suitable for use as a drug. Pharmaceutically acceptable salts include both inorganic and organic salts. A preferred class of salts is those formed by the compound of the present disclosure with an acid. Suitable acids for forming a salt include, but are not limited to: inorganic acids such as hydrochloric acid, hydrobromic acid, hydrofluoric acid, sulfuric acid, nitric acid, phosphoric acid, etc.; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, toluenesulphonic acid, benzenesulfonic acid, etc.; as well as acidic amino acids such as aspartic acid, glutamic acid, etc.

The term "solvate" refers to a complex formed by coordination of the compound of the present disclosure with solvent molecules in a specific ratio. 'Hydrate' refers to a complex formed by coordination of the compound of the present disclosure with water.

The present disclosure will be further illustrated in conjunction with specific examples. It should be understood that these examples are intended to illustrate the present disclosure and not to limit the scope of the present disclosure. In the following examples, experimental methods without specified conditions are generally carried out under conventional conditions or according to the conditions recommended by the manufacturer. Unless otherwise stated, percentages and parts are by weight.

### Synthesis Example 1

### Method 1:

### Synthesis of Compound QY-12-88:

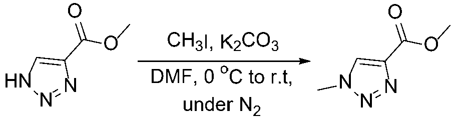

**Methyl** 1-methyl-1H-1,2,3-triazole-4-formate (QY-12-88): Methyl 1,2,3-triazole-4-formate (1.27 g, 10.0 mmol) was dissolved in 20 mL of N,N-dimethylformamide in a 100 mL round-bottom flask and added with potassium carbonate (0.83 g, 6.0 mmol) at room temperature. Iodomethane (1.50 g, 10.5 mmol) was slowly added to the reaction liquid under an ice bath. After being stirred for 1 h, the reaction liquid was gradually returned to room temperature and then continuously stirred for 16 h. The reaction was stopped until the substrate was completely consumed under real-time LC-MS monitoring. After the organic solvent was removed by rotary evaporation, the reaction liquid was diluted with dichloromethane, transferred to a separatory funnel, washed with distilled water (10 mL × 3), and extracted with dichloromethane (20 mL × 3). The organic phases were combined and washed with saturated sodium chloride solution (10 mL × 2). The organic phase was dried over anhydrous sodium sulfate, filtered and evaporated using a rotary evaporator to remove dichloromethane. The product was purified by silica gel column chromatography (PE: EA = 10-90%) to afford 305 mg of white powdery solid, with a yield of 22%. ESI-MS *m*/*z* 142.1 (M+H)⁺.

### Synthesis of Compound QY-12-95:

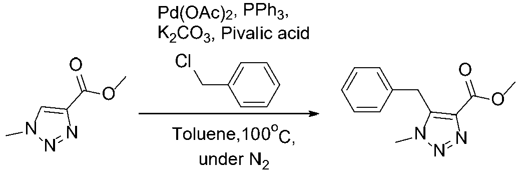

**Methyl 5-benzyl-1-methyl-1H-1,2,3-triazole-4-formate (QY-12-95):** In a 15 mL pressure-resistant bottle, 5 mL of toluene, QY-12-88 (305 mg, 2.16 mmol) were added and stirred evenly. Then, palladium acetate (24 mg, 0.11 mmol), triphenylphosphine (113 mg, 0.43 mmol), potassium carbonate (746 mg, 5.40 mmol), pivalic acid (66 mg, 0.65 mmol) were added, and benzyl chloride (274 mg, 2.16 mmol) was added dropwise. Under nitrogen protection, the reaction liquid was heated to 100°C and reacted overnight. After the reactants were completely consumed, toluene was removed by rotary evaporation. The reaction liquid was diluted with ethyl acetate, suction filtered through diatomaceous earth, and then evaporated using a rotary evaporator to remove the organic solvent. The product was purified by silica gel column chromatography (PE: EA = 0-80%) to afford 173 mg of a pale yellow transparent oily liquid, with a yield of 35%.ESI-MS *m*/*z* 232.1 (M+H)⁺.

### Synthesis of Compound QY-13-15:

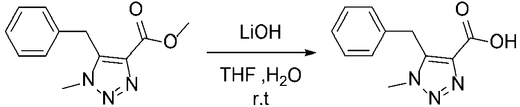

**5-benzyl-1-methyl-1H-1,2,3-triazole-4-formic acid (QY-13-15):** QY-12-95 (173 mg, 0.75 mmol) was dissolved in 3 mL of tetrahydrofuran and transferred to an 8 mL pressure-resistant bottle. Lithium hydroxide monohydrate (63 mg, 1.50 mmol) was dissolved in 0.8 mL of distilled water and added dropwise to the reaction liquid at room temperature. The reaction progress was monitored by real-time LC-MS. After 8 hours, the reactants were completely consumed. All solvents were removed from the reaction system by rotary evaporation. The crude product could be used directly for subsequent synthesis without purification. ESI-MS *m*/*z* 218.0 (M+H)⁺.

### Synthesis of Compound QY-13-17:

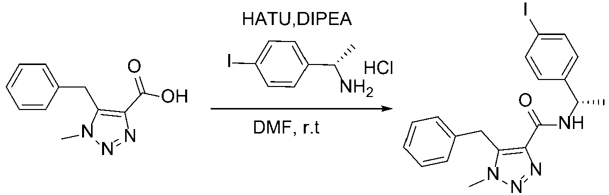

**(S)-5-benzyl-N-(1-(4-iodophenyl)ethyl)-1-methyl-1H-1,2,3-triazole-4-formamide (QY-13-17): QY-13-15** (0.75 mmol) was dissolved in 5 mL of N,N-dimethylformamide in a 15 mL pressure-resistant bottle. HATU (341 mg, 0.90 mmol) and 4-iodobenzylamine (174 mg, 0.75 mmol) were added, and N,N-diisopropylethylamine (289 mg, 2.24 mmol) was added dropwise at room temperature, with stirring continuously at room temperature. The reaction was monitored by real-time LC-MS. After the reaction was complete, the reaction liquid was diluted with 15 mL of dichloromethane, transferred to a separatory funnel, washed with 10 mL of distilled water, and extracted with dichloromethane (10 mL × 3). The combined organic phases were washed with saturated sodium chloride solution (10 mL × 2). The resultant organic phase was dried over anhydrous sodium sulfate, filtered, and evaporated using a rotary evaporator to remove dichloromethane. The product was purified by silica gel column chromatography (PE: EA = 0-100%) to afford 153 mg of a white foamy solid, with a yield of 47%. ESI-MS *m*/*z* 447.0 (M+H)⁺.

### Synthesis of Compound QY-18-26:

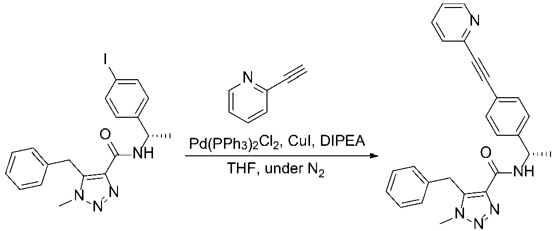

**(S)-5-benzyl-1-methyl-N-(1-(4-(pyridin-2-ylethynyl)phenyl)ethyl)-1H-1,2,3-triazole-4-formamide (QY-18-26):** In an 8 mL pressure-resistant bottle, 1 mL of tetrahydrofuran and **QY-13-15** (32 mg, 0.072 mmol) were added in sequence and stirred until they were completely dissolved. Then 2-ethynylpyridine (11 mg, 0.11 mmol), cuprous iodide (2.7 mg, 0.014 mmol), N,N-diisopropylethylamine (37 mg, 0.288 mmol), and bis(triphenylphosphine)palladium(II) chloride (5 mg, 0.007 mmol) were added. The reaction liquid was stirred at room temperature under nitrogen protection. After the reactants were completely consumed, the reaction liquid was suction filtered through diatomaceous earth, and evaporated using a rotary evaporator to remove the organic solvent. The product was purified by silica gel column chromatography (PE:EA = 10-80%) to afford 10 mg of a yellow-brown oily liquid, with a yield of 33%. ESI-MS *m*/*z* 422.2 (M+H)⁺.

### Method 2:

### Synthesis of Compound QY-16-97:

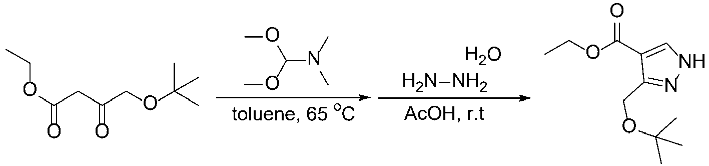

**Ethyl 3-(tert-butyloxymethyl)-1H-pyrazole-4-formate (QY-16-97):** Ethyl 4-(tert-butoxy)-3-oxobutanoate (2.98 g, 14.7 mmol) and dimethoxy-N,N-dimethylmethanamine (2.45 g, 20.6 mmol) were mixed uniformly in 25 mL of toluene, heated to 65°C, and stirred overnight for reaction, then the organic solvent was removed directly by rotary evaporation. 9 mL of glacial acetic acid was added to dissolve the reaction system, and hydrazine hydrate (1.03 g, 20.6 mmol) was slowly added to the reaction liquid with stirring at room temperature. The reaction liquid was stirred overnight at room temperature. The reaction liquid was diluted with 250 mL of ethyl acetate, transferred to a separatory funnel, washed with saturated sodium bicarbonate solution (50 mL × 2), washed with distilled water (10 mL × 2), washed with saturated sodium chloride solution (10 mL × 2), dried over anhydrous sodium sulfate, filtered and evaporated using a rotary evaporator to remove the organic solvent. The product was purified by silica gel column chromatography (PE: EA = 0-60%) to afford 1.76 g of an orange-yellow oily liquid, with a yield of 53%. ESI-MS *m*/*z* 227.1 (M+H)⁺.

### Synthesis of Compound QY-16-98:

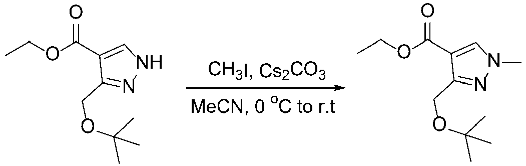

**Ethyl 3-(*tert*-butyloxymethyl)-1-methyl-1H-pyrazole-4-formate (QY-16-98):** In a 100 mL round-bottom flask, QY-16-97 (1.76 g, 7.8 mmol) was dissolved in 35 mL of acetonitrile, and added with cesium carbonate (6.34 g, 19.5 mmol) at room temperature. Iodomethane (1.32 g, 9.3 mmol) was slowly added to the reaction liquid under an ice bath. The reaction liquid was gradually returned to room temperature and then continuously stirred for 10 h. The reaction was stopped until the substrate was completely consumed under real-time LC-MS monitoring. After the organic solvent was removed by rotary evaporation, the reaction liquid was diluted with ethyl acetate, transferred to a separatory funnel, washed with distilled water (10 mL × 2), washed with saturated sodium chloride solution (10 mL × 2). The organic phase was dried over anhydrous sodium sulfate, filtered and evaporated using a rotary evaporator to remove dichloromethane. The product was purified by silica gel column chromatography (PE:EA = 0-80%) to afford 624 mg of a pale yellow transparent oily liquid, with a yield of 33%. ESI-MS *m*/*z* 241.2 (M+H)⁺.

### Synthesis of Compound QY-17-11:

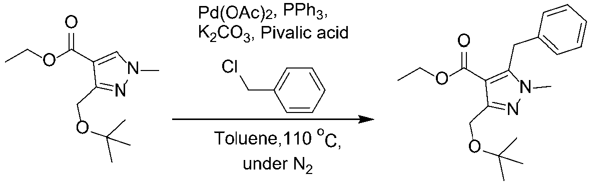

**Ethyl 5-benzyl-3-(tert-butyloxymethyl)-1-methyl-1H-pyrazole-4-formate (QY-17-11):** In a 30 mL pressure-resistant bottle, 10 mL of toluene, QY-16-98 (603 mg, 2.51 mmol) were added and stirred evenly. Then, palladium acetate (56 mg, 0.25 mmol), triphenylphosphine (262 mg, 1.0 mmol), potassium carbonate (1214 mg, 8.79 mmol), and pivalic acid (77 mg, 0.75 mmol) were added, and benzyl chloride (413 mg, 3.26 mmol) was added dropwise. Under nitrogen protection, the reaction mixture was heated to 110°C and reacted overnight. After the reactants were completely consumed, toluene was removed by rotary evaporation. The reaction solution was diluted with ethyl acetate, suction filtered through diatomaceous earth, and then evaporated using a rotary evaporator to remove the organic solvent. The product was purified by silica gel column chromatography (PE:EA = 0-50%) to afford 108 mg of a pale yellow transparent oily liquid, with a yield of 13%. ESI-MS *m*/*z* 331.1 (M+H)⁺.

### Synthesis of Compound QY-17-54:

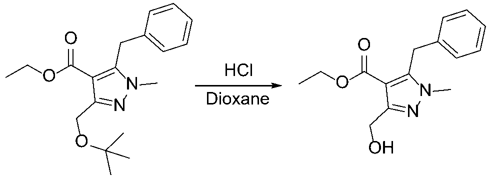

**Ethyl 5-benzyl-3-(hydroxymethyl)-1-methyl-1H-pyrazole-4-formate (QY-17-54): QY-17-11** (108 mg, 0.33 mmol) was directly dissolved in 2 mL of a solution of hydrochloric acid in dioxane. After stirring at room temperature for 4 hours, the reaction was complete. The product was separated and purified by C18 reverse-phase chromatography column (CH₃CN: H₂O = 10-90%) to afford 60 mg of a light yellow transparent oily liquid, with a yield of 66%. ESI-MS *m*/*z* 275.1 (M+H)⁺.

### Synthesis of Compound QY-17-69:

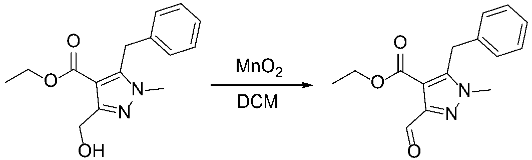

**Ethyl 5-benzyl-3-formyl-1-methyl-1H-pyrazole-4-formate (QY-17-69): QY-17-54** (86 mg, 0.31 mmol) was directly dissolved in 4 mL of dichloromethane, and added with manganese dioxide powder (136 mg, 1.57 mmol). The mixture was heated to reflux and stirred overnight. The reaction was monitored by LC-MS. After the reaction was complete, the reaction liquid was filtered with a membrane to remove solids, and dichloromethane was removed by rotary evaporation. The resultant pale yellow oily liquid product could be used directly in the next step without further purification. ESI-MS *m*/*z* 273.1 (M+H)⁺.

### Synthesis of Compound QY-18-57:

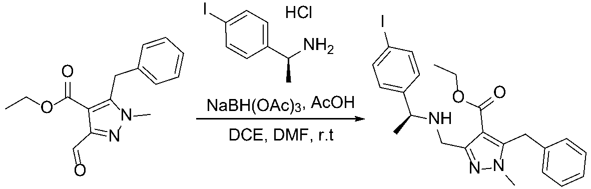

**Ethyl (S)-5-benzyl-3-(((1-(4-iodophenyl)ethyl)amino)methyl)-1-methyl-1H-pyrazole-4-formate (QY-18-57): QY-17-69** (0.169 mmol) was mixed into 3 mL of 1,2-dichloroethane and added to a 15 mL pressure-resistant bottle with stirring. (S)-1-(4-iodophenyl)ethan-1-amine hydrochloride (62 mg, 0.22 mmol), glacial acetic acid (3 drops), and sodium triacetoxyborohydride (90 mg, 0.42 mmol) were added and stirred at room temperature overnight. The reaction was stopped until the reaction was complete under LC-MS monitoring. The organic solvent was removed by rotary evaporation. After the reaction system was mixed well in dimethyl sulfoxide and filtered through a membrane, the product was separated and purified by C18 reverse-phase chromatography column (CH₃CN: H₂O = 10-90%) to afford 73 mg of a colorless transparent oily liquid, with a yield of 86%. ESI-MS *m*/*z* 504.1 (M+H)⁺.

### Synthesis of Compound QY-18-59:

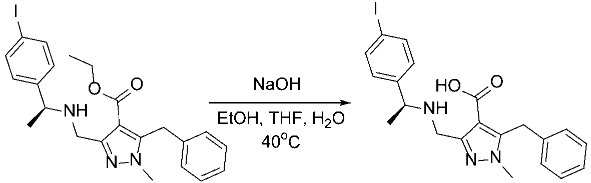

**(S)-5-benzyl-3-(((1-(4-iodophenyl)ethyl)amino)methyl)-1-methyl-1H-pyrazole-4-formic acid (QY-18-59): QY-18-57** (73 mg, 0.145 mmol) was dissolved in 2 mL of ethanol and 2 mL of tetrahydrofuran, and transferred to a 15 mL pressure-resistant bottle. Sodium hydroxide (58 mg, 1.45 mmol) was dissolved in 1.0 mL of distilled water, and added dropwise to the reaction liquid at room temperature. The reaction liquid was heated to 40 °C and stirred overnight. The reaction was stopped until the reaction was complete under LC-MS monitoring. The reaction system was acidified to a weakly acidic pH with 2N hydrochloric acid, filtered with a membrane, separated and purified by C18 reverse-phase chromatography column (CH₃CN: H₂O = 0-80%) to afford 46 mg of a colorless transparent oily liquid, with a yield of 67%. ESI-MS *m*/*z* 476.0 (M+H)⁺.

### Synthesis of Compound QY-18-62:

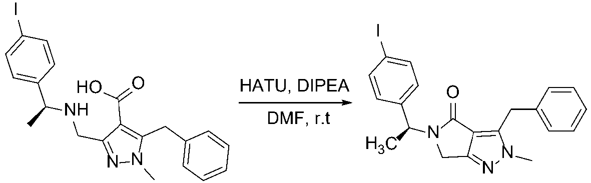

**(S)-3-benzyl-5-(1-(4-iodophenyl)ethyl)-2-methyl-5,6-dihydropyrrolo[3,4-c]pyrazol-4(2H)-one (QY-18-62): QY-18-59** (46 mg, 0.097 mmol) was dissolved in 2.5 mL of N,N-dimethylformamide in a 15 mL pressure-resistant bottle, followed by the addition of HATU (44 mg, 0.116 mmol). N,N-diisopropylethylamine (50 mg, 0.388 mmol) was added dropwise at room temperature, with continuously stirring at room temperature. The reaction was monitored by real-time LC-MS. After the reaction was complete, trifluoroacetic acid was added to adjust the reaction liquid to a weakly acidic pH. The reaction liquid was filtered through a membrane, and separated and purified by C18 reverse-phase chromatography column (CH₃CN: H₂O = 10-80%) to obtain 27 mg of a colorless, transparent oily liquid, with a yield of 61%. ESI-MS *m*/*z* 457.9 (M+H)⁺.

### Synthesis of Compound QY-18-63:

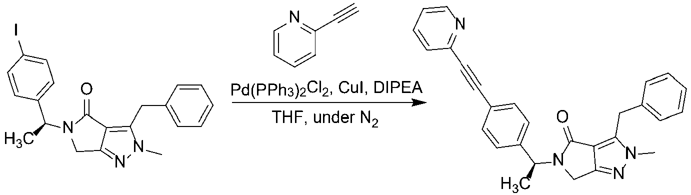

**(S)-3-benzyl-2-methyl-5-(1-(4-(pyridin-2-ylethynyl)phenyl)ethyl)-5,6-dihydropyrrolo[3,4-c]pyrazol-4(2H)-one (QY-18-63):** In a 15 mL pressure-resistant bottle, 1 mL of tetrahydrofuran, **QY-18-62** (46 mg, 0.097 mmol) were added in sequence and stirred until they were completely dissolved. Then 2-ethynylpyridine (9.1 mg, 0.089 mmol), cuprous iodide (2.2 mg, 0.012 mmol), N,N-diisopropylethylamine (50 mg, 0.388 mmol), and bis(triphenylphosphine)palladium(II) chloride (4.1 mg, 0.006 mmol) were added. The reaction liquid was stirred at room temperature under nitrogen protection. After the reactants were

completely consumed, the reaction liquid was suction filtered through diatomaceous earth, and evaporated using a rotary evaporator to remove the organic solvent. The product was separated and purified by silica gel column chromatography (PE: EA = 10-100%) to afford 21 mg of a yellow-brown oily liquid, with a yield of 82%. ESI-MS *m*/*z* 433.2 (M+H)⁺.

The compounds shown in Table 1 were obtained by replacing different synthetic substrates according to the above method:

**Table 1**

| | | | |
|---|---|---|---|
| QY-17-24 | 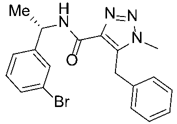 | QY-17-25 | 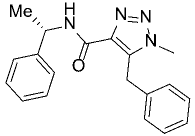 |
| QY-18-15 | 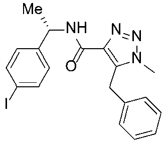 | QY-18-25 | 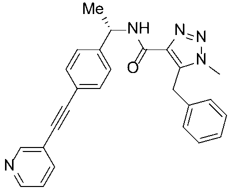 |
| QY-18-26 | 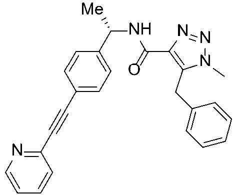 | QY-18-27 | 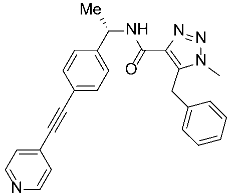 |
| QY-18-28 | 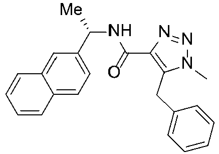 | QY-18-41 | 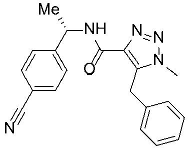 |
| QY-18-42 | 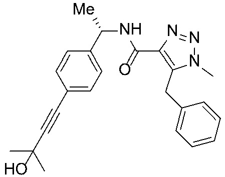 | QY-18-43 | 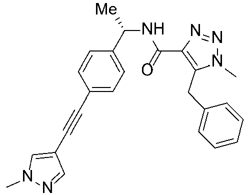 |
| QY-18-44 | 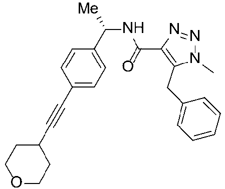 | QY-18-47 | 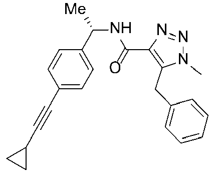 |
| QY-18-49 | 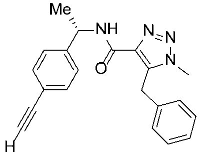 | QY-18-63 | 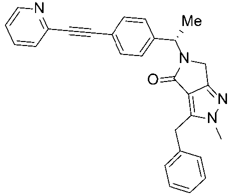 |
| QY-18-77 | 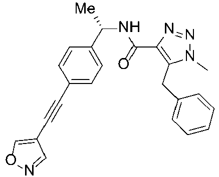 | QY-18-102 | 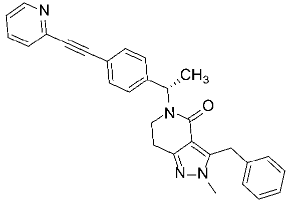 |
| QY-19-53 | 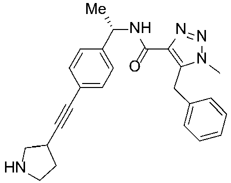 | QY-19-54 | 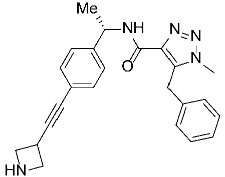 |
| QY-19-55 | 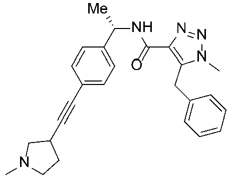 | QY-19-56 | 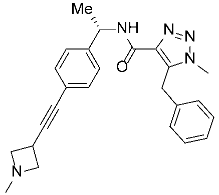 |
| QY-19-60 | 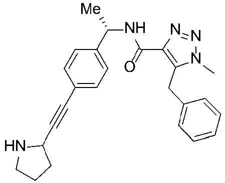 | QY-19-62 | 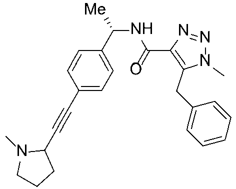 |
| SYL-30-52 | 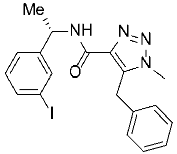 | SYL-30-56 | 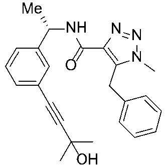 |
| SYL-30-58 | 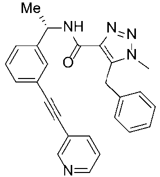 | SYL-30-62 | 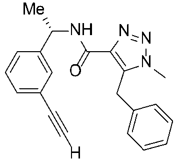 |
| SYL-30-65 | 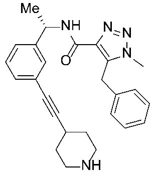 | QY-20-1 | 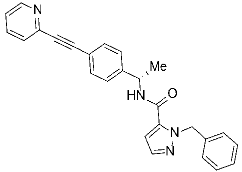 |
| QY-20-2 | 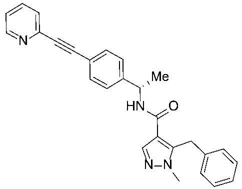 | QY-20-5 | 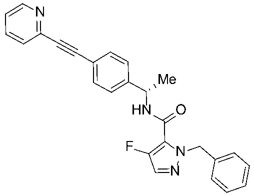 |
| QY-20-18 | 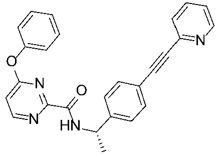 | QY-20-53 | 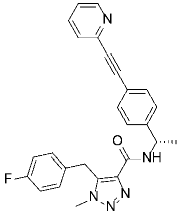 |
| QY-20-54 | 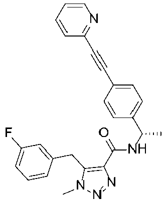 | QY-20-55 | 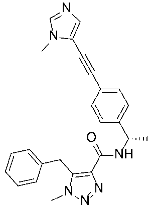 |
| QY-20-67 | 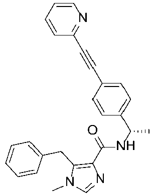 | QY-20-72 | 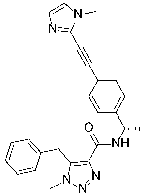 |
| QY-20-73 | 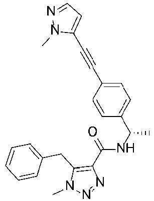 | QY-20-74 | 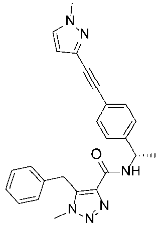 |
| QY-20-76 | 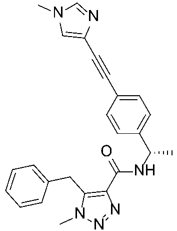 | QY-20-80 | 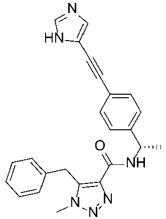 |
| QY-20-81 | 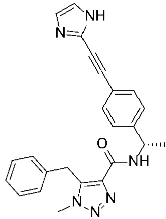 | QY-20-82 | 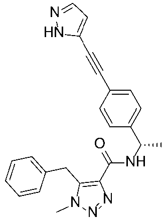 |
| IRCBC-002 | 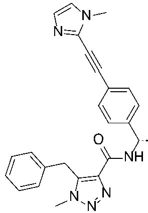 | IRCBC-003 | 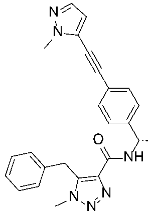 |
| IRCBC-004 | 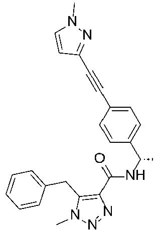 | IRCBC-005 | 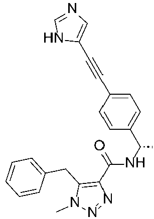 |
| IRCBC-006 | 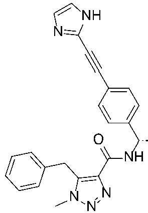 | IRCBC-010 | 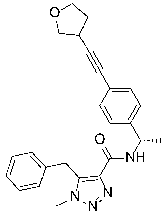 |
| IRCBC-007 | 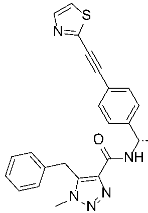 | IRCBC-016 | 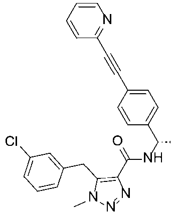 |
| IRCBC-033 | 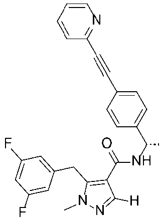 | IRCBC-050 | 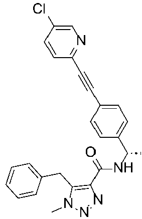 |
| IRCBC-043 | 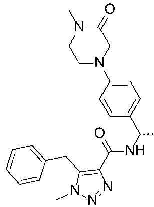 | IRCBC-054 | 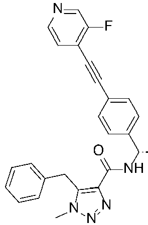 |
| IRCBC-041 | 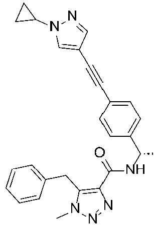 | IRCBC-046 | 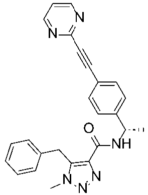 |
| IRCBC-049 | 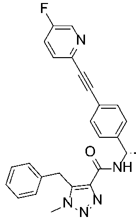 | IRCBC-048 | 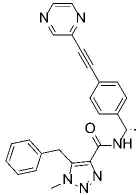 |
| IRCBC-052 | 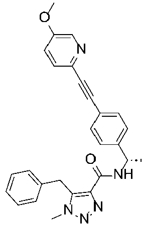 | IRCBC-067 | 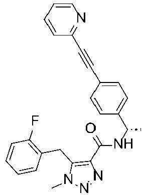 |
| IRCBC-032 | 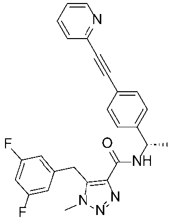 | IRCBC-053 | 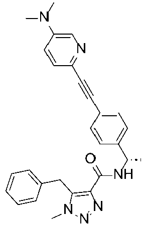 |
| IRCBC-051 | 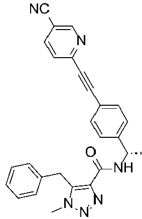 | IRCBC-058 | 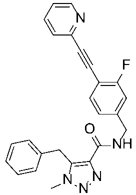 |
| IRCBC-059 | 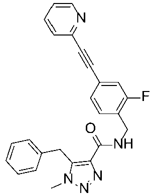 | IRCBC-071 | 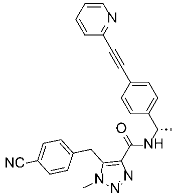 |
| IRCBC-072 | 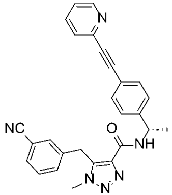 | IRCBC-095 | 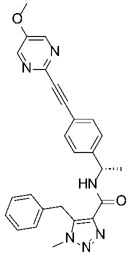 |
| IRCBC-096 | 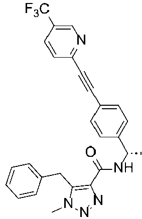 | IRCBC-094 | 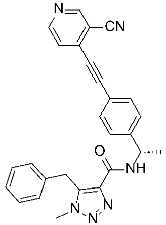 |
| IRCBC-093 | 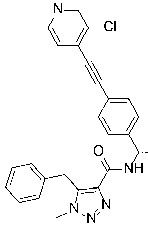 | IRCBC-101 | 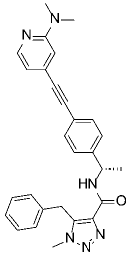 |
| IRCBC-057 | 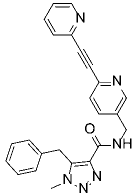 | IRCBC-102 | 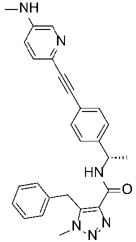 |
| IRCBC-091 | 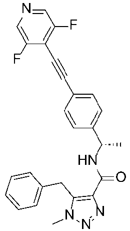 | IRCBC-105 | 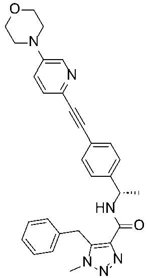 |
| IRCBC-099 | 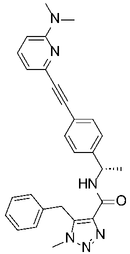 | IRCBC-106 | 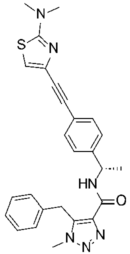 |
| IRCBC-100 | 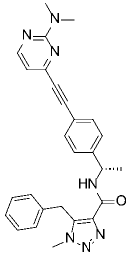 | IRCBC-119 | 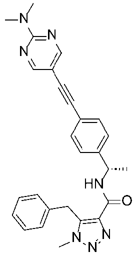 |
| IRCBC-098 | 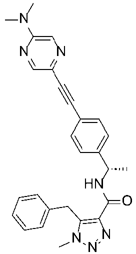 | IRCBC-122 | 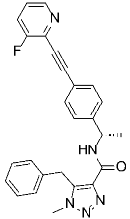 |
| IRCBC-120 | 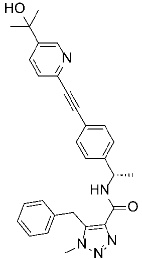 | IRCBC-131 | 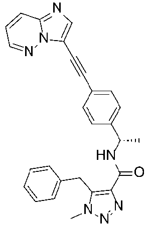 |
| IRCBC-121 | 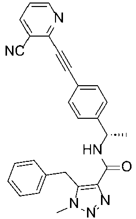 | IRCBC-103 | 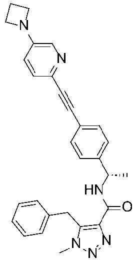 |
| IRCBC-125 | 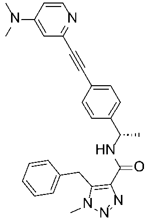 | IRCBC-104 | 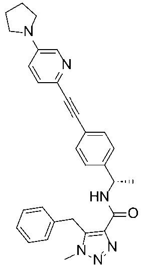 |
| IRCBC-145 | 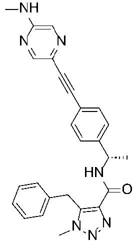 | IRCBC-129 | 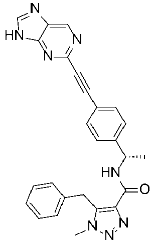 |
| IRCBC-097 | 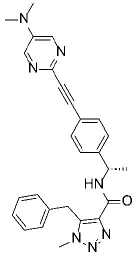 | IRCBC-146 | 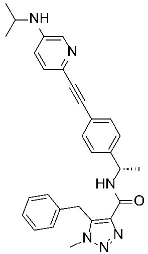 |
| IRCBC-134 | 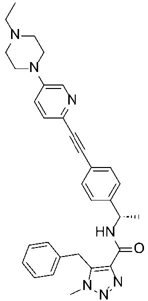 | IRCBC-124 | 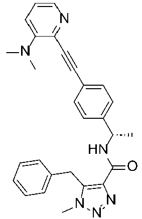 |
| IRCBC-123 | 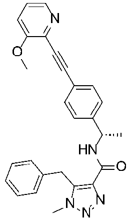 | IRCBC-149 | 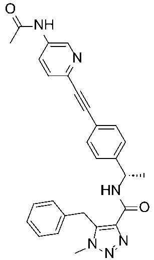 |
| IRCBC-150 | 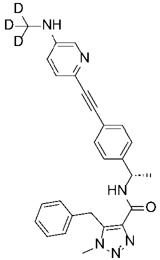 | IRCBC-036 | 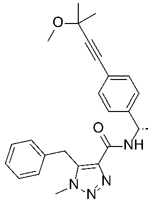 |
| IRCBC-152 | 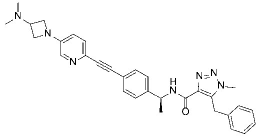 | IRCBC-154 | 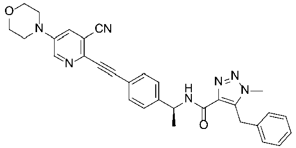 |
| IRCBC-153 | 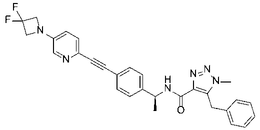 | IRCBC-159 | 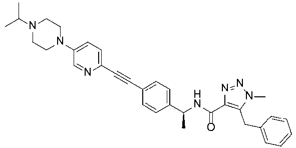 |
| IRCBC-155 | 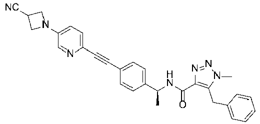 | | |
| IRCBC-156 | 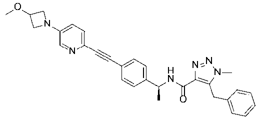 | IRCBC-157 | 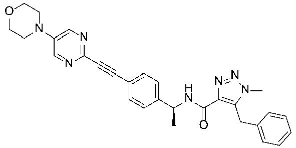 |
| IRCBC-161 | 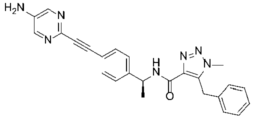 | IRCBC-158 | 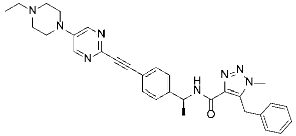 |
| IRCBC-008 | 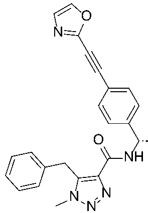 | IRCBC-259 | 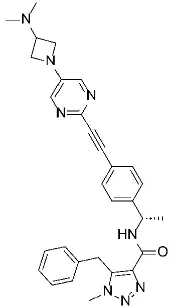 |
| IRCBC-260 | 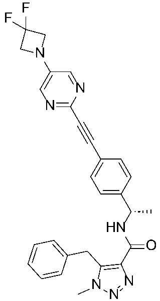 | IRCBC-258 | 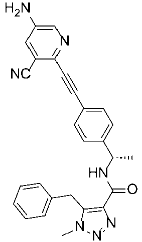 |
| IRCBC-160 | 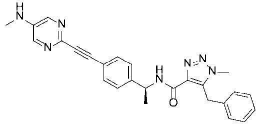 | IRCBC-261 | 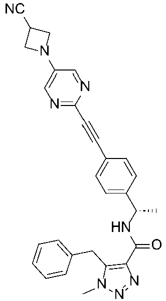 |
| IRCBC-257 | 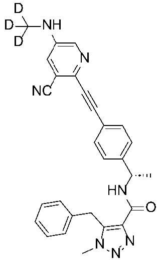 | IRCBC-262 | 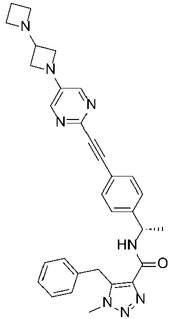 |
| IRCBC-256 | 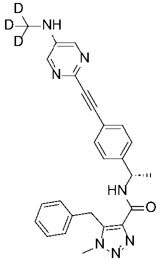 | IRCBC-276 | 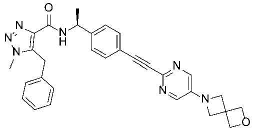 |
| IRCBC-269 | 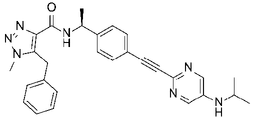 | IRCBC-271 | 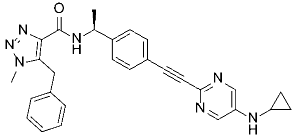 |
| IRCBC-265 | 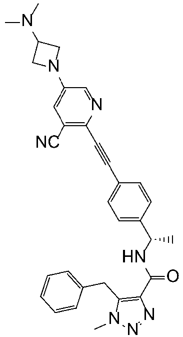 | IRCBC-266 | 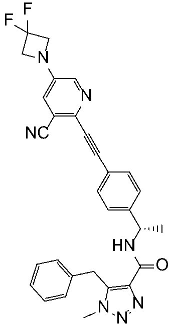 |
| IRCBC-267 | 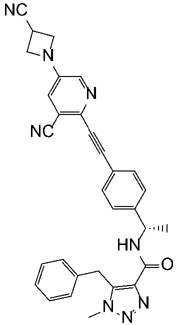 | IRCBC-268 | 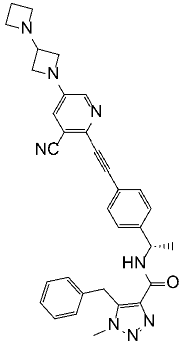 |
| IRCBC-270 | 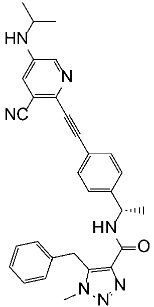 | IRCBC-272 | 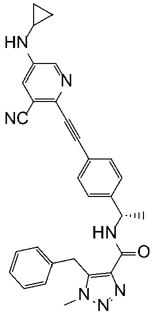 |
| IRCBC-273 | 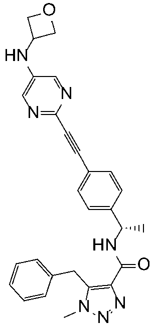 | IRCBC-274 | 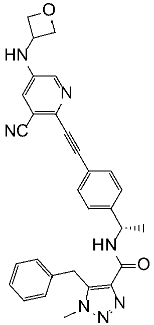 |
| IRCBC-275 | 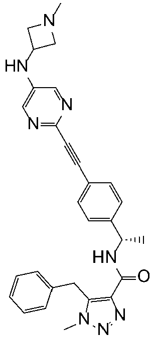 | IRCBC-277 | 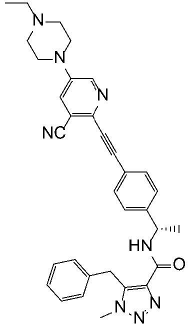 |
| IRCBC-279 | 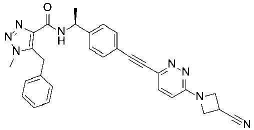 | IRCBC-280 | 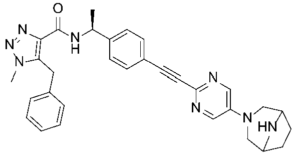 |
| IRCBC-281 | 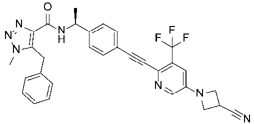 | IRCBC-282 | 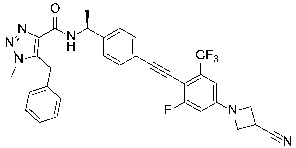 |
| IRCBC-283 | 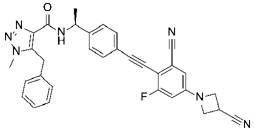 | IRCBC-284 | 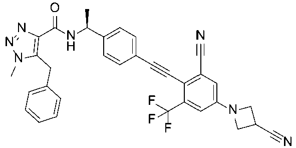 |
| IRCBC-285 | 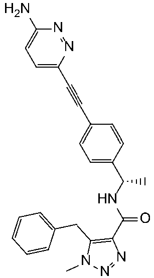 | IRCBC-286 | 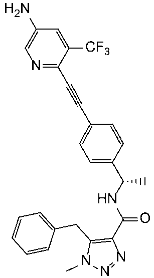 |
| IRCBC-287 | 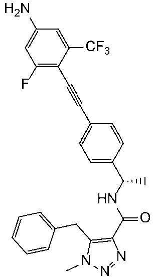 | IRCBC-288 | 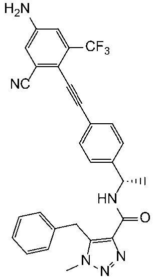 |
| IRCBC-289 | 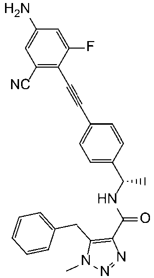 | IRCBC-290 | 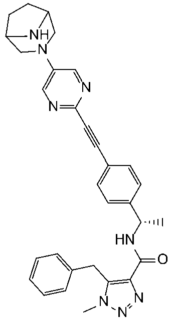 |
| IRCBC-291 | 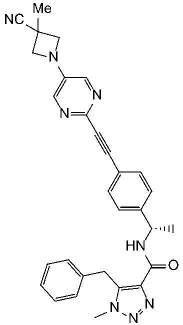 | IRCBC-292 | 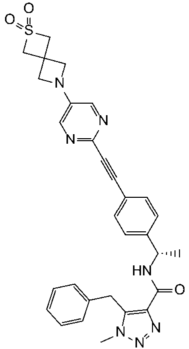 |
| IRCBC-293 | 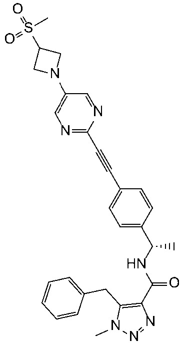 | IRCBC-294 | 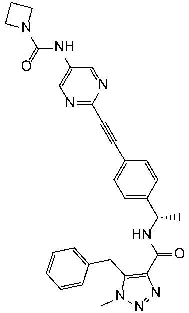 |
| IRCBC-295 | 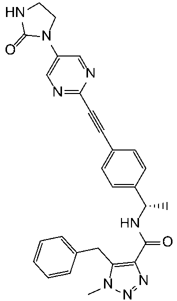 | IRCBC-296 | 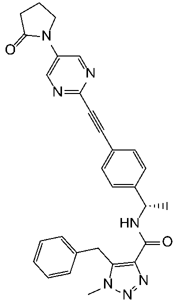 |

NMR data of the compounds shown in Table 1 are as follows:
**QY-17-24:** ¹H NMR (400 MHz, DMSO-*d*₆) δ 9.06 (d, *J =* 8.4 Hz, 1H), 7.65 (t, *J =* 1.8 Hz, 1H), 7.42 (dt, *J =* 7.9, 1.8 Hz, 2H), 7.32 - 7.25 (m, 3H), 7.24 - 7.15 (m, 3H), 5.16 (p, *J* = 7.3 Hz, 1H), 4.41 (q, *J =* 15.3 Hz, 2H), 3.89 (s, 3H), 1.49 (d, *J* = 7.1 Hz, 3H). ESI-MS *m*/*z* 399.0 (M+H)⁺.
**QY-17-25:** ¹H NMR (400 MHz, DMSO-*d*₆) δ 8.92 (d, *J* = 8.5 Hz, 1H), 7.42 (d, *J* = 7.1 Hz, 2H), 7.37 - 7.09 (m, 8H), 5.18 (p, *J* = 7.3 Hz, 1H), 4.42 (q, *J =* 15.3 Hz, 2H), 3.88 (s, 3H), 1.50 (d, *J* = 7.1 Hz, 3H). ESI-MS *m*/*z* 321.1 (M+H)⁺.
**QY-18-15:** ¹H NMR (400 MHz, DMSO-*d*₆) δ 8.96 (d, *J =* 8.3 Hz, 1H), 7.73 - 7.61 (m, 2H), 7.33 - 7.13 (m, 7H), 5.12 (p, *J =* 7.3 Hz, 1H), 4.40 (q, *J =* 15.3 Hz, 2H), 3.88 (s, 3H), 1.48 (d, *J=* 7.1 Hz, 3H). ESI-MS m/z 446.9 (M+H)⁺.
**QY-18-25:** ¹H NMR (400 MHz, DMSO-*d*₆) δ 9.02 (d, *J* = 8.4 Hz, 1H), 8.75 (d, *J* = 2.1 Hz, 1H), 8.59 (dd, *J =* 4.9, 1.7 Hz, 1H), 7.98 (dt, *J* = 8.0, 2.0 Hz, 1H), 7.56 (d, *J* = 8.0 Hz, 2H), 7.52 - 7.44 (m, 3H), 7.28 (t, *J =* 7.3 Hz, 2H), 7.24 - 7.15 (m, 3H), 5.21 (q, *J =* 7.4 Hz, 1H), 4.42 (q, *J*= 15.3 Hz, 2H), 3.89 (s, 3H), 1.52 (d, *J* = 7.1 Hz, 3H). ESI-MS m/z 422.2 (M+H)⁺.
**QY-18-26:** ¹H NMR (400 MHz, DMSO-*d*₆) δ 9.03 (d, *J* = 8.4 Hz, 1H), 8.61 (d, *J* = 4.8 Hz, 1H), 7.86 (td, *J* = 7.8, 1.9 Hz, 1H), 7.64 (d, *J =* 7.8 Hz, 1H), 7.58 (d, *J* = 7.9 Hz, 2H), 7.50 (d, *J =* 7.9 Hz, 2H), 7.42 (dd, *J* = 7.6, 4.9 Hz, 1H), 7.28 (t, *J =* 7.3 Hz, 2H), 7.24 - 7.15 (m, 3H), 5.22 (p, *J* = 7.1 Hz, 1H), 4.42 (q, *J* = 15.3 Hz, 2H), 3.89 (s, 3H), 1.52 (d, *J* = 7.0 Hz, 3H). ESI-MS *m*/*z* 422.2 (M+H)⁺.
**QY-18-27:** ¹H NMR (400 MHz, DMSO-*d*₆) δ 9.04 (d, *J =* 8.3 Hz, 1H), 8.69 (s, 2H), 7.65 (d, *J =* 5.1 Hz, 2H), 7.60 (d, *J =* 8.1 Hz, 2H), 7.52 (d, *J =* 8.0 Hz, 2H), 7.28 (t, *J =* 7.3 Hz, 2H), 7.24 - 7.16 (m, 3H), 5.22 (p, *J* = 7.2 Hz, 1H), 4.42 (q, *J =* 15.3 Hz, 2H), 3.89 (s, 3H), 1.52 (d, *J =* 7.1 Hz, 3H). ESI-MS *m*/*z* 422.2 (M+H)⁺.
**QY-18-28:** ¹H NMR (400 MHz, DMSO-*d*₆) δ 9.01 (d, *J* = 8.4 Hz, 1H), 7.89 (d, *J* = 8.6 Hz, 4H), 7.64 (dd, *J* = 8.4, 1.8 Hz, 1H), 7.48 (pd, *J* = 7.1, 1.7 Hz, 2H), 7.28 - 7.22 (m, 2H), 7.22 - 7.14 (m, 3H), 5.35 (p, *J =* 7.2 Hz, 1H), 4.52 - 4.21 (m, 2H), 3.88 (s, 3H), 1.60 (d, *J =* 7.0 Hz, 3H). ESI-MS *m*/*z* 371.2 (M+H)⁺.
**QY-18-41:** ¹H NMR (400 MHz, DMSO-*d*₆) δ 9.12 (d, *J* = 8.2 Hz, 1H), 7.80 (d, *J* = 8.0 Hz, 2H), 7.61 (d, *J* = 8.0 Hz, 2H), 7.30 - 7.24 (m, 2H), 7.24 - 7.12 (m, 3H), 5.22 (p, *J* = 7.3 Hz, 1H), 4.40 (q, *J =* 15.3 Hz, 2H), 3.88 (s, 3H), 1.51 (d, *J =* 7.1 Hz, 3H). ESI-MS *m*/*z* 346.1 (M+H)⁺.
**QY-18-42:** ¹H NMR (400 MHz, DMSO-*d*₆) δ 8.95 (d, *J* = 8.4 Hz, 1H), 7.40 (d, *J* = 8.0 Hz, 2H), 7.34 (d, *J* = 7.9 Hz, 2H), 7.27 (d, *J=* 7.2 Hz, 2H), 7.20 (dd, *J* = 14.8, 7.1 Hz, 3H), 5.18 (p, *J* = 7.1 Hz, 1H), 4.41 (q, *J =* 15.3 Hz, 2H), 3.88 (s, 3H), 1.49 (d, *J* = 7.0 Hz, 3H), 1.45 (s, 6H). ESI-MS *m*/*z* 403.2 (M+H)⁺.
**QY-18-43:** ¹H NMR (400 MHz, DMSO-*d*₆) δ 8.97 (d, *J =* 8.3 Hz, 1H), 8.05 (s, 1H), 7.67 (s, 1H), 7.43 (s, 4H), 7.28 (t, *J =* 7.3 Hz, 2H), 7.24 - 7.16 (m, 3H), 5.19 (p, *J* = 7.2 Hz, 1H), 4.42 (q, *J* = 15.3 Hz, 2H), 3.88 (s, 3H), 3.85 (s, 3H), 1.50 (d, *J =* 7.0 Hz, 3H). ESI-MS *m*/*z* 425.2 (M+H)⁺.
**QY-18-44:** ¹H NMR (400 MHz, DMSO-*d*₆) δ 8.95 (d, *J* = 8.4 Hz, 1H), 7.37 (q, *J* = 8.4 Hz, 4H), 7.28 (dd, *J* = 7.9, 6.3 Hz, 2H), 7.24 - 7.13 (m, 3H), 5.17 (p, *J =* 6.9 Hz, 1H), 4.41 (q, *J =* 15.3 Hz, 2H), 3.88 (s, 3H), 3.83 - 3.76 (m, 2H), 3.48 - 3.40 (m, 2H), 2.87 (tt, *J =* 9.0, 4.1 Hz, 1H), 1.90 - 1.76 (m, 2H), 1.64 - 1.54 (m, 2H), 1.49 (d, *J =* 7.0 Hz, 3H). ESI-MS *m*/*z* 429.2 (M+H)⁺.
**QY-18-47:** ¹H NMR (400 MHz, DMSO-*d*₆) δ 8.93 (d, *J* = 8.6 Hz, 1H), 7.36 (d, *J* = 8.0 Hz, 2H), 7.33 - 7.24 (m, 4H), 7.24 - 7.15 (m, 3H), 5.16 (p, *J =* 7.4 Hz, 1H), 4.41 (q, *J =* 15.3 Hz, 2H), 3.88 (s, 3H), 1.57 - 1.50 (m, 1H), 1.49 (s, 3H), 0.92 - 0.82 (m, 2H), 0.75 - 0.66 (m, 2H). ESI-MS *m*/*z* 385.1 (M+H)⁺.
**QY-18-49:** ¹H NMR (400 MHz, DMSO-*d*₆) δ 8.98 (d, *J* = 8.4 Hz, 1H), 7.54 - 7.37 (m, 4H), 7.31 - 7.24 (m, 2H), 7.24 - 7.14 (m, 3H), 5.18 (p, *J =* 7.2 Hz, 1H), 4.41 (q, *J* = 15.3 Hz, 2H), 4.12 (s, 1H), 3.88 (s, 3H), 1.49 (d, *J =* 7.1 Hz, 3H). ESI-MS m/z 345.2 (M+H)⁺.
**QY-18-63:** ¹H NMR (400 MHz, DMSO-*d*₆) δ 8.61 (d, *J* = 4.8 Hz, 1H), 7.86 (td, *J* = 7.7, 1.8 Hz, 1H), 7.65 (d, *J =* 7.8 Hz, 1H), 7.60 (d, *J =* 8.0 Hz, 2H), 7.42 (dd, *J =* 7.6, 5.0 Hz, 1H), 7.38 (d, *J* = 8.0 Hz, 2H), 7.32 (d, *J* = 5.8 Hz, 4H), 7.23 (dt, *J* = 5.8, 2.8 Hz, 1H), 5.46 (q, *J* = 7.2 Hz, 1H), 4.40 (d, *J =* 16.3 Hz, 1H), 4.19 (s, 2H), 4.00 (d, *J =* 16.3 Hz, 1H), 3.78 (s, 3H), 1.61 (d, *J =* 7.2 Hz, 3H). ESI-MS m/z 433.2 (M+H)⁺.
**QY-18-77:** ¹H NMR (400 MHz, DMSO-*d*₆) δ 9.43 (s, 1H), 9.01 (d, *J =* 8.4 Hz, 1H), 8.95 (s, 1H), 7.49 (q, *J =* 8.2 Hz, 4H), 7.28 (t, *J =* 7.3 Hz, 2H), 7.24 - 7.14 (m, 3H), 5.21 (p, *J =* 7.3 Hz, 1H), 4.42 (q, *J =* 15.4 Hz, 2H), 3.89 (s, 3H), 1.51 (d, *J =* 7.1 Hz, 4H). ESI-MS *m*/*z* 412.1 (M+H)⁺.
**QY-18-102:** ¹H NMR (400 MHz, DMSO-*d*₆) δ 8.62 (d, *J* = 4.8 Hz, 1H), 7.87 (td, *J* = 7.7, 1.8 Hz, 1H), 7.64 (dd, *J* = 17.6, 7.9 Hz, 3H), 7.43 (d, *J =* 7.9 Hz, 3H), 7.36 - 7.26 (m, 4H), 7.25 - 7.18 (m, 1H), 5.97 (q, *J =* 7.1 Hz, 1H), 4.39 (d, *J =* 4.9 Hz, 2H), 3.64 (s, 3H), 3.50 - 3.42 (m, 1H), 3.12 - 3.04 (m, 1H), 2.80 - 2.62 (m, 2H), 1.54 (d, *J =* 7.1 Hz, 3H). ESI-MS *m*/*z* 447.2 (M+H)⁺.
**QY-19-53:** ¹H NMR (400 MHz, DMSO-*d*₆) δ 8.98 (d, *J* = 8.4 Hz, 1H), 7.43 - 7.34 (m, 4H), 7.28 (t, *J=* 7.3 Hz, 2H), 7.24 - 7.15 (m, 3H), 5.17 (p, *J =* 7.3 Hz, 1H), 4.53 - 4.29 (m, 2H), 3.88 (s, 3H), 3.45 - 3.39 (m, 2H), 3.26 - 3.18 (m, 2H), 3.15 - 3.09 (m, 1H), 3.04 - 3.00 (m, 1H), 2.29 - 2.19 (m, 1H), 1.96 - 1.86 (m, 1H), 1.49 (d, *J =* 7.1 Hz, 3H). ESI-MS *m*/*z* 414.2 (M+H)⁺.
**QY-19-54:** ¹H NMR (400 MHz, DMSO-*d*₆) δ 9.00 (d, *J* = 8.4 Hz, 1H), 8.77 (s, 1H), 7.50 - 7.36 (m, 4H), 7.28 (dd, *J =* 8.0, 6.4 Hz, 2H), 7.24 - 7.12 (m, 3H), 5.18 (p, *J =* 7.1 Hz, 1H), 4.41 (q, *J =* 15.3 Hz, 2H), 4.15 (t, *J* = 8.8 Hz, 2H), 4.01 - 3.90 (m, 3H), 3.88 (s, 3H), 1.49 (d, *J* = 7.0 Hz, 3H). ESI-MS m/z 400.2 (M+H)⁺.
**QY-19-55:** ¹H NMR (400 MHz, DMSO-*d*₆) δ 8.98 (d, *J* = 8.4 Hz, 1H), 7.39 (q, *J* = 8.4 Hz, 4H), 7.28 (dd, *J* = 8.0, 6.4 Hz, 2H), 7.24 - 7.13 (m, 3H), 5.17 (p, *J* = 7.2 Hz, 1H), 4.50 - 4.29 (m, 2H), 3.88 (s, 3H), 3.46 - 3.38 (m, 2H), 3.20 - 3.00 (m, 3H), 2.69 (s, 3H), 2.42 - 2.32 (m, 1H), 2.06 - 1.96 (m, 1H), 1.49 (d, *J=* 7.0 Hz, 3H). ESI-MS *m*/*z* 428.2 (M+H)⁺.
**QY-19-56:** ¹H NMR (400 MHz, DMSO-*d*₆) δ 8.99 (d, *J* = 8.4 Hz, 1H), 7.48 - 7.35 (m, 4H), 7.28 (t, *J =* 7.3 Hz, 2H), 7.24 - 7.13 (m, 3H), 5.18 (p, *J* = 7.2 Hz, 1H), 4.41 (q, *J* = 15.3 Hz, 2H), 4.10 - 4.00 (m, 2H), 3.88 (s, 3H), 3.82 - 3.66 (m, 3H), 2.64 (s, 3H), 1.49 (d, *J =* 7.0 Hz, 3H). ESI-MS *m*/*z* 414.2 (M+H)⁺.
**QY-19-60:** ¹H NMR (400 MHz, DMSO-*d*₆) δ 8.98 (d, *J* = 8.4 Hz, 1H), 7.39 (q, *J* = 8.0 Hz, 4H), 7.28 (t, *J* = 7.4 Hz, 2H), 7.23 - 7.15 (m, 3H), 5.18 (p, *J* = 7.2 Hz, 1H), 4.41 (q, *J* = 15.3 Hz, 2H), 4.10 (t, *J* = 6.6 Hz, 1H), 3.88 (s, 3H), 3.06 - 2.98 (m, 1H), 2.92 - 2.84 (m, 1H), 2.16 - 2.04 (m, 1H), 1.90 - 1.67 (m, 3H), 1.49 (d, *J =* 7.1 Hz, 3H). ESI-MS *m*/*z* 414.2 (M+H)⁺.
**QY-19-62:** ¹H NMR (400 MHz, DMSO-*d*₆) δ 8.98 (d, *J* = 8.4 Hz, 1H), 7.45 - 7.34 (m, 4H), 7.28 (dd, *J* = 8.1, 6.6 Hz, 2H), 7.24 - 7.13 (m, 3H), 5.17 (p, *J =* 7.3 Hz, 1H), 4.41 (q, *J =* 15.3 Hz, 2H), 3.88 (s, 3H), 3.39 (s, 1H), 2.80 (s, 1H), 2.41 (s, 0H), 2.37 (s, 3H), 2.13 (q, *J* = 7.3 Hz, 1H), 1.81 (ddt, *J* = 24.7, 18.6, 8.0 Hz, 3H), 1.49 (d, *J* = 7.1 Hz, 3H). ESI-MS *m*/*z* 428.2 (M+H)⁺.
**SYL-30-52:** ¹H NMR (400 MHz, DMSO-*d*₆) δ 9.08 - 8.98 (m, 1H), 7.82 (d, *J =* 3.0 Hz, 1H), 7.59 (d, *J* = 7.6 Hz, 1H), 7.43 (d, *J* = 7.4 Hz, 1H), 7.28 (dt, *J* = 7.2, 3.5 Hz, 2H), 7.24 - 7.10 (m, 4H), 5.28 - 4.90 (m, 1H), 4.56 - 4.27 (m, 2H), 3.89 (d, *J =* 2.7 Hz, 3H), 1.48 (dd, *J* = 6.8, 2.8 Hz, 3H). ESI-MS *m*/*z* 447.1 (M+H)⁺.
**SYL-30-56:** ¹H NMR (400 MHz, DMSO-*d*₆) δ 9.01 (d, *J* = 8.4 Hz, 1H), 7.47 (s, 1H), 7.40 (d, *J* = 7.7 Hz, 1H), 7.35 - 7.13 (m, 7H), 5.15 (t, *J =* 7.6 Hz, 1H), 4.41 (q, *J =* 15.3 Hz, 2H), 3.88 (s, 3H), 1.49 (d, *J =* 7.0 Hz, 3H), 1.45 (s, 6H). ESI-MS m/z 403.2 (M+H)⁺.
**SYL-30-58:** ¹H NMR (400 MHz, DMSO-*d*₆) δ 9.04 (d, *J* = 8.4 Hz, 1H), 8.78 (d, *J* = 2.2 Hz, 1H), 8.61 (dd, *J* = 4.9, 1.7 Hz, 1H), 8.02 (dt, *J =* 8.0, 2.0 Hz, 1H), 7.67 (d, *J =* 1.9 Hz, 1H), 7.54 - 7.45 (m, 3H), 7.41 (t, *J* = 7.6 Hz, 1H), 7.30 - 7.24 (m, 2H), 7.22 - 7.13 (m, 3H), 5.20 (p, *J* = 7.1 Hz, 1H), 4.42 (q, *J =* 15.3 Hz, 2H), 3.89 (s, 3H), 1.53 (d, *J* = 7.1 Hz, 3H). ESI-MS *m*/*z* 422.2 (M+H)⁺.
**SYL-30-62:** ¹H NMR (400 MHz, DMSO-*d*₆) δ 9.03 (d, *J* = 8.4 Hz, 1H), 7.66 (s, 1H), 7.53 (d, *J = 7.7* Hz, 1H), 7.48 (d, *J =* 7.7 Hz, 1H), 7.40 (t, *J =* 7.7 Hz, 1H), 7.27 (t, *J* = 7.4 Hz, 2H), 7.23 - 7.14 (m, 3H), 5.18 (p, *J =* 7.3 Hz, 1H), 4.51 - 4.32 (m, 2H), 3.89 (s, 3H), 1.51 (d, *J =* 7.0 Hz, 3H). ESI-MS *m*/*z* 345.1 (M+H)⁺.
**SYL-30-65:** ¹H NMR (400 MHz, DMSO-*d*₆) δ 8.99 (d, *J* = 8.4 Hz, 1H), 8.52 (s, 1H), 8.39 (s, 1H), 7.48 (s, 1H), 7.42 (d, *J* = 7.6 Hz, 1H), 7.35 - 7.24 (m, 4H), 7.23 - 7.15 (m, 3H), 5.15 (p, *J* = 7.2 Hz, 1H), 4.50 - 4.26 (m, 2H), 3.88 (s, 3H), 3.29 - 3.21 (m, 2H), 3.10 - 2.93 (m, 3H), 2.08 - 1.98 (m, 2H), 1.81 - 1.70 (m, 2H), 1.49 (d, *J =* 7.0 Hz, 3H). ESI-MS *m*/*z* 428.2 (M+H)⁺.
**QY-20-1:** ¹H NMR (400 MHz, DMSO-*d*₆) δ 8.91 (d, J = 8.0 Hz, 1H), 8.71 - 8.53 (m, 1H), 7.87 (td, J = 7.8, 1.9 Hz, 1H), 7.66 (d, J = 7.8 Hz, 1H), 7.61 - 7.52 (m, 3H), 7.42 (td, J = 7.9, 5.7 Hz, 3H), 7.33 - 7.21 (m, 3H), 7.10 (dd, J = 7.4, 2.0 Hz, 2H), 7.01 (d, J = 2.1 Hz, 1H), 5.68 (s, 2H), 5.14 (p, J = 7.2 Hz, 1H), 1.46 (d, J = 7.1 Hz, 3H). ESI-MS m/z 407.1 (M+H)⁺.
**QY-20-2:** ¹H NMR (400 MHz, DMSO-*d*₆) δ 8.62 (d, J = 5.0 Hz, 1H), 8.44 (d, J = 7.9 Hz, 1H), 8.07 (s, 1H), 7.88 (td, J = 7.7, 2.0 Hz, 1H), 7.66 (d, J = 7.8 Hz, 1H), 7.59 (dd, J = 8.2, 1.9 Hz, 2H), 7.51 - 7.38 (m, 3H), 7.29 - 7.23 (m, 2H), 7.21 - 7.11 (m, 3H), 5.17 (p, J = 7.1 Hz, 1H), 4.46 - 4.35 (m, 2H), 3.65 (s, 3H), 1.47 (d, J = 7.0 Hz, 3H). ESI-MS m/z 421.2 (M+H)⁺.
**QY-20-5:** ¹H NMR (400 MHz, DMSO-*d*₆) δ 8.89 (d, J = 7.9 Hz, 1H), 8.70 - 8.56 (m, 1H), 7.87 (td, J = 7.7, 1.8 Hz, 1H), 7.66 (dd, J = 6.1, 4.3 Hz, 2H), 7.56 (d, J = 7.9 Hz, 2H), 7.48 - 7.35 (m, 3H), 7.33 - 7.25 (m, 3H), 7.09 (dd, J = 6.6, 2.9 Hz, 2H), 5.59 - 5.36 (m, 2H), 5.12 (p, J = 7.2 Hz, 1H), 1.42 (d, J = 7.0 Hz, 3H). ESI-MS *m*/*z* 425.2 (M+H)⁺.
**QY-20-18:** ¹H NMR (400 MHz, DMSO-*d*₆) δ 9.12 (d, J = 8.3 Hz, 1H), 8.80 (d, J = 5.7 Hz, 1H), 8.66 - 8.56 (m, 1H), 7.88 (td, J = 7.8, 1.8 Hz, 1H), 7.66 (d, J = 7.9 Hz, 1H), 7.57 (d, J = 8.0 Hz, 2H), 7.54 - 7.41 (m, 5H), 7.34 (t, J = 7.4 Hz, 1H), 7.29 (d, J = 7.9 Hz, 2H), 7.17 (d, J = 5.7 Hz, 1H), 5.12 (p, J = 7.2 Hz, 1H), 1.48 (d, J = 7.0 Hz, 3H). ESI-MS *m*/*z* 420.1 (M+H)⁺.
**QY-20-53:** ¹H NMR (400 MHz, DMSO-*d*₆) δ 9.06 (d, J = 8.3 Hz, 1H), 8.70 - 8.53 (m, 1H), 7.86 (td, J = 7.7, 1.8 Hz, 1H), 7.65 (d, J = 7.8 Hz, 1H), 7.58 (d, J = 8.2 Hz, 2H), 7.50 (d, J = 8.1 Hz, 2H), 7.42 (ddd, J = 7.6, 4.9, 1.3 Hz, 1H), 7.29 - 7.21 (m, 2H), 7.16 - 7.07 (m, 2H), 5.22 (p, J = 7.3 Hz, 1H), 4.40 (q, J = 15.3 Hz, 2H), 3.90 (s, 3H), 1.52 (d, J = 7.1 Hz, 3H). ESI-MS m/z 440.2 (M+H)⁺.
**QY-20-54:** ¹H NMR (400 MHz, DMSO-*d*₆) δ 9.08 (d, J = 8.3 Hz, 1H), 8.72 - 8.55 (m, 1H), 7.87 (td, J = 7.8, 1.9 Hz, 1H), 7.66 (d, J = 7.8 Hz, 1H), 7.58 (d, J = 8.1 Hz, 2H), 7.50 (d, J = 8.1 Hz, 2H), 7.46 - 7.40 (m, 1H), 7.33 (q, J = 7.4 Hz, 1H), 7.11 - 7.00 (m, 3H), 5.22 (p, J = 7.2 Hz, 1H), 4.50 - 4.37 (m, 2H), 3.92 (s, 3H), 1.52 (d, J = 7.1 Hz, 3H). ESI-MS *m*/*z* 440.2 (M+H)⁺.
**QY-20-55:** ¹H NMR (400 MHz, DMSO-*d*₆) δ 9.06 (d, J = 8.3 Hz, 1H), 8.90 (s, 1H), 7.96 (d, J = 1.4 Hz, 1H), 7.60 (d, J = 8.1 Hz, 2H), 7.52 (d, J = 8.1 Hz, 2H), 7.28 (dd, J = 8.0, 6.5 Hz, 2H), 7.24 - 7.16 (m, 3H), 5.21 (p, J = 7.2 Hz, 1H), 4.51 - 4.33 (m, 2H), 3.89 (s, 3H), 3.86 (s, 3H), 1.52 (d, J = 7.1 Hz, 3H). ESI-MS *m*/*z* 425.2 (M+H)⁺.
**IRCBC-002:**¹H NMR (400 MHz, DMSO-d6) δ 9.08 (d, J = 8.2 Hz, 1H), 7.66 (d, J = 8.1 Hz, 2H), 7.57 (t, J =8.6 Hz, 3H), 7.30 (dd, J = 8.0, 6.5 Hz, 2H), 7.26 -7.16 (m, 3H), 5.30-5.19 (m, 1H), 4.57-4.29(m, 2H), 3.91 (s, 3H), 3.85 (s, 3H), 1.54 (d, J = 7.1 Hz, 3H). ESI-MS m/z =425.1 (M+H)+.
**IRCBC-003:**¹H NMR (400 MHz, DMSO-*d*₆) δ 9.01 (d, *J* = 8.3 Hz, 1H), 7.54 (d, *J* = 8.2 Hz, 2H), 7.50 - 7.44 (m, 3H), 7.25 (dd, *J* = 8.1, 6.6 Hz, 2H), 7.21 - 7.12 (m, 3H), 6.58 (d, *J* = 2.0 Hz, 1H), 5.21 - 5.10 (m, 1H), 4.45 - 4.32 (m, 2H), 3.90 (s, 3H), 3.86 (s, 3H), 1.48 (d, *J* = 7.1 Hz, 3H). ESI-MS m/z=425.2 (M+H)+.
**IRCBC-004:**¹H NMR (400 MHz, DMSO-*d*₆) δ 9.03 (d, *J* = 8.4 Hz, 1H), 7.79 (d, *J =* 2.3 Hz, 1H), 7.53 - 7.45 (m, 4H), 7.30 (dd, *J* = 8.1, 6.5 Hz, 2H), 7.25 - 7.17 (m, 3H), 6.52 (d, *J* = 2.3 Hz, 1H), 5.29 - 5.16 (m, 1H), 4.43 (q, *J =* 15.3 Hz, 2H), 3.90 (s, 3H), 3.88 (s, 3H), 1.53 (d, *J* = 7.1 Hz, 3H). ESI-MS m/z= 425.2(M+H)+.
**IRCBC-006:**¹H NMR (400 MHz, DMSO-*d*₆) δ 9.03 (d, J = 8.4 Hz, 1H), 7.67 - 7.54 (m, 6H), 7.48 (s, 3H), 7.34- 7.18 (m, 2H), 5.22 (t, J = 7.6 Hz, 1H), 4.50 - 4.36 (m, 2H), 3.90 (s, 3H), 1.53 (d, J = 7.1 Hz,3H).. ESI-MS m/z= 411.2(M+H)+.
**IRCBC-007:**¹H NMR (400 MHz, DMSO-*d*₆) δ 9.09 (d, J = 8.4 Hz, 1H), 8.07 - 7.90 (m, 1H), 7.59 (dd, J =43.8, 7.9 Hz, 4H), 7.38 - 7.17 (m, 5H), 5.23 (d, J = 9.4 Hz, 1H), 4.44 (q, J = 15.2 Hz, 2H), 3.91(s, 3H), 1.54 (d, J = 7.1 Hz, 3H). ESI-MS m/z= 428.0(M+H)+.
**IRCBC-005:**¹H NMR (400 MHz, DMSO-*d*₆)δ 9.03 (s, 1H), 7.74 (s, 1H), 7.56 (s, 1H), 7.46 (s, 4H), 7.30 (t, J =7.3 Hz, 1H), 7.26 - 7.18 (m, 3H), 5.21 (t, J = 7.5 Hz, 1H), 4.50 - 4.39 (m, 2H), 3.90 (s, 3H), 1.53(d, J = 7.1 Hz, 3H). ESI-MS m/z= 411.1(M+H)+.
**IRCBC-010:**¹H NMR (400 MHz, DMSO-*d*₆) δ 9.00 (d, J = 8.4 Hz, 1H), 7.39 (q, J = 8.2 Hz, 6H), 7.30 (dd, J = 8.1, 6.6 Hz, 3H), 7.26 - 7.17 (m, 4H), 5.23 - 5.13 (m, 1H), 4.43 (q, J = 15.3 Hz, 2H), 3.98 (t, J = 7.6 Hz, 1H), 3.90 (s, 4H), 3.85 (td, J = 8.2, 5.9 Hz, 1H), 3.80 - 3.72 (m, 1H), 3.59 (dd, J = 8.0, 6.8 Hz, 1H), 3.30 - 3.22 (m, 1H), 2.31 - 2.22 (m, 1H), 2.04 - 1.88 (m, 1H), 1.50 (d, J = 7.1 Hz, 4H).ESI-MS m/z=414.9 (M+H)+.
**IRCBC-016:**¹H NMR (400 MHz, DMSO-*d*₆) )δ 9.09 (d, J = 8.4 Hz, 1H), 8.64 - 8.60 (m, 1H), 7.86 (td, J = 7.7,1.8 Hz, 1H), 7.65 (dt, J = 7.9, 1.1 Hz, 1H), 7.62 - 7.57 (m, 2H), 7.54 - 7.49 (m, 2H), 7.43 (ddd, J= 7.6, 4.9, 1.2 Hz, 1H), 7.38 - 7.28 (m, 3H), 7.17 (dt, J = 6.9, 1.8 Hz, 1H), 5.24 (p, J = 7.1 Hz,1H), 4.51 - 4.39 (m, 2H), 3.94 (s, 3H), 1.54 (d, J = 7.1 Hz, 3H).ESI-MS m/z= 456.0(M+H)+.
**IRCBC-008:**¹H NMR (400 MHz, Chloroform-d) δ 7.68 (d, J = 0.8 Hz, 1H), 7.61 - 7.57 (m, 2H), 7.54 (d, J = 7.9 Hz, 1H), 7.45 - 7.39 (m, 2H), 7.27 (dd, J = 8.0, 6.3 Hz, 2H), 7.22 (dd, J = 6.7, 1.2 Hz, 2H), 7.15 (dd, J = 6.8, 1.9 Hz, 2H), 5.31 (p, J = 7.1 Hz, 1H), 4.56 (d, J = 15.7 Hz, 1H), 4.39 (d, J = 15.6 Hz, 1H), 3.84 (s, 3H), 1.61 (d, J = 7.0 Hz, 3H).ESI-MS m/z=412.0 (M+H)+.
**IRCBC-032:** ¹H NMR (400 MHz, DMSO-d6) δ 9.07 (d, J = 8.3 Hz, 1H), 8.64 - 8.56 (m, 1H), 7.85 (td, J = 7.7,1.8 Hz, 1H), 7.63 (d, J = 7.8 Hz, 1H), 7.57 (d, J = 8.2 Hz, 2H), 7.49 (d, J = 8.1 Hz, 2H), 7.45 - 7.37 (m, 1H), 7.10 (tt, J = 9.4, 2.4 Hz, 1H), 6.96 (h, J = 4.6 Hz, 2H), 5.22 (t, J = 7.5 Hz, 1H), 4.54-4.34 (m, 2H), 3.94 (s, 3H), 1.52 (d, J = 7.1 Hz, 3H)..ESI-MS m/z= 457.9 (M+H)+.
**IRCBC-036:**¹H NMR (400 MHz, Deuterium Oxide) δ 7.52 (d, J = 8.1 Hz, 1H), 7.41 (d, J = 8.3 Hz, 2H), 7.34 (d, J = 8.0 Hz, 2H), 7.28 - 7.25 (m, 2H), 7.24 - 7.19 (m, 1H), 7.17 - 7.12 (m, 2H), 5.29 (p, J = 7.2 Hz, 1H), 4.55 (d, J = 15.6 Hz, 1H), 4.40 (d, J = 15.6 Hz, 1H), 3.83 (s, 3H), 3.42 (s, 3H), 1.59 (d, J = 7.0 Hz, 3H), 1.54 (s, 6H).ESI-MS m/z=417.2 (M+H)+.
**IRCBC-154:**¹H NMR (400 MHz, Chloroform-d) δ 8.46 (d, J = 3.0 Hz, 1H), 7.70 - 7.63 (m, 2H), 7.59 (d, J = 8.1 Hz, 1H), 7.45 (d, J = 7.9 Hz, 2H), 7.37 - 7.28 (m, 4H), 7.22 - 7.15 (m, 2H), 5.34 (q, J = 7.3 Hz, 1H), 4.60 (d, J = 15.6 Hz, 1H), 4.43 (d, J = 15.7 Hz, 1H), 3.96 - 3.90 (m, 4H), 3.88 (d, J = 1.5 Hz, 3H), 3.32 (dd, J = 5.9, 3.9 Hz, 4H), 1.65 (d, J = 7.0 Hz, 3H).ESI-MS m/z= 531.9(M+H)+.
**IRCBC-159:**¹H NMR (400 MHz, Chloroform-d) δ 8.29 (d, J = 2.9 Hz, 1H), 7.61 - 7.51 (m, 3H), 7.39 (dd, J = 8.5, 7.1 Hz, 3H), 7.33 - 7.19 (m, 5H), 7.18 - 7.08 (m, 3H), 5.34 (d, J = 6.8 Hz, 1H), 4.56 (d, J = 15.6 Hz, 1H), 4.41 (d, J = 15.6 Hz, 1H), 3.84 (s, 3H), 3.35 - 3.28 (m, 4H), 2.72 (dd, J = 6.2, 3.9 Hz, 4H), 1.61 (d, J = 7.0 Hz, 3H), 1.11 (d, J = 6.5 Hz, 6H). ESI-MS m/z= 548.4(M+H)+.
**IRCBC-051:**¹H NMR (400 MHz, DMSO-*d*₆)δ 9.05 - 9.01 (m, 2H), 8.37 (dd,J = 8.2, 2.2 Hz, 1H), 7.84 (dd,J = 8.2, 0.8Hz, 1H), 7.63 (d,J = 8.3 Hz, 2H), 7.53 (d,J = 8.3 Hz, 2H), 7.28 (t,J = 7.2 Hz, 2H), 7.23 - 7.16 (m, 3H),5.26 - 5.19 (m, 1H), 4.42 (q,J = 15.3 Hz, 2H), 3.89 (s, 3H), 1.52 (d,J = 7.1 Hz, 3H). ESI-MS m/z= 447.2(M+H)+.
**IRCBC-059:**¹H NMR (400 MHz, DMSO-*d*₆)δ 9.18 (t,J = 6.2 Hz, 1H), 8.63 - 8.60 (m, 1H), 7.87 (td,J = 7.7, 1.8 Hz,1H), 7.66 (d,J = 7.8 Hz, 1H), 7.50 - 7.39 (m, 4H), 7.30 (t,J = 7.2 Hz, 2H), 7.22 (dd,J = 8.6, 7.0 Hz, 3H),4.55 (d,J = 6.1 Hz, 2H), 4.46 (s, 2H), 3.90 (s, 3H). ESI-MS m/z= 426.1(M+H)+.
**IRCBC-067:**¹H NMR (400 MHz, DMSO-*d*₆)δ 9.02 (d, J = 8.4 Hz, 1H), 8.60 (ddd, J = 4.9, 1.8, 0.9 Hz, 1H),7.85 (td, J = 7.7, 1.8 Hz, 1H), 7.63 (dt, J = 7.9, 1.1 Hz, 1H), 7.59-7.53 (m, 2H), 7.51 - 7.45 (m,2H), 7.41 (ddd, J = 7.7, 4.9, 1.2 Hz, 1H), 7.29 (tdd, J = 7.6, 5.4, 1.8 Hz, 1H), 7.19 (ddd, J = 9.8,8.3, 1.3 Hz, 1H), 7.09 (td, J = 7.5, 1.3 Hz, 1H), 7.00 (td, J = 7.7, 1.8 Hz, 1H), 5.28 -5.09 (m, 1H),4.57 -4.36 (m, 2H), 3.90 (s, 3H), 1.50 (d, J = 7.1 Hz, 3H).ESI-MS m/z= 440.1(M+H)+.
**IRCBC-033:**¹H NMR (400 MHz, DMSO-*d*₆)) δ 8.62 (d,J = 4.5 Hz, 1H), 8.52 (d,J = 7.9 Hz, 1H), 8.10 (s, 1H), 7.86 (dt,J = 7.7, 3.9 Hz, 1H), 7.65 (d,J = 7.8 Hz, 1H), 7.59 (d,J = 8.1 Hz, 2H), 7.50 - 7.40 (m, 3H), 7.07 (d,J =9.4 Hz, 1H), 6.92 (d,J = 6.7 Hz, 2H), 5.19 (t,J = 7.3 Hz, 1H), 4.44 (d,J = 8.4 Hz, 2H), 3.71 (s, 3H), 1.49(d,J = 7.0 Hz, 3H). ESI-MS m/z=456.9 (M+H)+.
**IRCBC-041:**¹H NMR(400 MHz, CDCl3) δ 7.69 - 7.37 (m, 7H), 7.20 (s, 2H), 5.34 (s, 1H), 4.60 (d,J = 16.3 Hz, 1H),4.45 (d,J = 15.4 Hz, 1H), 3.87 (s, 4H), 1.64 (d,J = 6.4 Hz, 3H), 1.29 (s, 2H), 1.24 - 0.87 (m, 4H).ESI-MS m/z=451.1 (M+H)+.
**IRCBC-043:**¹H NMR (400 MHz, DMSO-*d*₆) δ 8.76 (d,J = 8.6 Hz, 1H), 7.30 (t,J = 7.8 Hz, 4H), 7.21 (s, 3H), 6.92 (d,J= 8.7 Hz, 2H), 5.17 - 5.08 (m, 1H), 4.44 (d,J = 10.8 Hz, 2H), 3.89 (s, 3H), 3.73 (s, 2H), 3.44 (d,J = 6.6Hz, 4H), 2.90 (s, 3H), 1.48 (d,J = 7.0 Hz, 3H). ESI-MS m/z= 432.9(M+H)+.
**IRCBC-049:**¹H NMR (400 MHz, DMSO-*d*₆)δ 9.06 (d,J = 8.4 Hz, 1H), 8.63 (d,J= 2.8 Hz, 1H), 7.86 - 7.73 (m, 2H),7.59 (d,J = 8.2 Hz, 2H), 7.51 (d,J = 8.2 Hz, 2H), 7.29 (d,J = 7.4 Hz, 2H), 7.21 (t,J = 6.5 Hz, 3H), 5.27 -5.19 (m, 1H), 4.43 (q,J = 15.4 Hz, 2H), 3.90 (s, 3H), 1.53 (d,J = 7.1 Hz, 3H). ESI-MS m/z=440.0 (M+H)+.
**IRCBC-050:**¹H NMR (400 MHz, DMSO-*d*₆)δ 9.05 (d,J = 8.3 Hz, 1H), 8.67 (d,J = 2.4 Hz, 1H), 8.01 (dd,J = 8.3, 2.4Hz, 1H), 7.71 (s, 1H), 7.60 (d,J = 8.1 Hz, 2H), 7.52 (d,J = 8.2 Hz, 2H), 7.29 (d,J = 7.5 Hz, 2H), 7.20 (d,J = 7.1 Hz, 3H), 5.27 - 5.19 (m, 1H), 4.44 (q,J = 15.3 Hz, 2H), 3.90 (s, 3H), 1.54 (d,J = 7.0 Hz, 3H). ESI-MS m/z=456.1 (M+H)+.
**IRCBC-054:**¹H NMR (400 MHz, DMSO-*d*₆) δ 9.08 (d,J = 8.4 Hz, 1H), 8.73 (s, 1H), 8.51 (d,J = 4.8 Hz, 1H), 7.71 -7.66 (m, 1H), 7.63 (d,J = 8.3 Hz, 2H), 7.54 (d,J = 8.3 Hz, 2H), 7.30 (t,J = 7.2 Hz, 2H), 7.22 (dd,J =13.6, 7.1 Hz, 3H), 5.29 - 5.19 (m, 1H), 4.44 (q,J = 15.2 Hz, 2H), 3.91 (s, 3H), 1.54 (d,J = 7.1 Hz, 3H). ESI-MS m/z= 440.1(M+H)+.
**IRCBC-096:**¹H NMR(400 MHz, CDCl3) δ 9.03 (d,J = 8.3 Hz, 1H), 8.99 (s, 1H), 8.27 (dd,J = 8.4, 2.2 Hz, 1H), 7.86(d,J = 8.2 Hz, 1H), 7.63 (d,J = 8.3 Hz, 2H), 7.52 (d,J = 8.3 Hz, 2H), 7.27 (d,J = 7.5 Hz, 2H), 7.19 (dd,J = 7.3, 6.0 Hz, 3H), 5.26 - 5.18 (m, 1H), 4.41 (d,J = 14.5 Hz, 2H), 3.88 (s, 3H), 1.52 (d,J = 7.1 Hz, 3H).ESI-MS m/z= 490.1 (M+H)+.
IRCBC-097: 1H NMR (400 MHz, Chloroform-d) δ 8.18 (s, 2H), 7.62 (d, J = 8.0 Hz, 2H), 7.55 (d, J = 8.1 Hz, 1H), 7.39 (d, J = 8.0 Hz, 2H), 7.28 (s, 2H), 7.22 (s, 1H), 7.16 (d, J = 7.4 Hz, 2H), 5.39 - 5.25 (m, 1H), 4.57 (d, J = 15.6 Hz, 1H), 4.41 (d, J = 15.6 Hz, 1H), 3.84 (s, 3H), 3.06 (s, 6H), 1.61 (d, J = 6.9 Hz, 3H).ESI-MS m/z= 466.2(M+H)+
**IRCBC-093:**¹H NMR (400 MHz, DMSO-*d*₆) δ 9.05 (d, J = 8.3 Hz, 1H), 8.77 (s, 1H), 8.57 (d, J = 5.0 Hz, 1H), 7.67 (d, J = 5.0 Hz, 1H), 7.61 (d, J = 8.2 Hz, 2H), 7.53 (d, J = 8.2 Hz, 2H), 7.28 (t, J = 7.2 Hz, 2H), 7.23 - 7.16 (m, 3H), 5.27 - 5.18 (m, 1H), 4.42 (q, J = 15.3 Hz, 2H), 3.89 (s, 3H), 1.52 (d, J = 7.1 Hz, 3H).ESI-MS m/z= 456.2(M+H)+.
**IRCBC-095:**¹H NMR (400 MHz, DMSO-d6) δ 9.03 (d, J = 8.3 Hz, 1H), 8.59 (s, 2H), 7.60 (d, J = 8.3 Hz, 2H), 7.50 (d, J = 8.3 Hz, 2H), 7.32 - 7.25 (m, 2H), 7.24 - 7.15 (m, 3H), 5.22 (p, J = 7.2 Hz, 1H), 4.42 (q, J = 15.3 Hz, 2H), 3.96 (s, 3H), 3.89 (s, 3H), 1.53 (d, J = 7.1 Hz, 3H). ESI-MS m/z= 453.0(M+H)+.
**IRCBC-094:**¹H NMR (400 MHz, DMSO-*d*₆) δ 9.10 (s, 1H), 9.04 (d,J = 8.3 Hz, 1H), 8.88 (d,J = 5.2 Hz, 1H), 7.78 (d,J= 5.2 Hz, 1H), 7.63 (d,J = 8.2 Hz, 2H), 7.56 (d,J = 8.3 Hz, 2H), 7.27 (d,J = 7.5 Hz, 2H), 7.20 (dd,J =14.1, 7.1 Hz, 3H), 5.27 - 5.18 (m, 1H), 4.42 (q,J = 15.3 Hz, 2H), 3.89 (s, 3H), 1.53 (d,J = 7.1 Hz, 3H).ESI-MS m/z= 893.6(2M+H)+.
**IRCBC-056:**¹H NMR (400 MHz, DMSO-*d*₆)δ 9.29 (t,J = 6.2 Hz, 1H), 8.63 (dd,J = 11.8, 2.9 Hz, 2H), 7.89 (td,J = 7.7, 1.7 Hz,1H), 7.81 (dd,J = 8.0, 2.1 Hz, 1H), 7.70 (t,J = 6.9 Hz, 2H), 7.46 (ddd,J = 7.6, 4.9, 1.0 Hz, 1H), 7.30 (t,J= 7.2 Hz, 2H), 7.22 (t,J = 7.7 Hz, 3H), 4.53 (d,J = 6.2 Hz, 2H), 4.46 (s, 2H), 3.90 (s, 3H).ESI-MS m/z= 408.9(M+H)+.
**IRCBC-101:**¹H NMR (400 MHz, DMSO-*d*₆) δ 9.02 (d,J = 8.4 Hz, 1H), 8.10 (d,J = 5.0 Hz, 1H), 7.56 (d,J = 8.3 Hz,2H), 7.49 (d,J = 8.3 Hz, 2H), 7.29 (t,J = 7.2 Hz, 2H), 7.21 (dd,J = 13.6, 7.1 Hz, 3H), 6.73 (s, 1H), 6.66(dd,J = 5.1, 1.1 Hz, 1H), 5.27 - 5.17 (m, 1H), 4.42 (q,J = 15.3 Hz, 2H), 3.89 (s, 3H), 3.04 (s, 6H), 1.52(d,J = 7.1 Hz, 3H).ESI-MS m/z=465.2 (M+H)+.
**IRCBC-046:**¹H NMR (400 MHz, DMSO-*d*₆) δ 9.05 (d,J = 8.3 Hz, 1H), 8.84 (d,J = 4.9 Hz, 2H), 7.63 (d,J = 8.3 Hz,2H), 7.52 (dd,J = 8.6, 3.6 Hz, 3H), 7.29 (dd,J = 9.9, 4.5 Hz, 2H), 7.24 - 7.16 (m, 3H), 5.22 (p,J = 7.1Hz, 1H), 4.42 (dd,J = 31.0, 15.3 Hz, 2H), 3.89 (s, 3H), 1.53 (d,J = 7.1 Hz, 3H).ESI-MS m/z=423.1 (M+H)+.
**IRCBC-048:**¹H NMR (400 MHz, DMSO-*d*₆)δ 9.04 (d,J = 8.3 Hz, 1H), 8.87 (d,J= 1.5 Hz, 1H), 8.69 (dd,J = 2.5, 1.6Hz, 1H), 8.64 (d,J = 2.5 Hz, 1H), 7.63 (d,J = 8.3 Hz, 2H), 7.52 (d,J = 8.2 Hz, 2H), 7.27 (d,J = 7.5 Hz,2H), 7.20 (dd,J = 7.3, 6.0 Hz, 3H), 5.27 - 5.18 (m, 1H), 4.42 (q,J = 15.3 Hz, 2H), 3.89 (s, 3H), 1.53(d,J= 7.1 Hz, 3H).ESI-MS m/z=423.0 (M+H)+.
**IRCBC-052:**¹H NMR (400 MHz, DMSO-*d*₆) δ 9.00 (d,J = 8.3 Hz, 1H), 8.31 (d,J = 2.9 Hz, 1H), 7.60 (d,J = 8.7 Hz,1H), 7.54 (d,J = 8.2 Hz, 2H), 7.50 - 7.40 (m, 3H), 7.31 - 7.15 (m, 6H), 5.25 - 5.17 (m, 1H),4.42 (q,J =15.4 Hz, 2H), 3.88 (d,J = 6.9 Hz, 6H), 1.52 (d,J = 7.1 Hz, 3H).ESI-MS m/z=452.1 (M+H)+.
**IRCBC-064:**¹H NMR (400 MHz, DMSO-*d*₆)δ 9.00 (d,J = 8.3 Hz, 1H), 8.31 (d,J= 2.9 Hz, 1H), 7.60 (d,J = 8.7 Hz,1H), 7.54 (d,J = 8.2 Hz, 2H), 7.50 - 7.40 (m, 3H), 7.31 - 7.15 (m, 6H), 5.25 - 5.17 (m, 1H), 4.42 (q,J =15.4 Hz, 2H), 3.88 (d,J = 6.9 Hz, 6H), 1.52 (d,J = 7.1 Hz, 3H). ESI-MS m/z=422.1 (M+H)+.
**IRCBC-053:**¹H NMR (400 MHz, DMSO-*d*₆)δ 8.98 (d,J = 8.4 Hz, 1H), 8.10 (d,J= 3.0 Hz, 1H), 7.47 (q,J = 8.4 Hz,4H), 7.40 (d,J = 8.8 Hz, 1H), 7.28 (t,J = 7.2 Hz, 2H), 7.23 - 7.17 (m, 3H), 7.06 (dd,J = 8.9, 3.1 Hz, 1H),5.20 (p,J = 7.1 Hz, 1H), 4.42 (d,J = 14.2 Hz, 2H), 3.89 (s, 3H), 2.99 (s, 6H), 1.51 (d,J = 7.1 Hz, 3H). ESI-MS m/z= 465.1 (M+H)+.
**IRCBC-058:**¹H NMR (400 MHz, DMSO-*d*₆)) δ 9.26 (t,J = 6.3 Hz, 1H), 8.62 (d,J = 4.2 Hz, 1H), 7.87 (td,J = 7.7, 1.8Hz, 1H), 7.65 (t,J = 7.6 Hz, 2H), 7.44 (ddd,J = 7.7, 4.9, 1.1 Hz, 1H), 7.30 (t,J = 7.5 Hz, 3H), 7.22 (t,J =6.6 Hz, 4H), 4.51 (d,J = 6.3 Hz, 2H), 4.46 (s, 2H), 3.90 (s, 3H). ESI-MS m/z= 426.1(M+H)+.
**IRCBC-071:**¹H NMR (400 MHz, DMSO-*d*₆) δ 9.07 (d, J = 8.3 Hz, 1H), 8.65 - 8.58 (m, 1H), 7.85 (td, J = 7.7,1.9 Hz, 1H), 7.76 (d, J = 8.2 Hz, 2H), 7.64 (d, J = 7.8 Hz, 1H), 7.58 (d, J = 8.2 Hz, 2H), 7.49 (d, J= 8.1 Hz, 2H), 7.44 - 7.36 (m, 3H), 5.20 (q, J = 7.4 Hz, 1H), 4.52 (q, J = 15.7 Hz, 2H), 3.92 (s,3H), 1.52 (d, J = 7.0 Hz, 3H).ESI-MS m/z=446.8 (M+H)+.
**IRCBC-072:**¹H NMR (400 MHz, DMSO-*d*₆)δ 9.08 (d,J = 8.4 Hz, 1H), 8.60 (ddd,J = 4.8, 1.7, 0.9 Hz, 1H), 7.85 (td,J= 7.7, 1.8 Hz, 1H), 7.73 - 7.69 (m, 2H), 7.65 - 7.62 (m, 1H), 7.57 (dd,J = 7.5, 5.6 Hz, 2H), 7.54 - 7.47(m, 4H), 7.41 (ddd,J = 7.6, 4.9, 1.1 Hz, 1H), 5.26 - 5.16 (m, 1H), 4.47 (dd,J = 31.6, 15.5 Hz, 2H), 3.94(s, 3H), 1.52 (d,J = 7.1 Hz, 3H). ESI-MS m/z=447.2 (M+H)+.
**IRCBC-057:**¹H NMR (400 MHz, DMSO-*d*₆)δ 9.28 (d,J = 6.4 Hz, 1H), 8.63 (dd,J = 11.8, 3.0 Hz, 2H), 7.89 (td,J =7.7, 1.8 Hz, 1H), 7.81 (dd,J = 8.0, 2.2 Hz, 1H), 7.70 (t,J = 7.1 Hz, 2H), 7.47 (ddd,J = 7.6, 4.8, 1.1 Hz,1H), 7.33 - 7.28 (m, 2H), 7.23 (dd,J = 8.7, 7.0 Hz, 4H), 4.54 (d,J = 6.2 Hz, 2H), 4.46 (s, 2H), 3.90 (s,3H). ESI-MS m/z= 409.2(M+H)+.
**IRCBC-091:**¹H NMR (400 MHz, DMSO-*d*₆) δ 9.07 (d,J = 8.3 Hz, 1H), 8.68 (s, 2H), 7.63 (d,J = 8.3 Hz, 2H), 7.55 (d,J= 8.3 Hz, 2H), 7.29 (t,J = 7.2 Hz, 2H), 7.24 - 7.17 (m, 3H), 5.27 - 5.19 (m, 1H), 4.42 (q,J = 15.3 Hz,2H), 3.89 (s, 3H), 1.53 (d,J = 7.1 Hz, 3H).ESI-MS m/z= 458.1(M+H)+.
**IRCBC-099:**¹H NMR (400 MHz, DMSO-*d*₆) δ 9.02 (d,J = 8.4 Hz, 1H), 7.57 - 7.53 (m, 2H), 7.52 - 7.45 (m, 3H), 7.32 -7.25 (m, 2H), 7.24 - 7.17 (m, 3H), 6.82 (d,J = 7.0 Hz, 1H), 6.67 (d,J = 8.6 Hz, 1H), 5.27 - 5.17 (m, 1H),4.43 (dd,J = 30.4, 15.3 Hz, 2H), 3.89 (s, 3H), 3.03 (s, 6H), 1.52 (d,J =7.1 Hz, 3H).ESI-MS m/z= 465.3(M+H)+.
**IRCBC-100:**¹H NMR (400 MHz, DMSO-*d*₆) δ 9.03 (d,J = 8.4 Hz, 1H), 8.38 (d,J = 4.9 Hz, 1H), 7.61 (d,J = 8.3 Hz,2H), 7.51 (d,J = 8.3 Hz, 2H), 7.28 (t,J = 7.2 Hz, 2H), 7.24 - 7.17 (m, 3H), 5.27-5.18 (m, 1H), 4.42 (q,J= 15.3 Hz, 2H), 3.89 (s, 3H), 3.12 (s, 6H), 1.52 (d,J = 7.1 Hz, 3H).ESI-MS m/z= 466.2(M+H)+.
**IRCBC-102:**¹H NMR (400 MHz, DMSO-*d*₆)δ 8.99 (d, J = 8.4 Hz, 1H), 7.95 (d, J = 2.6 Hz, 1H), 7.50 - 7.43 (m, 4H), 7.35 (d, J = 8.5 Hz, 1H), 7.29 (t, J = 7.2 Hz, 2H), 7.24 - 7.17 (m, 3H), 6.86 (dd, J = 8.6, 2.9 Hz, 1H), 6.37 (q, J = 5.0 Hz, 1H), 5.25 - 5.16 (m, 1H), 4.43 (q, J = 15.3 Hz, 2H), 3.89 (s, 3H), 2.74 (d, J = 5.0 Hz, 3H), 1.52 (d, J = 7.1 Hz, 3H).ESI-MS m/z= 450.22(M+H)+.
**IRCBC-105:**¹H NMR (400 MHz, DMSO-*d*₆) δ 9.00 (d, J = 8.4 Hz, 1H), 8.32 (d, J = 2.8 Hz, 1H), 7.54 - 7.44 (m, 5H), 7.28 (d, J = 7.5 Hz, 3H), 7.24 - 7.16 (m, 3H), 5.21 (p, J = 7.2 Hz, 1H), 4.43 (d, J = 14.2 Hz, 2H), 3.89 (s, 3H), 3.78 - 3.72 (m, 4H), 3.28 - 3.24 (m, 4H), 1.52 (d, J = 7.1 Hz, 3H).ESI-MS m/z= 506.24(M+H)+.
**IRCBC-106:**¹H NMR (400 MHz, DMSO-*d*₆)δ 9.00 (d, J = 8.4 Hz, 1H), 7.48 (q, J = 8.4 Hz, 4H), 7.28 (d, J = 7.5 Hz, 2H), 7.20 (t, J = 6.8 Hz, 3H), 7.14 (s, 1H), 5.25 - 5.16 (m, 1H), 4.42 (d, J = 15.0 Hz, 2H), 3.89 (s, 3H), 3.05 (s, 6H), 1.52 (d, J = 7.1 Hz, 3H).ESI-MS m/z=470.19 (M+H)+.
**IRCBC-119:**¹H NMR (400 MHz, DMSO-*d*₆)1H NMR (400 MHz, DMSO-d6) δ 9.00 (d, J = 8.4 Hz, 1H), 8.53 (s, 2H), 7.47 (d, J = 4.3 Hz, 4H), 7.28 (d, J = 7.5 Hz, 2H), 7.24 - 7.17 (m, 3H), 5.21 (p, J = 7.1 Hz, 1H), 4.43 (q, J = 15.3 Hz, 2H), 3.89 (s, 3H), 3.17 (s, 6H), 1.51 (d, J = 7.1 Hz, 3H). ESI-MS m/z= 465.23(M+H)+.
**IRCBC-098:** ¹H NMR (400 MHz, DMSO-*d*₆) δ 9.03 (d, *J* = 8.3 Hz, 1H), 8.29 *(d, J=* 1.4 Hz, 1H), 8.19 (d, *J* = 1.5 Hz, 1H), 7.56 - 7.45 (m, 4H), 7.34 - 7.18 (m, 5H), 5.23 (t, *J* = 7.5 Hz, 1H), 4.49 -4.36 (m, 2H), 3.91 (s, 3H), 3.14 (s, 6H), 1.54 (d, *J= 7.0* Hz, 3H). ESI-MS m/z= 466.1 (M+H)+.
**IRCBC-120:** ¹H NMR (400 MHz, Chloroform-d) δ 8.66 (d, *J* = 2.5 Hz, 1H), 7.74 (dd, *J* = 8.2, 2.4 Hz, 1H), 7.49 (d, *J= 8.1* Hz, 3H), 7.42 (d, *J* = 8.2 Hz, 1H), 7.33 (d, *J* = 8.1 Hz, 2H), 7.25 - 7.16 (m, 3H), 7.16 - 7.09 (m, 2H), 7.07 (s, 1H), 5.25 (p, *J* = 7.1 Hz, 1H), 4.49 (m, 1H), 4.33 (m, 1H), 3.77 (s, 3H), 1.55 (s, 9H). ESI-MS m/z= 480.3(M+H)+.
**IRCBC-121:** ¹H NMR (400 MHz, Chloroform-d) δ 8.71 (dd, J= 4.9, 1.8 Hz, 1H), 7.90 (dd, *J* = 7.9, 1.8 Hz, 1H), 7.64 - 7.57 (m, 2H), 7.49 (d, *J* = 7.9 Hz, 1H), 7.37 (d, *J =* 8.2 Hz, 2H), 7.26 - 7.05 (m, 6H), 5.25 (p, *J* = 7.1 Hz, 1H), 4.49 (m, 1H), 4.32 (m, 1H), 3.77 (s, 3H), 1.55 (d, *J* = 7.0 Hz, 3H). ESI-MS m/z= 447.2 (M+H)+.
**IRCBC-122:**¹H NMR (400 MHz, Chloroform-d) δ 8.36 (dd, J = 3.9, 2.2 Hz, 1H), 7.54 (d, J = 8.1 Hz, 2H), 7.49 (d, J = 8.0 Hz, 1H), 7.41 - 7.35 (m, 2H), 7.20 (dt, J = 7.4, 3.7 Hz, 3H), 7.17 - 7.12 (m, 1H), 7.11 - 7.04 (m, 2H), 5.25 (p, J = 7.1 Hz, 1H), 4.49 (d, J = 15.7 Hz, 1H), 4.33 (d, J = 15.7 Hz, 1H), 3.77 (s, 3H), 1.55 (d, J = 6.9 Hz, 3H).ESI-MS m/z= 440.2 (M+H)+.
**IRCBC-125:** ¹H NMR (400 MHz, Chloroform-d) δ 8.15 (d, *J=* 6.1 Hz, 1H), 7.54 - 7.49 (m, 2H), 7.46 (d, *J* = 8.2 Hz, 1H), 7.35 - 7.28 (m, 2H), 7.21 (d, *J* = 1.7 Hz, 3H), 7.17 - 7.13 (m, 1H), 7.09 (dd, *J=* 7.0, 1.8 Hz, 2H), 6.71 (d, J= 2.7 Hz, 1H), 6.38 (dd, *J= 6.1,* 2.7 Hz, 1H), 5.25 (p, *J=* 7.1 Hz, 1H), 4.49 (m, 1H), 4.34 (m, 1H), 3.77 (s, 3H), 2.96 (s, 5H), 1.54 (d, *J=* 7.0 Hz, 3H). ESI-MS m/z=465.3 (M+H)+.
**IRCBC-103:** ¹H NMR (400 MHz, Chloroform-d) δ 7.85 (s, 1H), 7.57 (d, *J* = 8.0 Hz, 2H), 7.39 (t, *J= 8.4* Hz, 2H), 7.31 (d, *J=* 6.0 Hz, 4H), 7.26 (d, *J =* 6.9 Hz, 1H), 7.19 (d, *J=* 7.4 Hz, 2H), 6.69 (dd, *J =* 8.3, 2.7 Hz, 1H), 5.35 (p, *J* = 7.0 Hz, 1H), 4.60 (m, 1H), 4.44 (m, 1H), 4.02 (t, *J* = 7.3 Hz, 4H), 3.87 (s, 3H), 2.49 (p, *J* = 7.3 Hz, 2H), 1.65 (d, *J =* 7.0 Hz, 3H). ESI-MS m/z= 477.2 (M+H)+.
**IRCBC-104:** ¹H NMR (400 MHz, Chloroform-d) δ 7.99 (s, 1H), 7.57 (d, *J* = 8.0 Hz, 3H), 7.39 (d, *J= 8.2* Hz, 2H), 7.30 (d, *J* = 5.9 Hz, 4H), 7.19 (d, *J* = 7.4 Hz, 2H), 6.78 (d, *J* = 8.6 Hz, 1H), 5.38 - 5.30 (m, 1H), 4.59 (m, 1H), 4.44 (m 1H), 3.86 (s, 3H), 3.37 (d, *J =* 4.7 Hz, 3H), 2.08 (d, *J*= 7.1 Hz, 3H), 1.64 (d, *J=* 6.9 Hz, 3H), 0.90 (d, *J=* 6.9 Hz, 2H). ESI-MS m/z=491.1 (M+H)+.
**IRCBC-145:** ¹H NMR (400 MHz, Chloroform-d) δ 8.26 (s, 1H), 7.91 (s, 1H), 7.58 (t, *J=* 8.6 Hz, 3H), 7.41 (d, *J= 7.9* Hz, 2H), 7.30 (q, *J= 9.2,* 8.1 Hz, 3H), 7.19 (d, *J=* 7.5 Hz, 2H), 5.34 (p, *J* = 7.3 Hz, 1H), 4.98 (s, 1H), 4.59 (m, 1H), 4.44 (m, 1H), 3.87 (s, 3H), 3.04 (d, *J= 4.9* Hz, 3H), 1.64 (d, *J= 6.9* Hz, 3H). ESI-MS m/z=452.3 (M+H)+.
**IRCBC-123:** ¹H NMR (400 MHz, Chloroform-d) δ 8.25 (s, 1H), 7.67 - 7.61 (m, 2H), 7.57 (d, *J= 8.1* Hz, 1H), 7.42 (d, *J* = 7.9 Hz, 2H), 7.32 - 7.29 (m, 2H), 7.25 (d, *J=* 4.6 Hz, 3H), 7.19 (d, *J=* 7.4 Hz, 2H), 5.35 (p, *J=* 7.0 Hz, 1H), 4.59 (m, 1H), 4.44 (m, 1H), 3.96 (d, *J= 1.3* Hz, 3H), 3.87 (d, *J= 1.4* Hz, 3H), 1.64 (d, *J=* 6.9 Hz, 3H). ESI-MS m/z= 903.8(2M+H)+.
**IRCBC-129:** ¹H NMR (400 MHz, Chloroform-d) δ 9.24 (s, 1H), 8.30 (s, 1H), 7.66 (d, *J=* 7.8 Hz, 3H), 7.48 (d, *J =* 7.8 Hz, 2H), 7.32 (d, *J* = 8.1 Hz, 3H), 7.26 (d, *J* = 6.9 Hz, 1H), 7.18 (d, *J* = 7.2 Hz, 2H), 5.38 (t, *J =* 7.2 Hz, 1H), 4.62 (m, 1H), 4.43 (m, 1H), 3.89 (s, 3H), 1.66 (d, *J=* 7.0 Hz, 3H). ESI-MS m/z= 463.1 (M+H)+.
**IRCBC-131:** ¹H NMR (400 MHz, Chloroform-d) δ 8.40 (dd, J = 4.4, 1.6 Hz, 1H), 7.97 (s, 1H), 7.92 (dd, J = 9.2, 1.7 Hz, 1H), 7.57 - 7.51 (m, 2H), 7.49 (d, J = 8.1 Hz, 1H), 7.39 - 7.32 (m, 2H), 7.24 - 7.18 (m, 3H), 7.18 - 7.13 (m, 1H), 7.13 - 7.07 (m, 2H), 7.04 (dd, J = 9.2, 4.4 Hz, 1H), 5.31 - 5.20 (m, 1H), 4.49 (d, J = 15.6 Hz, 1H), 4.34 (d, J = 15.6 Hz, 1H), 3.77 (s, 3H), 1.55 (d, J = 6.9 Hz, 3H). ESI-MS m/z= 462.2 (M+H)+
**IRCBC-134:** ¹H NMR (400 MHz, Chloroform-d) δ 8.32 (s, 1H), 7.61 - 7.55 (m, 3H), 7.42 (t, J = 8.2 Hz, 3H), 7.32 (d, J = 7.2 Hz, 1H), 7.26 (d, J = 6.8 Hz, 1H), 7.19 (d, J = 7.5 Hz, 2H), 7.15 (dd, J = 8.9, 2.9 Hz, 1H), 5.35 (q, J = 7.0 Hz, 1H), 4.60 (d, J = 15.6 Hz, 1H), 4.44 (d, J = 15.7 Hz, 1H), 3.87 (d, J = 1.3 Hz, 3H), 3.35 (t, J = 5.1 Hz, 4H), 2.67 (t, J = 5.2 Hz, 4H), 2.53 (q, J = 7.2 Hz, 2H), 1.64 (d, J = 6.9 Hz, 3H), 1.20 - 1.16 (m, 3H). ESI-MS m/z= 534.6 (M+H)+
**IRCBC-146:** ¹H NMR (400 MHz, Chloroform-d) δ 8.00 (d, *J=* 2.9 Hz, 1H), 7.56 (d, *J=* 8.1 Hz, 3H), 7.42 - 7.26 (m, 5H), 7.26 - 7.21 (m, 1H), 7.21 - 7.15 (m, 2H), 6.82 (dd, *J* = 8.6, 2.9 Hz, 1H), 5.33 (p, *J =* 7.1 Hz, 1H), 4.58 (m, 1H), 4.43 (m, 1H), 3.86 (s, 3H), 3.76 (s, 1H), 3.68 (s, 1H), 1.63 (d, *J= 7.0* Hz, 3H), 1.26 (d, *J=* 6.2 Hz, 6H). ESI-MS m/z= 479.2 (M+H)+.
**IRCBC-124:** ¹H NMR (400 MHz, Chloroform-d) δ 8.20 (dd, *J=* 4.5, 1.5 Hz, 1H), 7.65 - 7.60 (m, 2H), 7.58 (d, *J* = 9.4 Hz, 2H), 7.43 (d, *J* = 8.0 Hz, 2H), 7.33 - 7.31 (m, 1H), 7.28 - 7.23 (m, 2H), 7.20 (td, *J=* 4.8, 3.0 Hz, 3H), 5.38 (d, *J=* 7.2 Hz, 1H), 4.61 (d, *J= 9.9* Hz, 1H), 4.44 (d, *J=* 4.6 Hz, 1H), 3.88 (d, *J=* 3.9 Hz, 3H), 3.06 (s, 4H), 1.71 (d, *J=* 7.0 Hz, 2H), 1.65 (d, *J=* 7.0 Hz, 3H). ESI-MS m/z=465.2 (M+H)+.
**IRCBC-149:** ¹H NMR (400 MHz, Chloroform-d) δ 8.47 (s, 1H), 8.19 (d, *J=* 8.7 Hz, 1H), 7.87 (s, 1H), 7.63 (d, *J* = 7.9 Hz, 1H), 7.55 (d, *J* = 7.9 Hz, 2H), 7.49 (d, *J* = 8.6 Hz, 1H), 7.40 (d, *J* = 8.0 Hz, 2H), 7.30 - 7.21 (m, 3H), 7.17 (d, J= 7.4 Hz, 2H), 5.31 (p, J= 7.1 Hz, 1H), 4.59 (m, 1H), 4.43 (m, 1H), 3.88 (s, 3H), 2.24 (s, 3H), 1.64 (d, *J=* 7.0 Hz, 3H). ESI-MS m/z= 479.0(M+H)+.
**IRCBC-150:** ¹H NMR (400 MHz, Chloroform-d) δ 7.92 (s, 1H), 7.46 (d, *J* = 8.1 Hz, 3H), 7.27 (dd, *J=* 8.0, 5.7 Hz, 3H), 7.23 - 7.04 (m, 6H), 6.73 (dd, *J=* 8.5, 2.8 Hz, 1H), 5.23 (p, *J=* 7.1 Hz, 1H), 4.47 (m, 1H), 4.32 (m, 1H), 3.75 (d, *J* = 2.0 Hz, 3H), 1.52 (d, *J* = 7.0 Hz, 3H). ESI-MS m/z= 454.2 (M+H)+.
**IRCBC-157:** ¹H NMR (400 MHz, Chloroform-d) δ 8.27 (s, 2H), 7.55 (d, *J* = 8.0 Hz, 2H), 7.46 (d, *J* = 8.1 Hz, 1H), 7.32 (d, *J* = 8.0 Hz, 2H), 7.24 - 7.05 (m, 5H), 5.25 (p, *J* = 7.2 Hz, 1H), 4.49 (m, 1H), 4.33 (m, 1H), 3.82 (t, *J* = 4.9 Hz, 4H), 3.77 (s, 3H), 3.21 (dd, *J* = 5.8, 4.0 Hz, 4H), 1.54 (d, *J= 7.0* Hz, 3H). ESI-MS m/z= 508.0(M+H)+.
**IRCBC-158:** ¹H NMR (400 MHz, Chloroform-d) δ 8.38 (s, 2H), 7.70 - 7.63 (m, 2H), 7.58 (d, *J= 8.1* Hz, 1H), 7.49 - 7.40 (m, 2H), 7.32 - 7.25 (m, 3H), 7.20 (dd, *J =* 6.8, 1.7 Hz, 2H), 5.35 (p, *J= 7.1* Hz, 1H), 4.60 (m, 1H), 4.44 (m, 1H), 3.88 (s, 3H), 3.42 - 3.35 (m, 4H), 2.70 - 2.63 (m, 4H), 2.53 (q, *J =* 7.2 Hz, 2H), 1.65 (d, *J=* 7.0 Hz, 3H), 1.18 (t, *J =* 7.2 Hz, 3H). ESI-MS m/z= 535.3(M+H)+.
**IRCBC-259:** ¹H NMR (400 MHz, Chloroform-d) δ 7.86 (s, 2H), 7.57 - 7.50 (m, 2H), 7.46 (d, J= 8.1 Hz, 1H), 7.31 (d, J= 8.1 Hz, 2H), 7.21 (d, J= 6.7 Hz, 2H), 7.14 *(d, J=* 7.3 Hz, 1H), 7.11 - 7.05 (m, 2H), 5.24 (p, *J* = 7.1 Hz, 1H), 4.49 (m, 1H), 4.34 (m, 1H), 4.03 (t, *J* = 7.3 Hz, 2H), 3.77 (d, *J= 4.2* Hz, 5H), 3.34 - 3.23 (m, 1H), 2.16 (s, 6H), 1.54 (d, *J* = 7.0 Hz, 3H). ESI-MS m/z= 521.5 (M+H)+.
**IRCBC-260:** ¹H NMR (400 MHz, Chloroform-d) δ 8.05 (s, 2H), 7.68 - 7.62 (m, 2H), 7.58 (d, *J= 8.1* Hz, 1H), 7.42 (dd, *J* = 8.8, 6.9 Hz, 2H), 7.32 - 7.24 (m, 3H), 7.21 - 7.15 (m, 2H), 5.35 (p, *J= 7.1* Hz, 1H), 4.59 (m, 1H), 4.49 - 4.38 (m, 5H), 3.87 (s, 3H), 1.64 (d, *J =* 7.0 Hz, 3H). ESI-MS m/z= 514.3 (M+H)+.
**IRCBC-153:** ¹H NMR (400 MHz, Chloroform-d) δ 7.82 (d, *J=* 3.1 Hz, 1H), 7.51 - 7.43 (m, 3H), 7.37 - 7.27 (m, 3H), 7.21 - 7.14 (m, 3H), 7.08 (d, *J =* 7.3 Hz, 2H), 6.69 (dt, *J* = 8.6, 2.5 Hz, 1H), 5.24 (p, *J =* 7.4 Hz, 1H), 4.49 (m, 1H), 4.34 (m, 1H), 4.25 (d, *J= 2.0* Hz, 4H), 3.76 (d, *J= 2.0* Hz, 3H), 1.54 (dd, *J=* 7.0, 2.0 Hz, 3H). ESI-MS m/z= 513.2 (M+H)+.
**IRCBC-156:** ¹H NMR (400 MHz, Chloroform-d) δ 7.87 (d, *J=* 2.8 Hz, 1H), 7.65 - 7.53 (m, 3H), 7.47 - 7.35 (m, 3H), 7.35 - 7.15 (m, 5H), 6.71 (dd, *J =* 8.5, 2.9 Hz, 1H), 5.34 (p, *J* = 6.9 Hz, 1H), 4.59 (m, 1H), 4.48 - 4.37 (m, 2H), 4.25 - 4.17 (m, 2H), 3.89 - 3.81 (m, 5H), 3.38 (s, 3H), 1.64 (d, *J= 7.0* Hz, 3H). ESI-MS m/z=507.3(M+H)+.
**IRCBC-161:** ¹H NMR (400 MHz, DMSO-*d*₆) δ 8.99 (d, *J=* 8.4 Hz, 1H), 8.10 (s, 2H), 7.52 *(d, J=* 8.4 Hz, 2H), 7.47 *(d, J=* 8.3 Hz, 2H), 7.32 - 7.24 (m, 2H), 7.24 - 7.15 (m, 3H), 5.97 (s, 2H), 5.20 (p, *J* = 7.2 Hz, 1H), 4.46 (m, 1H), 4.38 (m, 1H), 3.88 (s, 3H), 1.52 (d, *J* = 7.1 Hz, 3H). ESI-MS m/z=437.9 (M+H)+.
**IRCBC-152:** ¹H NMR (400 MHz, Chloroform-d) δ 7.77 (d, *J=* 2.8 Hz, 1H), 7.51 - 7.42 (m, 3H), 7.33 - 7.24 (m, 3H), 7.21 - 7.18 (m, 2H), 7.17 - 7.14 (m, 1H), 7.09 (dd, *J =* 6.9, 1.8 Hz, 2H), 6.60 (dd, *J* = 8.5, 2.9 Hz, 1H), 5.24 (p, *J* = 7.0 Hz, 1H), 4.49 (m, 1H), 4.34 (m, 1H), 3.98 (t, *J = 7.2* Hz, 2H), 3.77 (s, 3H), 3.70 (dd, *J* = 7.5, 5.4 Hz, 2H), 3.24 (dd, *J* = 6.8, 5.4 Hz, 1H), 2.16 (s, 6H), 1.54 (d, *J= 7.0* Hz, 3H). ESI-MS m/z= 520.5(M+H)+.
**IRCBC-155:** ¹H NMR (400 MHz, Chloroform-d) δ 7.86 (d, *J=* 2.8 Hz, 1H), 7.54 (t, *J* = 8.5 Hz, 3H), 7.39 (dd, J= 8.4, 6.7 Hz, 3H), 7.28 - 7.21 (m, 3H), 7.18 - 7.13 (m, 2H), 6.72 (dd, *J=* 8.5, 2.9 Hz, 1H), 5.31 (p, *J =* 7.2 Hz, 1H), 4.56 (m, 1H), 4.41 (m, 1H), 4.29 (dd, *J =* 8.5, 7.3 Hz, 2H), 4.20 (dd, *J=* 7.3, 6.2 Hz, 2H), 3.84 (s, 3H), 3.67 (tt, *J=* 8.5, 6.2 Hz, 1H), 1.61 (d, *J =* 7.0 Hz, 3H). ESI-MS m/z=502.3 (M+H)+.
**IRCBC-160:** ¹H NMR (400 MHz, Chloroform-d) δ 8.14 (s, 2H), 7.68 - 7.61 (m, 2H), 7.58 (d, *J= 8.1* Hz, 1H), 7.47 - 7.39 (m, 2H), 7.30 (d, *J=* 2.9 Hz, 2H), 7.25 (d, *J= 7.4* Hz, 1H), 7.22 - 7.16 (m, 2H), 5.35 (p, *J* = 7.1 Hz, 1H), 4.60 (m, 1H), 4.44 (m, 1H), 4.07 (d, *J* = 5.4 Hz, 1H), 3.88 (s, 3H), 2.97 (d, *J =* 5.3 Hz, 3H), 1.64 (s, 3H). ESI-MS m/z= 452.3 (M+H)+.
**IRCBC-257:** ¹H NMR (400 MHz, Chloroform-d) δ 8.08 (s, 1H), 7.58 - 7.51 (m, 2H), 7.47 (d, *J= 8.1* Hz, 1H), 7.33 (d, *J=* 7.8 Hz, 2H), 7.25 - 7.11 (m, 3H), 7.11 - 7.05 (m, 2H), 6.93 (t, *J=* 2.3 Hz, 1H), 5.24 (t, *J=* 7.3 Hz, 1H), 4.49 (m, 1H), 4.33 (m, 1H), 4.18 (s, 1H), 3.77 (d, *J=* 1.9 Hz, 3H), 1.58 - 1.53 (m, 3H). ESI-MS m/z= 479.3(M+H)+.
**IRCBC-256:** ¹H NMR (400 MHz, Chloroform-d) δ 8.12 (d, *J=* 3.8 Hz, 2H), 7.63 (dd, *J* = 8.8, 3.3 Hz, 2H), 7.59 (dd, *J =* 8.0, 3.8 Hz, 1H), 7.41 (d, *J= 7.9* Hz, 2H), 7.35 - 7.23 (m, 3H), 7.23 - 7.15 (m, 2H), 5.34 (t, *J* = 7.3 Hz, 1H), 4.59 (m, 1H), 4.43 (m, 1H), 4.18 (s, 1H), 3.87 (d, *J= 4.3* Hz, 3H), 1.64 (d, *J=* 6.9 Hz, 3H). ESI-MS m/z= 455.2(M+H)+.
**IRCBC-258:** ¹H NMR (400 MHz, Chloroform-d) δ 8.14 (d, *J= 2.9* Hz, 1H), 7.61 - 7.51 (m, 2H), 7.47 (d, *J* = 8.0 Hz, 1H), 7.33 (d, *J* = 8.1 Hz, 2H), 7.21 (d, *J* = 9.1 Hz, 2H), 7.17 - 7.13 (m, 1H), 7.14 - 7.03 (m, 3H), 5.24 (p, *J =* 7.0 Hz, 1H), 4.49 (m, 1H), 4.33 (m, 1H), 4.05 (s, 2H), 3.77 (s, 3H), 1.54 (d, *J=* 7.0 Hz, 3H). ESI-MS m/z= 462.2(M+H)+.
**IRCBC-261:** ¹H NMR (400 MHz, Chloroform-d) δ 8.02 (s, 2H), 7.66 (d, *J=* 8.1 Hz, 2H), 7.58 *(d, J=* 8.1 Hz, 1H), 7.43 *(d, J=* 8.1 Hz, 2H), 7.32 *(d, J=* 7.3 Hz, 1H), 7.26 (d, *J* = 6.9 Hz, 1H), 7.19 (d, *J* = 7.4 Hz, 2H), 5.39 - 5.31 (m, 1H), 4.60 (m, 1H), 4.48 - 4.36 (m, 3H), 4.32 (t, *J* = 6.8 Hz, 2H), 3.88 (s, 3H), 3.84 - 3.72 (m, 1H), 1.65 (d, *J =* 7.0 Hz, 4H). ESI-MS m/z= 503.4(M+H)+.
**IRCBC-262:** ¹H NMR (400 MHz, Chloroform-d) δ 7.94 (s, 2H), 7.64 (d, *J=* 8.2 Hz, 2H), 7.57 (d, *J=* 8.2 Hz, 1H), 7.42 (d, *J =* 8.2 Hz, 2H), 7.32 (d, *J =* 6.8 Hz, 2H), 7.25 *(t, J=* 7.3 Hz, 1H), 7.22 - 7.16 (m, 2H), 5.35 (p, *J =* 7.3 Hz, 1H), 4.60 (m, 1H), 4.44 (m, 1H), 4.06 (t, *J =* 7.4 Hz, 2H), 3.91 - 3.83 (m, 5H), 3.70 - 3.60 (m, 1H), 3.34 (t, *J= 7.1* Hz, 4H), 2.19 (p, *J* = 7.0 Hz, 2H), 1.64 (d, *J= 7.0* Hz, 3H). ESI-MS m/z=533.6 (M+H)+.
**IRCBC-269:** ¹H NMR (400 MHz, Chloroform-d) δ 8.07 (s, 2H), 7.61 (d, *J =* 8.2 Hz, 2H), 7.54 (s, 1H), 7.38 *(d, J=* 8.2 Hz, 2H), 7.28 (d, J= 6.9 Hz, 1H), 7.24 - 7.19 (m, 2H), 7.17 - 7.13 (m, 2H), 5.34 (d, *J=* 5.0 Hz, 1H), 4.56 (m, 1H), 4.43 (s, 1H), 3.84 (s, 3H), 3.75-3.70 (m, 2H), 1.61 (d, *J= 7.0* Hz, 3H), 1.24 (d, *J=* 7.0 Hz, 6H). ESI-MS m/z=480.1 (M+H)+.
**IRCBC-271:** ¹H NMR (400 MHz, Chloroform-d) δ 8.19 (s, 2H), 7.58 - 7.51 (m, 2H), 7.46 (d, *J= 8.1* Hz, 1H), 7.35 - 7.28 (m, 2H), 7.20 (s, 3H), 7.09 (dd, *J* = 6.9, 1.9 Hz, 2H), 5.25 (p, *J* = 7.2 Hz, 1H), 4.49 (m, 1H), 4.34 (m 1H), 4.29 (s, 1H), 3.77 (s, 3H), 2.45 (pd, *J* = 4.3, 3.1 Hz, 1H), 1.54 (d, *J= 6.9* Hz, 3H), 0.91 - 0.75 (m, 2H), 0.56 - 0.48 (m, 2H). ESI-MS m/z= 478.3(M+H)+.
**IRCBC-276:** ¹H NMR (400 MHz, Chloroform-d) δ 7.86 (s, 2H), 7.57 - 7.50 (m, 2H), 7.46 (d, *J= 8.1* Hz, 1H), 7.35 - 7.28 (m, 2H), 7.21 - 7.18 (m, 2H), 7.16 - 7.13 (m, 1H), 7.10 - 7.06 (m, 2H), 5.24 (p, *J=* 7.1 Hz, 1H), 4.80 (s, 4H), 4.49 (m, 1H), 4.33 (m, 1H), 4.13 (s, 4H), 3.77 (s, 3H), 1.54 (d, *J= 7.0* Hz, 3H). ESI-MS m/z= 520.3(M+H)+.

### Biological Test Example 1: Inhibitory Activity Test of RIPK1 Inhibitors on Programmed Cell Necrosis

The biological test protocol is adopted to test effects of the compound on TNF-induced programmed necrosis of FADD (Fas-associated death domain)-deficient Jurkat cells and L929 cells.

In order to verify the inhibitory effect of the compounds according to the present disclosure on programmed cell necrosis at cellular level, cell types closely associated with RIP1 pathway were selected, namely FADD-deficient Jurkat cells (human peripheral blood leukemia T cell line) and L929 cells. Two different stimulation methods were used: treating with tumor necrosis factor (TNFα) alone, or treating with TNFα in combination of mitochondrial-derived cysteine-aspartate activator (SMAC) SM164. Cell viability was determined by measuring chemiluminescence values, thereby assessing the bioactivity of the compounds in inhibiting programmed cell necrosis.

Method: FADD-deficient Jurkat cells: FADD-deficient Jurkat cells (human peripheral blood leukemia T cell line) were cultured in vitro. After reaching logarithmic growth phase, cells were collected and centrifuged at 1000 rpm for 5 minutes. A supernatant was discarded. A cell concentration was adjusted to 2.5×10⁵/mL, and cells were seeded into a 384-well plate at 40 µL per well. In the corresponding wells, 5 µL of SM164 (50 nM) and compounds diluted with cell culture medium were added. After pre-treatment at 37°C for 1 hour, 5 µL of TNFα (50 ng/mL) diluted with cell culture medium was added to each well as a stimulation group, while 5 µL of culture medium was added as a control group. After the plate was incubated in a cell culture incubator (37°C, 5% CO₂) for 14 hours, 15 µL of Cell Titer-Glo solution was added to each well. Then, the plate was incubated at room temperature for 30 minutes. Luminescence was measured to assess intracellular ATP levels. Cell viability was set at 100% for unstimulated DMSO control wells. L929 cells: L929 cells (mouse fibroblasts) were cultured in vitro, digested, and diluted to 6.25×10⁴/ml, then seeded into a 384-well plate at 40 µl per well. The plate was placed in a cell culture incubator (37°C, 5% CO₂) and cultured for 12 hours. In the corresponding wells, 5 µL of SM164 (500 nM) and compounds diluted with cell culture medium were added. After pre-treatment at 37°C for 1 hour, 5 µL of TNFα (500 ng/mL) diluted with cell culture medium was added to the wells as a stimulation group, while 5 µL of culture medium was added as a control group. After the plate was incubated in the cell incubator (37°C, 5% CO₂) for 14 hours, 15 µL of Cell Titer-Glo solution was added to each well. Then, the plate was incubated at room temperature for 30 minutes, and luminescence was measured to assess intracellular ATP levels. Cell viability was set at 100% for unstimulated DMSO control wells. EC₅₀ values of the compounds were calculated using Prism GraphPad statistical software. Results are shown in Table 2.

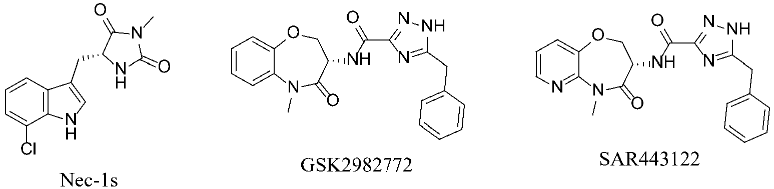

**Table 2. Test results of RIPK1 inhibitors on inhibition of programmed cell necrosis**

| Compound No. | EC₅₀ (nM) | | | |
|---|---|---|---|---|
| | FADD^{-/-} Jurkat cells (human-derived) | | L929 cells (mouse-derived) | |
| | TNFα | TNFα+ SM164 | TNFα | TNFα+ SM164 |
| Nec-1s | 228 | 900 | 443 | 1896 |
| GSK2982772 | 0.7 | 4.0 | >1000 | >1000 |
| SAR443122 | 0.9 | 7.0 | >1000 | >1000 |
| QY-18-25 | <0.01 | 0.02 | 0.3 | 1.2 |
| QY-18-26 | <0.01 | <0.01 | <0.01 | <0.01 |
| QY-18-27 | <0.01 | 0.01 | 0.04 | 0.06 |
| QY-18-43 | 0.02 | 0.06 | 0.05 | 0.08 |
| QY-18-44 | 0.04 | 0.3 | 0.2 | 0.3 |
| QY-18-47 | 0.7 | 61 | 1.0 | 73 |
| QY-18-77 | 0.3 | 32 | 733 | |
| QY-20-2 | 0.03 | 0.3 | 0.1 | 0.5 |
| QY-20-53 | <0.01 | 0.05 | 0.03 | 0.1 |
| QY-20-54 | <0.01 | <0.01 | 0.05 | 0.3 |
| QY-20-55 | 0.1 | 1.4 | 2.2 | 23 |
| IRCBC-002 | 0.392 | 23.26 | 55.663 | 118.39 |
| IRCBC-003 | 0.095 | 2.33 | 1.114 | 7.7 |
| IRCBC-004 | 0.019 | 0.661 | 0.558 | 1.51 |
| IRCBC-005 | 0.019 | 1.118 | 2.092 | 3.416 |
| IRCBC-006 | 2.026 | 67.1 | 81.903 | 374.37 |
| IRCBC-007 | 0.072 | 0.77 | 0.631 | 2 |
| IRCBC-008 | 0.055 | 0.676 | 1.858 | 35.72 |
| IRCBC-010 | 0.481 | 13.444 | 3.265 | 6.712 |
| IRCBC-016 | 1.693 | 16.414 | 5.851 | 20.27 |
| IRCBC-033 | 0.066 | 1.566 | 0.771 | 4.10654 |
| IRCBC-036 | 0.222 | 12.567 | 13.054 | 57.442 |
| IRCBC-041 | 0.004 | 0.543 | 0.771 | 2.122605 |
| IRCBC-043 | 0.683 | 5.111 | >1µM | >1µM |
| IRCBC-046 | 0.001 | 0.087 | 0.203 | 0.43 |
| IRCBC-048 | 0.018 | 1.92 | 2.932 | 8.638 |
| IRCBC-049 | 0.002 | 0.111 | 0.118 | 0.485 |
| IRCBC-050 | 0.001 | 0.104 | 0.105 | 0.202 |
| IRCBC-051 | 0.006 | 0.845 | 1.623 | 4.529 |
| IRCBC-052 | 0.001 | 0.033 | 0.068 | 0.13 |
| IRCBC-053 | 0.000014 | 0.001 | 0.004 | 0.011 |
| IRCBC-054 | 0.001 | 0.097 | 0.103 | 0.338 |
| IRCBC-057 | 0.537 | 3.896 | 12.555 | 35.516 |
| IRCBC-058 | 0.018 | 1.253 | 1.503 | 35.23 |
| IRCBC-059 | 0.025 | 2.261 | 3.068 | 14.787 |
| IRCBC-067 | 0.004 | 0.261 | 0.264 | 0.887 |
| IRCBC-071 | 0.006 | 0.641 | 0.914 | 2.99 |
| IRCBC-072 | 0.049 | 2.725 | 1.786 | 17.281 |
| IRCBC-091 | 0.004 | 0.145 | 0.455 | 1.293 |
| IRCBC-093 | 0.001 | 0.262 | 0.374 | 1.482 |
| IRCBC-094 | 0.001 | 0.304 | 0.403 | 2.162 |
| IRCBC-095 | 0.0001 | 0.002 | 0.01 | 0.052 |
| IRCBC-096 | 0.006 | 0.406 | >1µM | >1µM |
| IRCBC-097 | 0.00002 | 0.0005 | 0.001 | 0.003 |
| IRCBC-098 | 0.00004 | 0.004 | 0.011 | 0.062 |
| IRCBC-099 | 0.195 | 3.457 | 0.399 | 0.734 |
| IRCBC-100 | 0.084 | 1.789 | 0.561 | 1.213 |
| IRCBC-101 | 0.036 | 0.582 | 0.405 | 2.103 |
| IRCBC-102 | 0.00007 | 0.0005 | 0.01 | 0.018 |
| IRCBC-103 | 0.00005 | 0.001 | 0.044 | 0.104 |
| IRCBC-104 | 0.0001 | 0.002 | 0.071 | 0.12 |
| IRCBC-105 | 0.00003 | 0.0002 | 0.008 | 0.004 |
| IRCBC-106 | 0.004 | 0.256 | 0.139 | 0.3 |
| IRCBC-119 | 0.164 | 5.794 | 20.85 | 70.884 |
| IRCBC-120 | 0.002 | 0.079 | 0.438 | 1.74 |
| IRCBC-121 | 0.0001 | 0.01 | 0.012 | 0.069 |
| IRCBC-122 | 0.0005 | 0.035 | 0.026 | 0.032 |
| IRCBC-123 | 0.00013 | 0.004 | 0.246 | 1.054 |
| IRCBC-124 | 0.0002 | 0.034 | 1.932 | 5.871 |
| IRCBC-125 | 0.01 | 0.145 | 0.445 | 1.678 |
| IRCBC-129 | 0.00007 | 0.00034 | 0.06 | 0.196 |
| IRCBC-131 | 0.003 | 0.16 | 2.046 | 8.754 |
| IRCBC-134 | 0.00006 | 0.002 | 0.001 | 0.005 |
| IRCBC-145 | 0.00004 | 0.007 | 0.063 | 0.127 |
| IRCBC-146 | 0.00007 | 0.0002 | 0.0004 | 0.0014 |
| IRCBC-149 | <0.00002 | 0.003 | 0.045 | 0.066 |
| IRCBC-150 | <0.00002 | 0.0003 | 0.001 | 0.001 |
| IRCBC-152 | <0.00002 | 0.0003 | 0.0009 | 0.0009 |
| IRCBC-153 | <0.00002 | <0.00002 | 0.0008 | 0.0001 |
| IRCBC-154 | 0.00002 | 0.0004 | 0.0006 | 0.002 |
| IRCBC-155 | <0.00002 | <0.00002 | 0.0002 | <0.00002 |
| IRCBC-156 | <0.00002 | 0.0004 | 0.0006 | 0.00055 |
| IRCBC-157 | 0.00004 | 0.0005 | 0.0002 | 0.0005 |
| IRCBC-158 | 0.00007 | 0.0005 | 0.0003 | 0.0007 |
| IRCBC-159 | 0.0003 | 0.002 | 0.003 | 0.006 |
| IRCBC-160 | 0.000045 | 0.000278 | 0.000646 | 0.002343 |
| IRCBC-161 | <0.00002 | 0.0002 | 0.002 | 0.002 |
| IRCBC-256 | 0.000141 | 0.000555 | 0.000764 | 0.001212 |
| IRCBC-257 | 0.000008 | 0.000103 | 0.000347 | 0.001172 |
| IRCBC-258 | <0.00002 | 0.000024 | 0.00012 | 0.000259 |
| IRCBC-259 | 0.00009 | 0.0005 | 0.00003 | 0.001 |
| IRCBC-260 | 0.00006 | 0.0001 | 0.00002 | 0.0003 |
| IRCBC-261 | <0.00002 | 0.000036 | 0.000097 | 0.000159 |
| IRCBC-262 | 0.000018 | 0.000055 | 0.000101 | 0.000242 |
| IRCBC-269 | 0.000012 | 0.001676 | 0.000126 | 0.000659 |
| IRCBC-271 | 0.000038 | 0.011086 | 0.000765 | 0.006176 |
| IRCBC-276 | 0.000032 | 0.000208 | 0.000071 | 0.000099 |

Experimental results in Table 2 indicate that, no matter they were stimulated with TNFα alone, or stimulated with TNFα in combination of SM164, preferred compounds of the present disclosure, such as QY-18-26, QY-18-27, QY-20-53, QY-20-54, etc. all exhibited stronger programmed necroptosis inhibitory activity on human derived FADD-deficient Jurkat cells compared to a clinical inhibitor SAR443122; moreover, on mouse derived L929 cells where SAR443122 was nearly inactive, the preferred compounds of the present disclosure were still able to effectively inhibit programmed necroptosis.

### Biological test Example 2: Testing the effect of a representative compound QY-18-26 on RIPK1 kinase activity

A biological test protocol was adopted to test effects of the compound QY-18-26 on kinase activity of RIPK1 (1-330) protein. Purified RIPK1 (1-330) protein in vitro retained complete kinase activity domain and maintained good kinase activity. A known clinical **RIPK1** inhibitor SAR443122 was used as a control.

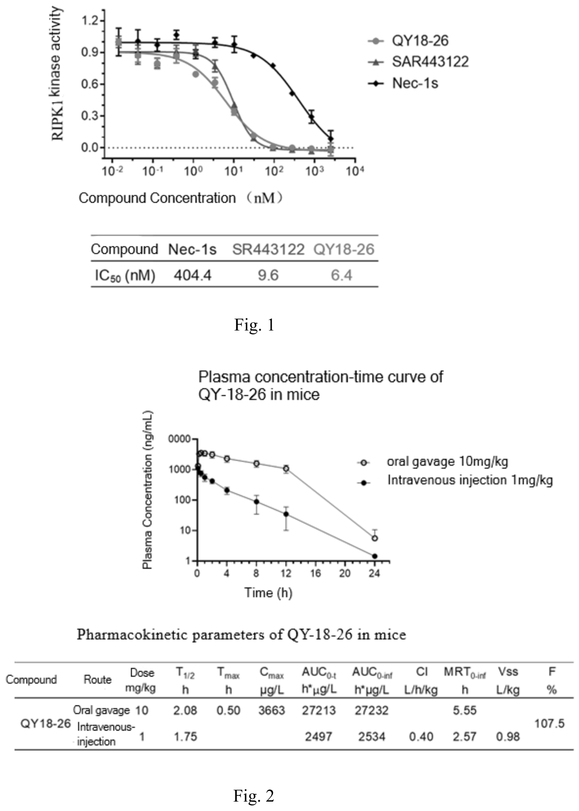

Method: Into a 384-well plate, RIPK1 (1-330) protein with a final concentration of 2 µM and ATP with a corresponding concentration (in 1X kinase buffer) with a final concentration of 5 µL were added. At least 3 replicates were set for each group and reacted at 37°C for 2 hours. 5 µL of ADP-Glo reagent was added to stop the kinase reaction and remove any remaining ATP in the reaction system. The plate was incubated at room temperature for 40 minutes, added with 10 µL of Kinase Detection Reagent, which was used to convert ADP to ATP and introduce luciferase and luciferin to detect ATP in the system, and reacted at room temperature for 1 hour. Luminescence was measured using a 7500 Fast Real-Time PCR System. IC₅₀ values of compounds in inhibiting the kinase reaction were calculated using Prism GraphPad statistical software. Experimental results are shown in Fig.1.

The experimental results show that the representative compound QY-18-26 exhibits concentration-dependent effective inhibition against **RIPK1** kinase, with an effective half-maximal inhibitory concentration of 6.4 nM, which is superior to the control SAR443122 (effective half-maximal inhibitory concentration of 9.6 nM).

### Biological test Example 3: Pharmacokinetic properties of a representative compound QY-18-26

A biological test protocol was adopted to test drug metabolism of the compound in living mice.

To verify the pharmacokinetic properties of the compounds of the present disclosure in vivo, the representative compound QY-18-26 was selected. Single administration via oral gavage (PO, 10 mg/kg) or intravenous injection (IV, 1 mg/kg) was used to test the pharmacokinetic properties of the compound in mice (n = 3). Experimental results are shown in Fig. 2.

The results indicate that after a single dose of QY-18-26, it rapidly reached a peak concentration in mice and exhibited a very high maximum plasma concentration (Cₘₐₓ), exposure (AUC), and bioavailability (F). After oral gavage administration of 10 mg/kg, the plasma concentration remained above an effective inhibitory concentration (5 ng/mL) for most of the 24-hour period.

### Biological test Example 4: Brain penetration properties of a representative compound QY-18-26

A biological test protocol was adopted to test brain/plasma drug concentrations of the compound in living mice and rats.

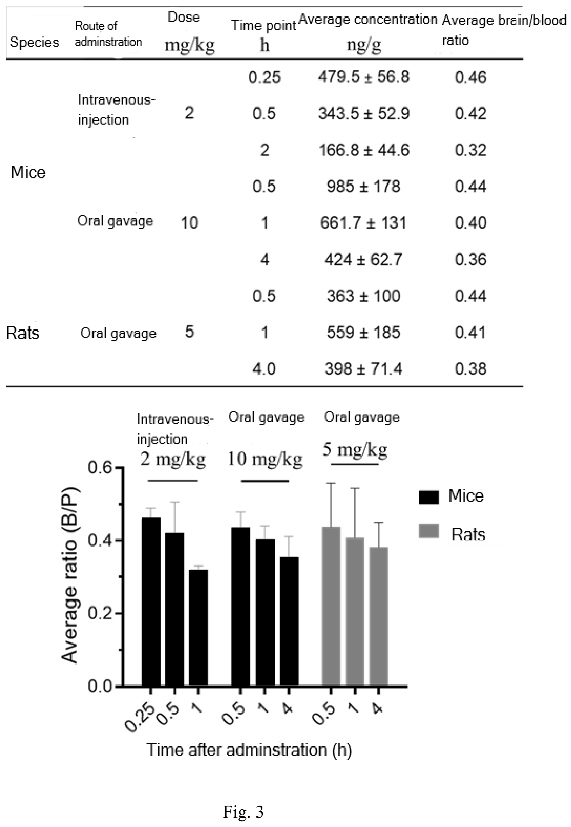

To verify the brain penetration properties of the compounds of the present disclosure in vivo, a representative compound QY-18-26 was selected. Single administration via oral gavage (PO, 10 mg/kg) or intravenous injection (IV, 2 mg/kg) was used to test brain/plasma drug concentrations of the compound in mice (n = 3) at different time points. At the same time, single administration via oral gavage (PO, 5 mg/kg) was used to test brain/plasma drug concentrations in rats (n = 3) at different time points. Experimental results are shown in Fig. 3.

After a single dose of QY-18-26, the concentration in the brains of rats and mice was about 40% of the concentration in plasma, and the brain-plasma distribution remained stable at 4 hours. The brain drug concentration was well above the effective inhibitory concentration (5 ng/g), indicating that this compound had ideal brain-penetration properties.

### Biological test Example 5: Effect of a representative compound QY-18-26 on TNFα-induced systemic inflammatory response syndrome

A biological test protocol was adopted as follows: systemic inflammatory response syndrome (SIRS), also known as an inflammatory storm, refers to a nonspecific systemic inflammatory response caused by severe infections, multiple trauma, burns, ischemia-reperfusion, acute pancreatitis, and other infectious or non-infectious injuries. Under this condition, a large number of inflammatory factors are released, and in severe cases, the body loses control over the inflammatory response, leading to multiple organ failure and even death. Tail vein injection of tumor necrosis factor TNFα can induce systemic inflammatory response syndrome in mice, causing a drop in body temperature and death of mice. Effects of the representative compound QY-18-26 on TNFα-induced systemic inflammatory response syndrome were tested by monitoring changes in body temperature and death in mice. A known RIPK1 inhibitor Nec-1s was used as a control. The clinical inhibitor SAR443122 had very poor inhibitory activity against mouse derived RIPK1 and was therefore not used.

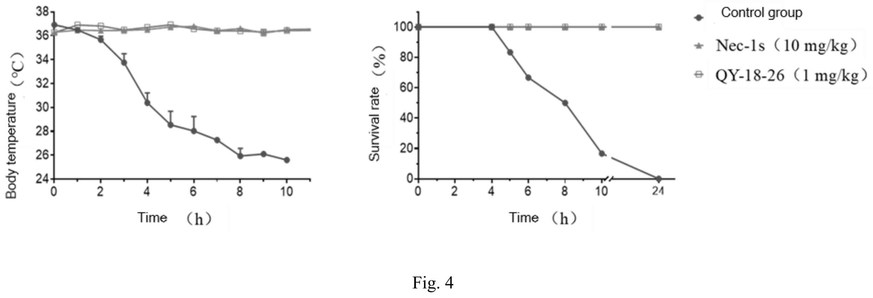

Method: The compound to be tested was dissolved in 0.5% carboxymethyl cellulose at the required concentration one day in advance and sonicated overnight. 200 µl was administered via oral gavage to each mouse, and 20 minutes later, 10 mg of TNFα (dissolved in 125 µl of PBS) was administered via tail vein injection. Body temperature changes of mice were monitored using an infrared thermometer after injection. Effects of the compound were calculated using Prism GraphPad statistical software. Experimental results are shown in Fig. 4.

The experimental results show that in the absence of inhibitors, all mice injected with TNFα died within hours; the compound QY-18-26 can effectively counteract TNFα-induced body temperature drop, inflammatory response, and death at a low dose (1 mg/kg).

## Claims

1. A compound, or a pharmaceutically acceptable salt, a hydrate, or a solvate thereof, wherein the compound is represented by Formula I: 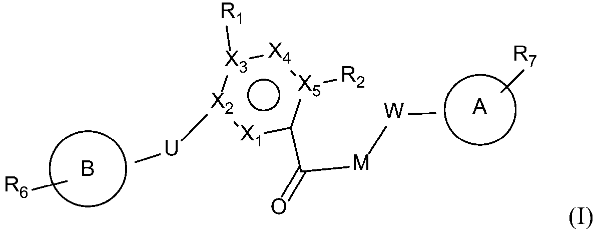 wherein:
X₁ is selected from the group consisting of: CH, N and a chemical bond;
X₂, X₃, X₄ and X₅ are each independently selected from the group consisting of: CH, and N;
with a proviso that a ring formed by X₁, X₂, X₃, X₄ and X₅ is an aromatic ring;
M is selected from the group consisting of: O, S, NR₃, CHR₃ and C(R₃)₂;
W and U are each independently selected from the group consisting of: O, S, NR₄, CHR₄ and C(R₄)₂;
ring A and ring B are each independently selected from the group consisting of: substituted or unsubstituted C6-C10 aryl, and substituted or unsubstituted 5-12 membered heteroaryl;
R₁ and R₂ are each independently selected from the group consisting of: none, H, substituted or unsubstituted C1-C6 alkyl, and halogen;
and when M is NR₃, CHR₃ or C(R₃)₂, R₂, R₃ can form a substituted or unsubstituted 5-7 membered ring together with the C or N atoms they are connected to, as well as -C-C(O)-;
R₃ and R₄ are selected from the group consisting of: H, substituted or unsubstituted C1-C6 alkyl, and halogen;
R₆ is selected from the group consisting of: H, halogen, CN, substituted or unsubstituted C1-C6 alkyl, substituted or unsubstituted C2-C6 alkenyl, substituted or unsubstituted C2-C6 alkynyl, and -CH≡CR₅; wherein, R₅ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
R₇ is selected from the group consisting of: H, halogen, CN, substituted or unsubstituted C1-C6 alkyl, substituted or unsubstituted C2-C6 alkenyl, substituted or unsubstituted C2-C6 alkynyl, and -CH≡CR₈; wherein, R₈ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
wherein, said substituted refers to the hydrogen atoms on the group are replaced by one or more (such as 2, 3, 4 and the like) substituents selected from the group consisting of: halogen, C1-C6 alkyl, halogenated C1-C6 alkyl, C3-C8 cycloalkyl, halogenated C3-C8 cycloalkyl, C1-C6 alkoxy, halogenated C1-C6 alkoxy, C1-C6 hydroxyalkyl, methylsulfonyl, -S(=O)₂NH₂, oxo (=O), -CN, hydroxy, -NH₂, carboxyl, C2-C6 acylamino (-C(=O)-N(Rc)₂ or -NH-C(=O) (Rc), Rc is H or C1-C5 alkyl), C1-C6 alkyl-(C2-C6 acylamino), C1-C6 amino, deuterated C1-C6 amino, -NHRd (Rd is C3-C8 cycloalkyl, a 4-7 membered heterocyclic group or a heterocyclic group substituted with C1-C6 alkyl), C6-C10 aryl, a 5-7 membered heteroaryl group having 1-3 heteroatoms selected from N, S and O, a 4-8 membered heterocyclic group having 1-3 heteroatoms selected from N, S and O, and a 4-7 membered heterocyclic group substituted with 1 or 2 Re (Re is halogen, C1-C6 alkyl, C1-C6 amino, -CN, C1-C6 alkoxy or a 4-7 membered heterocyclic group).

2. The compound according to claim 1, or a pharmaceutically acceptable salt, a hydrate, or a solvate thereof, wherein the compound is represented by Formula II: 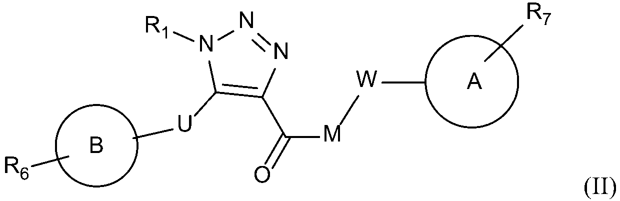 wherein M, W, U, A, B, R₁, R₆, R₇ are as defined according to claim 1.

3. The compound according to claim 1, or a pharmaceutically acceptable salt, a hydrate, or a solvate thereof, wherein the ring A and ring B are each independently selected from the group consisting of: substituted or unsubstituted phenyl, and substituted or unsubstituted 5-7 membered heteroaryl;
wherein said substituted is as defined according to claim 1.

4. The compound according to claim 1, or a pharmaceutically acceptable salt, a hydrate, or a solvate thereof, wherein the ring A and ring B are each independently selected from the group consisting of:

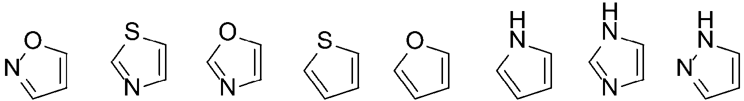

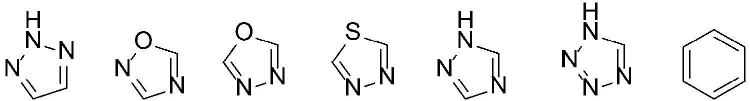

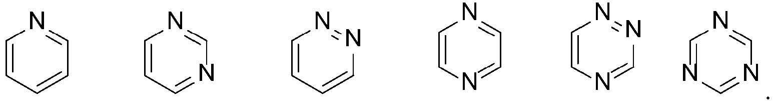

5. The compound according to claim 1, or a pharmaceutically acceptable salt, a hydrate, or a solvate thereof, wherein, in Formula **I,** M is NR₃, and/or
W is CHR₄;
R₆ is selected from the group consisting of: H, halogen, CN, and substituted or unsubstituted C1-C6 alkyl;
R₇ is selected from the group consisting of: H, halogen, CN, substituted or unsubstituted C1-C6 alkyl, substituted or unsubstituted C2-C6 alkynyl, and -CH≡CR₈; wherein, R₈ is selected from the group consisting of: substituted or unsubstituted C1-C6 alkyl, substituted or unsubstituted C6-C10 aryl, substituted or unsubstituted 5-12 membered heteroaryl, substituted or unsubstituted C3-C8 cycloalkyl, and a substituted or unsubstituted 4-12 membered heterocyclic group;
R₃, R₄, and said substituted are as defined according to claim 1.

6. The compound according to claim 1, or a pharmaceutically acceptable salt, a hydrate, or a solvate thereof, wherein the compound is represented by Formula III: 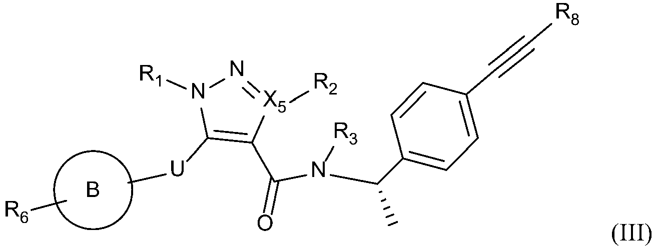 wherein M, W, U, A, B, X₅, R₁, R₂, R₃, R₆, R₈ are as defined according to claim 1.

7. The compound according to claim 1, wherein the compound is selected from the compounds listed in the following Table:
| Compound No. | Chemical structure | Compound No. | Chemical structure |
|---|---|---|---|
| QY-17-24 | 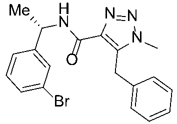 | QY-17-25 | 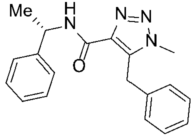 |
| QY-18-15 | 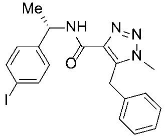 | QY-18-25 | 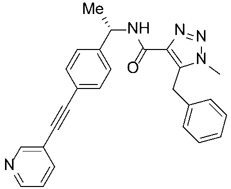 |
| QY-18-26 | 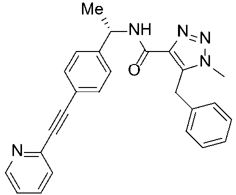 | QY-18-27 | 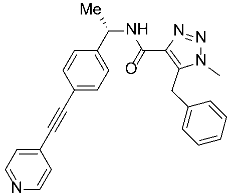 |
| QY-18-28 | 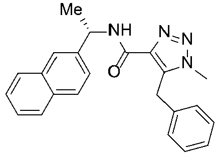 | QY-18-41 | 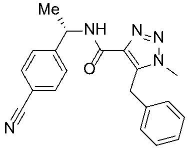 |
| QY-18-42 | 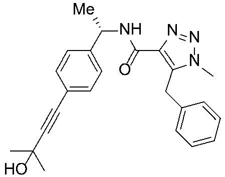 | QY-18-43 | 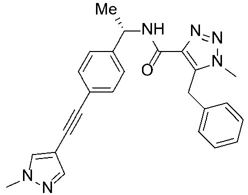 |
| QY-18-44 | 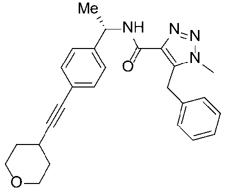 | QY-18-47 | 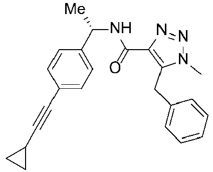 |
| QY-18-49 | 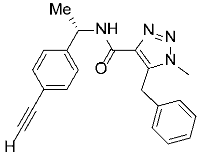 | QY-18-63 | 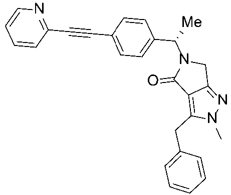 |
| QY-18-77 | 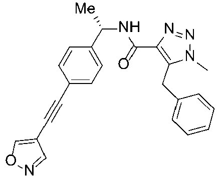 | QY-18-102 | 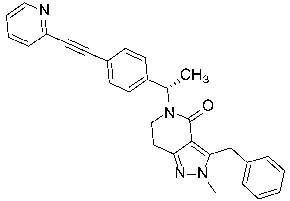 |
| QY-19-53 | 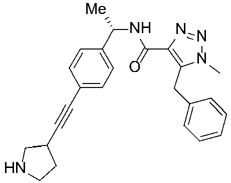 | QY-19-54 | 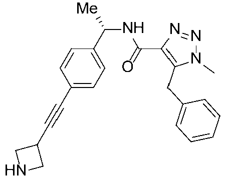 |
| QY-19-55 | 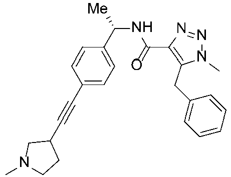 | QY-19-56 | 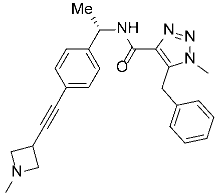 |
| QY-19-60 | 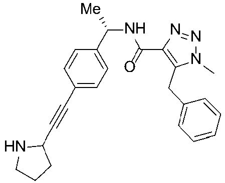 | QY-19-62 | 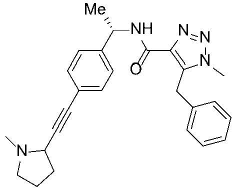 |
| SYL-30-52 | 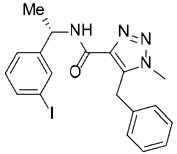 | SYL-30-56 | 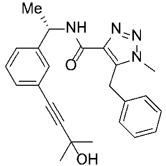 |
| SYL-30-58 | 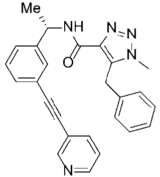 | SYL-30-62 | 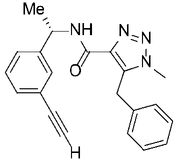 |
| SYL-30-65 | 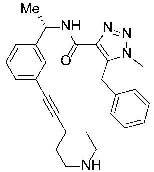 | QY-20-1 | 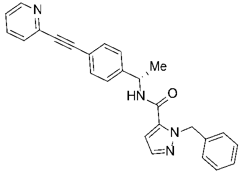 |
| QY-20-2 | 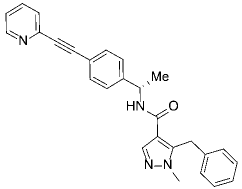 | QY-20-5 | 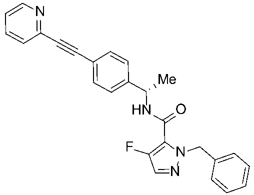 |
| QY-20-18 | 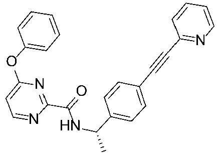 | QY-20-53 | 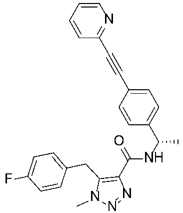 |
| QY-20-54 | 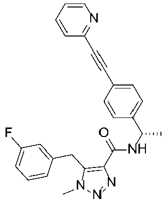 | QY-20-55 | 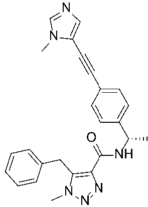 |
| QY-20-67 | 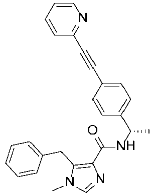 | QY-20-72 | 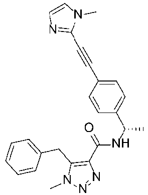 |
| QY-20-73 | 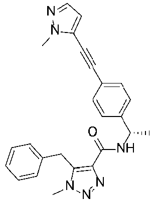 | QY-20-74 | 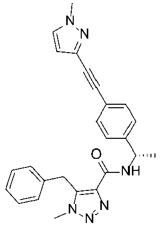 |
| QY-20-76 | 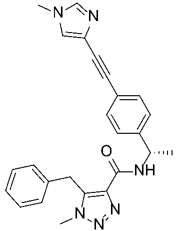 | QY-20-80 | 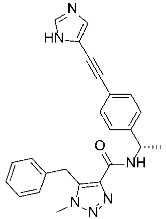 |
| QY-20-81 | 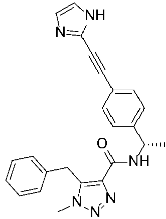 | QY-20-82 | 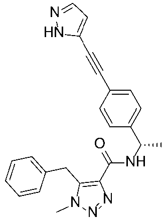 |
| IRCBC-002 | 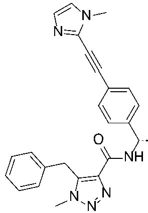 | IRCBC-003 | 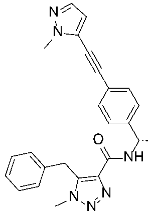 |
| IRCBC-004 | 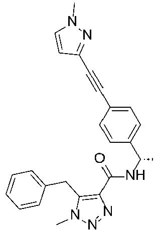 | IRCBC-005 | 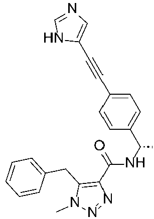 |
| IRCBC-006 | 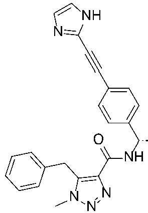 | IRCBC-010 | 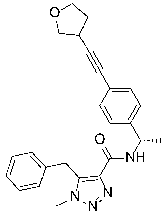 |
| IRCBC-007 | 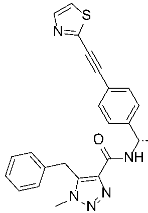 | IRCBC-016 | 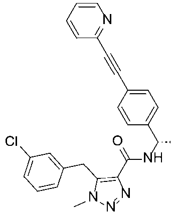 |
| IRCBC-033 | 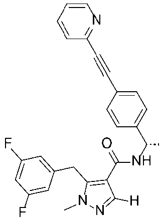 | IRCBC-050 | 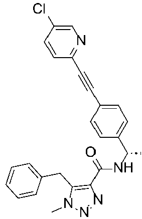 |
| IRCBC-043 | 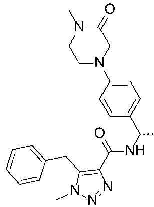 | IRCBC-054 | 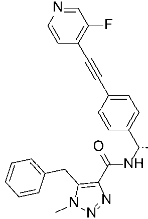 |
| IRCBC-041 | 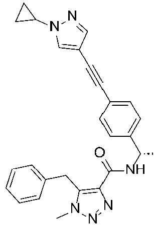 | IRCBC-046 | 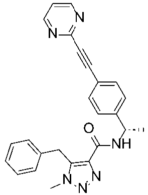 |
| IRCBC-049 | 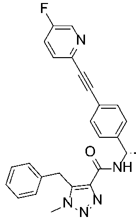 | IRCBC-048 | 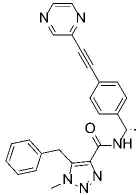 |
| IRCBC-052 | 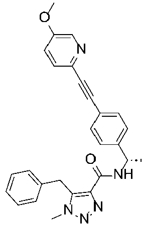 | IRCBC-067 | 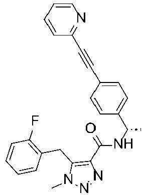 |
| IRCBC-032 | 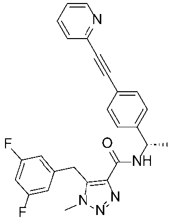 | IRCBC-053 | 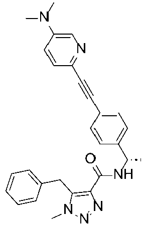 |
| IRCBC-051 | 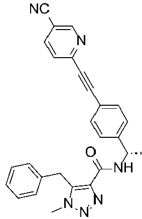 | IRCBC-058 | 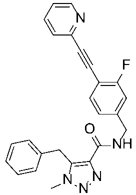 |
| IRCBC-059 | 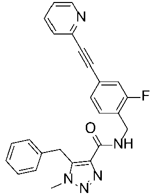 | IRCBC-071 | 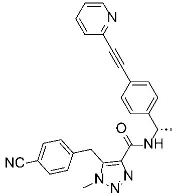 |
| IRCBC-072 | 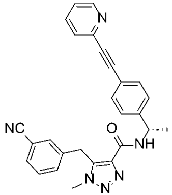 | IRCBC-095 | 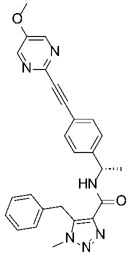 |
| IRCBC-096 | 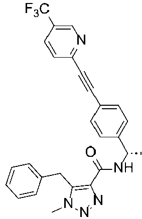 | IRCBC-094 | 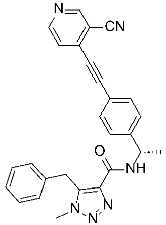 |
| IRCBC-093 | 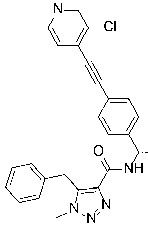 | IRCBC-101 | 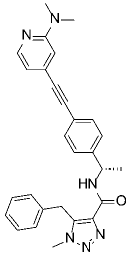 |
| IRCBC-057 | 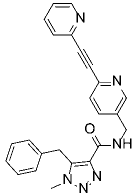 | IRCBC-102 | 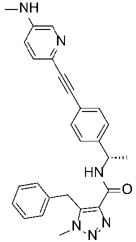 |
| IRCBC-091 | 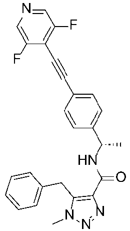 | IRCBC-105 | 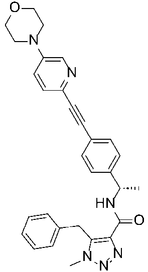 |
| IRCBC-099 | 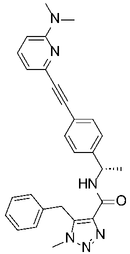 | IRCBC-106 | 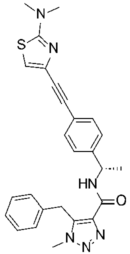 |
| IRCBC-100 | 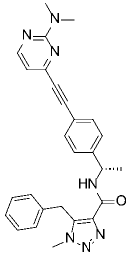 | IRCBC-119 | 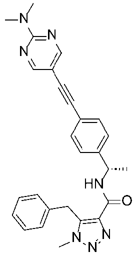 |
| IRCBC-098 | 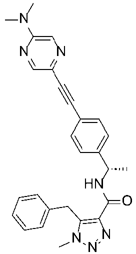 | IRCBC-122 | 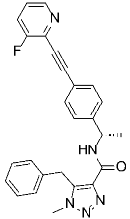 |
| IRCBC-120 | 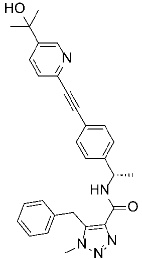 | IRCBC-131 | 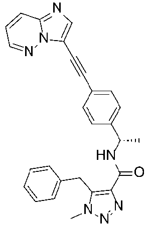 |
| IRCBC-121 | 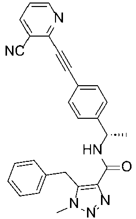 | IRCBC-103 | 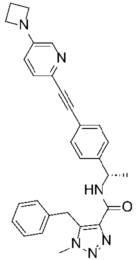 |
| IRCBC-125 | 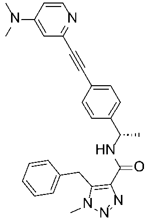 | IRCBC-104 | 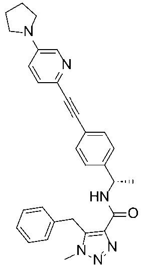 |
| IRCBC-145 | 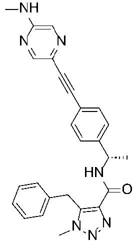 | IRCBC-129 | 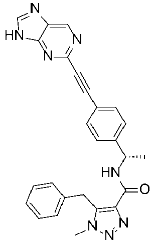 |
| IRCBC-097 | 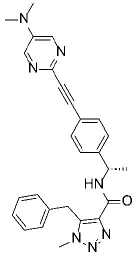 | IRCBC-146 | 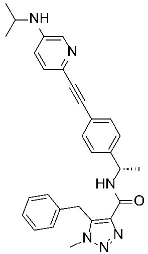 |
| IRCBC-134 | 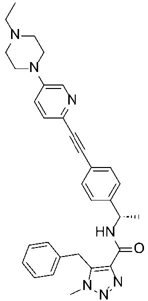 | IRCBC-124 | 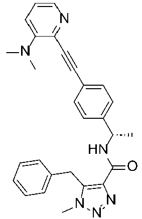 |
| IRCBC-123 | 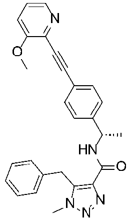 | IRCBC-149 | 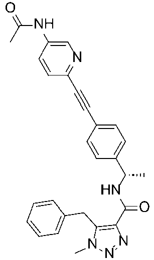 |
| IRCBC-150 | 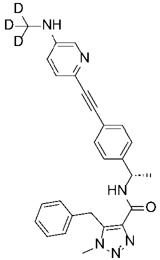 | IRCBC-036 | 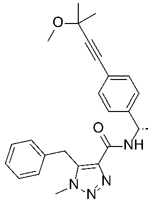 |
| IRCBC-152 | 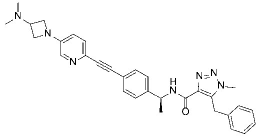 | IRCBC-154 | 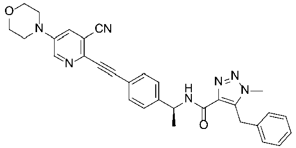 |
| IRCBC-153 | 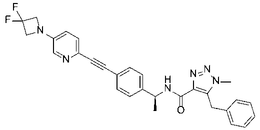 | IRCBC-159 | 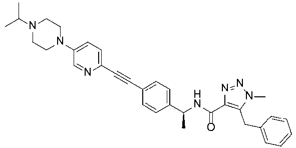 |
| IRCBC-155 | 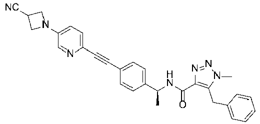 | | |
| IRCBC-156 | 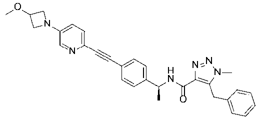 | IRCBC-157 | 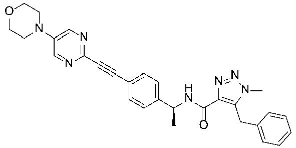 |
| IRCBC-161 | 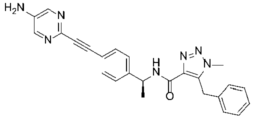 | IRCBC-158 | 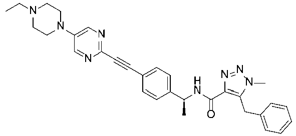 |
| IRCBC-008 | 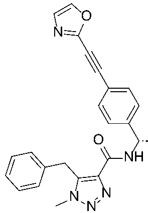 | IRCBC-259 | 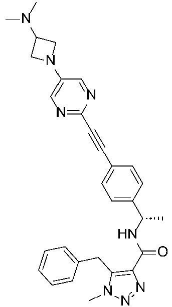 |
| IRCBC-260 | 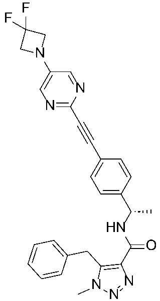 | IRCBC-258 | 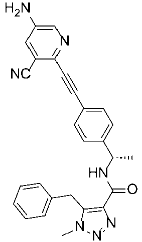 |
| IRCBC-160 | 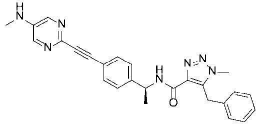 | IRCBC-261 | 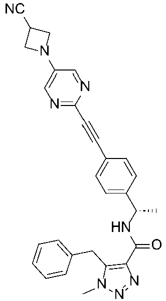 |
| IRCBC-257 | 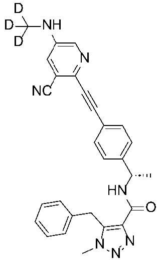 | IRCBC-262 | 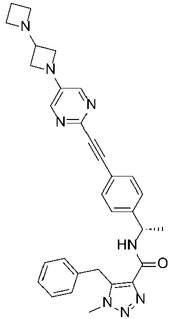 |
| IRCBC-256 | 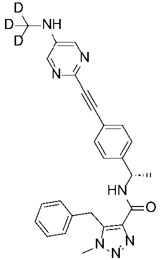 | IRCBC-276 | 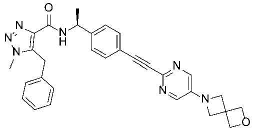 |
| IRCBC-269 | 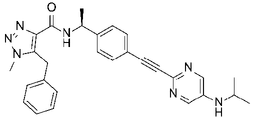 | IRCBC-271 | 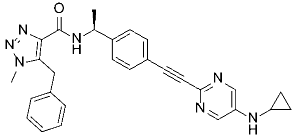 |
| IRCBC-265 | 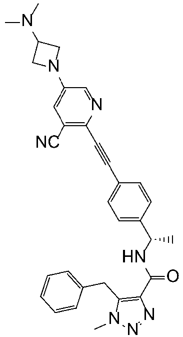 | IRCBC-266 | 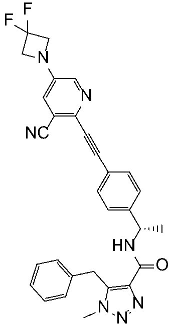 |
| IRCBC-267 | 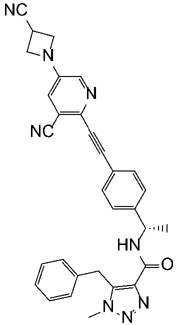 | IRCBC-268 | 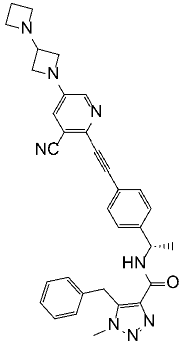 |
| IRCBC-270 | 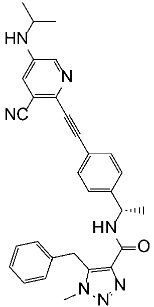 | IRCBC-272 | 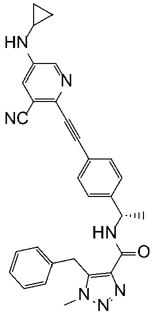 |
| IRCBC-273 | 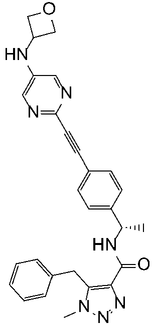 | IRCBC-274 | 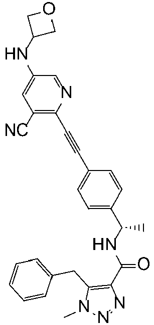 |
| IRCBC-275 | 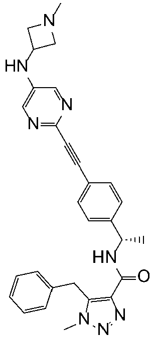 | IRCBC-277 | 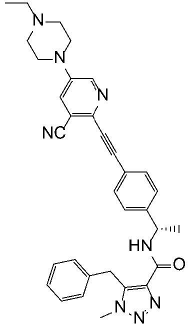 |
| IRCBC-279 | 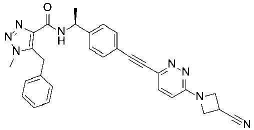 | IRCBC-280 | 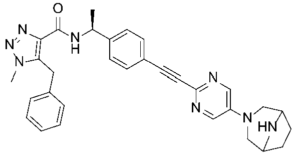 |
| IRCBC-281 | 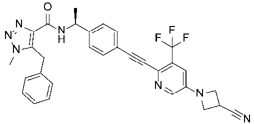 | IRCBC-282 | 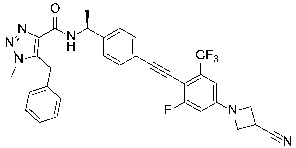 |
| IRCBC-283 | 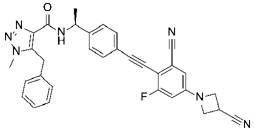 | IRCBC-284 | 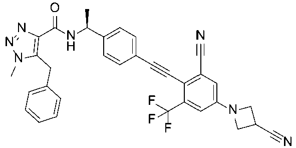 |
| IRCBC-285 | 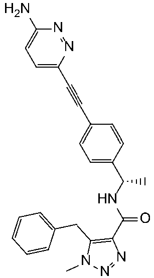 | IRCBC-286 | 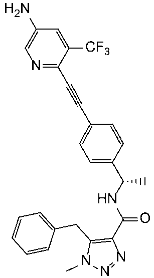 |
| IRCBC-287 | 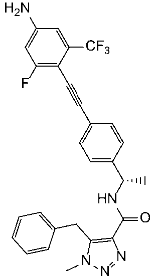 | IRCBC-288 | 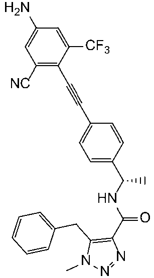 |
| IRCBC-289 | 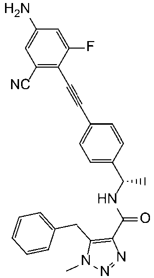 | IRCBC-290 | 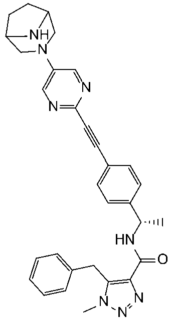 |
| IRCBC-291 | 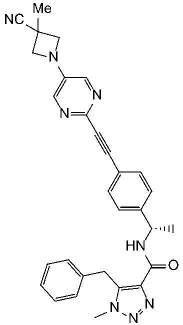 | IRCBC-292 | 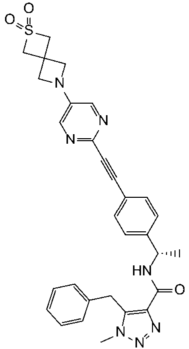 |
| IRCBC-293 | 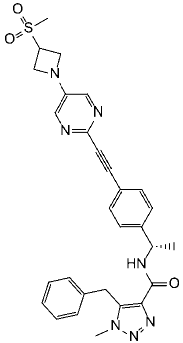 | IRCBC-294 | 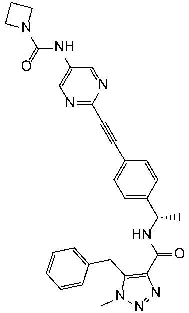 |
| IRCBC-295 . | 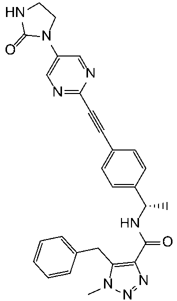 | IRCBC-296 | 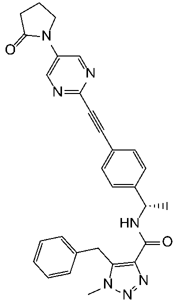 |

8. A pharmaceutical composition comprising (a) a therapeutically effective amount of the compound according to claim 1, or a pharmaceutically acceptable salt, a hydrate, or a solvate thereof, and (b) a pharmaceutically acceptable carrier.

9. Use of the compound according to claim 1, or the pharmaceutical composition according to claim 8 in the manufacture of drugs for treating or preventing a disease or condition associated with programmed cell necrosis and/or human receptor-interacting protein kinase 1 (RIPK1).

10. Use according to claim 9, wherein the disease or condition is one or more selected from the group consisting of: degenerative diseases, inflammation, ischemia-reperfusion injury, pathogenic infections, Parkinson's disease (PD), age-related macular degeneration, autoimmune diseases, retinal detachment induced photoreceptor necrosis, glaucoma, cisplatin-induced kidney injury and traumatic brain injury, atherosclerosis caused by hyperlipidemia, other diseases associated with RIPK1-dependent apoptosis, necrosis, or cytokine production, bacterial infections, viral infections, and lysosomal storage disorders.
